# EUROPEAN PATENT APPLICATION

(11) **EP 4 570 794 A1**
(43) Date of publication of application: **18.06.2025**
(21) Application number: 23852553.9
(22) Date of filing: 08.08.2023
(51) Int. Cl.: C07D 401/14, A61K 31/496, A61K 31/517, A61K 31/519, A61K 31/551, A61P 1/18, A61P 35/00, A61P 43/00, C07D 403/14, C07D 487/08, C07D 519/00

(54) **HETEROCYCLIC COMPOUND FOR INHIBITING AND/OR INDUCING DEGRADATION OF KRAS PROTEIN**

(30) Priority: 09.08.2022 JP 2022126817
(71) Applicant: Astellas Pharma, Inc., Chuo-ku Tokyo 103-8411 (JP)
(72) Inventor: YOSHINARI, Tomohiro, Tokyo 103-8411 (JP); ISHIOKA, Hiroki, Tokyo 103-8411 (JP); TAKAHASHI, Fumie, Tokyo 103-8411 (JP); KAMIKUBO, Takashi, Tokyo 103-8411 (JP); KAWAMINAMI, Eiji, Tokyo 103-8411 (JP); KURAMOTO, Kazuyuki, Tokyo 103-8411 (JP); NAGASHIMA, Takeyuki, Tokyo 103-8411 (JP); INAMURA, Kohei, Tokyo 103-8411 (JP)
(74) Representative: Hoffmann Eitle
(86) International application number: PCT/JP2023/028860
(87) International publication number: WO 2024/034591

(57) **Abstract**

To provide a compound useful as an active ingredient of a pharmaceutical composition for treating cancer, in particular, pancreatic cancer. The present inventors have studied about a compound that is useful as an active ingredient of a pharmaceutical composition for treating cancer, in particular, pancreatic cancer and have found that heterocyclic compounds represented by the formula (I) have an excellent degradation-inducing action on a KRAS protein and/or a KRAS inhibition activity and can be used as a therapeutic agent for cancer, in particular, pancreatic cancer, thus completing the present invention. The heterocyclic compound of the present invention or a salt thereof can be used as a therapeutic agent for cancer, in particular, pancreatic cancer.

## Description

### Technical Field

The present invention relates to pharmaceutical compositions and, in particular, to a heterocyclic compound that is excellent in a degradation-inducing action on a KRAS protein and/or that is expected to be useful as a KRAS inhibitor and to be useful as an active ingredient of a pharmaceutical composition for treating cancer, in particular, pancreatic cancer.

### Background Art

Pancreatic cancer mainly including pancreatic ductal adenocarcinoma is a cancer with a very poor prognosis having a five-years survival rate of 10% or less (CA Cancer J. Clin., 2016, 66, p.7-30), and about 460,000 new cases are reported per year in the world (CA Cancer J. Clin., 2018, 68, p.394-424). The most effective therapy for treating pancreatic cancer is a surgery. However, the cancer has often metastasized since early detection is difficult, and the therapeutic effect of a surgery cannot be expected in many cases. When the cancer is not treated by operation, chemotherapy or radiotherapy is adopted, but the survival rate is not so good. Today, the FOLFIRINOX therapy (multidrug treatment of three chemotherapy agents of 5-FU, irinotecan and oxaliplatin, plus levofolinate) is used as a standard therapy of pancreatic cancer. However, due to the strong toxicity, the subject patient has to be cautiously selected, for example, the therapy is to be applied only to patients of an ECOG performance status of 1 or less (J. Clin. Oncol., 2018, 36, p.2545-2556). As a molecular target drug, an epidermal growth factor receptor (EGFR) inhibitor, Erlotinib, has been approved in a combination therapy with Gemcitabine. However, the extension of the overall survival is only about two weeks as compared with Gemcitabine alone, and no satisfying therapeutic effect has been achieved. A highly effective therapeutic agent remains needed (J. Clin. Oncol., 2007, 25, p.1960-1966).

RAS proteins are low molecular weight guanosine triphosphate (GTP)-binding proteins of about 21 kDa constituted of 188-189 amino acids and include four main types of proteins (KRAS (KRAS 4A and KRAS 4B), NRAS and HRAS) produced by three genes of a KRAS gene, an NRAS gene and an HRAS gene. RAS proteins are divided into an active GTP-binding type and an inactive GDP-binding type. A RAS protein is activated by replacement of guanosine diphosphate (GDP) with GTP due to, for example, ligand stimulation to a membrane receptor, such as EGFR. The active RAS binds to effector proteins as much as twenty, such as RAF, PI3K and RALGDS, to activate the downstream signal cascade. On the other hand, the active RAS is converted to the inactive type by replacement of GTP with GDP due to the intrinsic GTP hydrolysis (GTPase) activity. The GTPase activity is enhanced by a GTPase-activating protein (GAP). As can be seen from the above statement, RAS bears an important function of "molecular switch" in an intracellular signal transduction pathway for EGFR or the like and plays a critical role in the processes of cell growth, proliferation, angiogenesis and the like (Nature Rev. Cancer, 2011, 11, p.761-774, Nature Rev. Drug Discov., 2014, 13, p.828-851, Nature Rev. Drug Discov., 2016, 15, p.771-785).

Substitution of an amino acid by spontaneous mutation of the RAS gene results in a constant activated state due to hypofunction of RAS as GTPase or hyporeactivity to GAP, and then, signals are continuously sent downstream. The excessive signaling causes carcinogenesis or cancer growth acceleration. It is said that pancreatic ductal adenocarcinoma occurs through a weakly heteromorphic stage and a subsequent highly heteromorphic stage in the pancreatic intraepithelial neoplasia (PanIN), and mutation of the KRAS gene has already been recognized in an initial stage of PanIN. Subsequently, abnormality occurs in INK4A, p53 and SMAD4, which are tumor suppression genes, leading to malignancy (Nature Rev. Cancer, 2010, 10, p.683-695). Furthermore, in 90% or more of the cases of pancreatic ductal adenocarcinoma, mutation is seen in the KRAS gene, and a majority of them are a spontaneous point mutation in the codon 12 located in the KRAS exon 2 (Cancer Cell 2017, 32, p.185-203). As can be seen from the above statement, KRAS plays a critical role in the processes of carcinogenesis and development of pancreatic cancer.

As a mutation of a KRAS gene, G12C mutant KRAS, G12D mutant KRAS and the like are known. G12C mutant KRAS frequently occurs in non-small-cell lung cancer but occurs few percent in pancreatic cancer (Cancer Cell 2014, 25, p.272-281), and a therapeutic agent against another KRAS mutation is desired. G12D mutant KRAS is seen in about 34% of the cases of pancreatic cancer, and this rate is reported to be the highest in KRAS mutations (Nat. Rev. Cancer, 2018, 18, p.767-777).

Patent Documents 1 and 2 disclose RAS inhibitors, and Patent Documents 1 and 2 disclose compounds represented by the following formulae (A) and (B) (the meanings of the symbols in the formulae can be found in the patent documents), respectively.

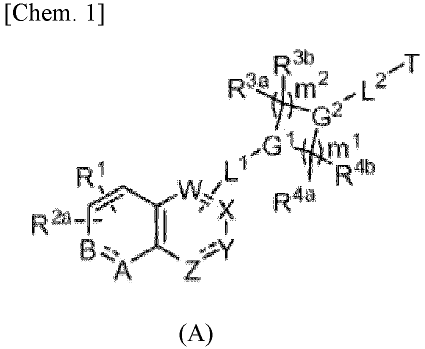

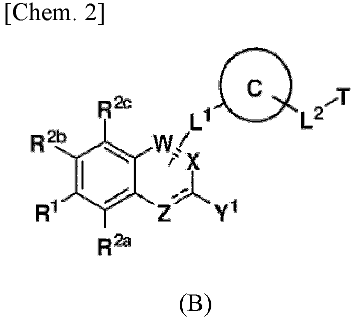

Moreover, Patent Document 3 discloses a G12C mutant KRAS inhibitor, Patent Document 4 discloses a G12D mutant KRAS inhibitor, and Patent Document 5 discloses a pan-KRAS inhibitor.

In recent years, as a technique for inducing degradation of a target protein, bifunctional compounds collectively called as PROTAC (PROteolysis-TArgeting Chimera) or SNIPER (Specific and Nongenetic IAP-dependent Protein Eraser) are found and are expected as one novel technique of drug development modality (Drug. Discov. Today Technol., 2019, 31, p15-27). Such a bifunctional compound promotes formation of a composite of the target protein and an E3 ligase in a cell, and degradation of the target protein is induced using the ubiquitin-proteasome system. The ubiquitin-proteasome system is one of intracellular protein degradation mechanisms. A protein called E3 ligase recognizes a protein to be degraded to convert the protein into ubiquitin, whereby degradation by proteasome is promoted. Typical examples of the E3 ligases include Von Hippel-Lindau (VHL), celebron (CRBN), inhibitor of apoptosis protein (IAP) and mouse double minute 2 homolog (MDM2).

The bifunctional compounds composed of a ligand of an E3 ligase an are compounds in which a ligand of a target protein and a ligand of an E3 ligase are bound via a Linker, and some bifunctional compounds for degrading a KRAS protein have ever been reported (Non-patent Document 1, Patent Document 6, Patent Document 7, Patent Document 8, Patent Document 9, and Patent Document 10). In recent years, bifunctional compounds for inducing degradation of a G12D mutant KRAS have also been reported (Patent Document 14, Patent Document 15, Patent Document 16, Patent Document 17, Patent Document 18, Patent Document 19, and Patent Document 20).

On the other hand, in recent years, several protein degradation-inducing techniques different from a method of using the bifunctional compounds composed of a ligand of an E3 ligase have also been reported. One of the novel techniques is a protein degradation-inducing technique using a ligand of the molecular chaperone heat shock protein 90 (HSP90) (Patent Document 11). HSP90 and a chaperone complex containing HSP90 are known to interact with many different E3 ligases (Cell, 2012, 150, p.987-1001), and by bringing them close together by a bifunctional compound using a target protein and a ligand of HSP90, the target protein is converted into ubiquitin via an E3 ligase in the HSP90 complex. As a result, the polyubiquitinated target protein is degraded by proteasome. In other words, such a bifunctional compound promotes formation of a composite of the target protein and an HSP90 protein, thereby inducing degradation of the target protein.

The bifunctional compounds composed of a ligand of a molecular chaperone are compounds in which a ligand of a target protein and a ligand of HSP90 are bound via a Linker, and some bifunctional compounds for degrading a KRAS protein have ever been reported (Patent Document 12 and Patent Document 13). However, no bifunctional compound for inducing degradation of mutant KRAS other than the G12C mutant KRAS, for example, G12D mutant KRAS or the like, is reported as of now.

### Citation List

### Patent Document

Patent Document 1: WO 2016/049568
Patent Document 2: WO 2017/172979
Patent Document 3: WO 2018/143315
Patent Document 4: WO 2022/002102
Patent Document 5: WO 2022/132200
Patent Document 6: US Patent Application Publication No. 2018/0015087
Patent Document 7: WO 2019/195609
Patent Document 8: WO 2020/018788
Patent Document 9: WO 2021/051034
Patent Document 10: WO 2021/207172
Patent Document 11: WO 2020/206608
Patent Document 12: WO 2022/078414
Patent Document 13: WO 2022/078470
Patent Document 14: WO 2022/148421
Patent Document 15: WO 2022/148422
Patent Document 16: WO 2022/173032
Patent Document 17: WO 2022/228576
Patent Document 18: Chinese Patent Application Publication No. 115785199
Patent Document 19: WO 2023/077441
Patent Document 20: WO 2023/280026

### Non-Patent Document

Non-patent Document 1: Cell. Chem. Biol., 2020, 27, p.19-31

### Summary of Invention

### Technical Problem

A pharmaceutical composition, for example, a heterocyclic compound that is excellent in a degradation-inducing action on a KRAS protein and/or that is expected to be useful as a KRAS inhibitor and to be useful as an active ingredient of a pharmaceutical composition for treating cancer, in particular, pancreatic cancer, for example, mutant KRAS-positive pancreatic cancer, is provided.

### Solution to Problem

The present inventors have intensively and extensively studied about a compound that is useful as an active ingredient of a pharmaceutical composition for treating cancer, in particular, pancreatic cancer. As a result, the present inventors have found that a heterocyclic compound of a formula (I), for example, a bifunctional compound of the formula (I) characterized in that a substituent on the 8-position of a heterocyclic compound selected from the group consisting of quinazoline and quinoline is bound to a ligand of an E3 ligase via a linker, has an excellent degradation-inducing action on a KRAS protein and/or a KRAS inhibition activity, thus completing the present invention.

Specifically, the present invention relates to a compound of the formula (I) or a salt thereof and a pharmaceutical composition that contains a compound of the formula (I) or a salt thereof and one or more pharmaceutically acceptable excipients. 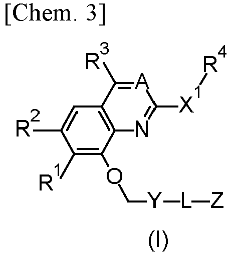 (In the formula,
A is CR^{A} or N,
R^{A} is H or C₁₋₃ alkyl,
R¹ is naphthyl optionally substituted with OH, or R¹ is a group selected from the group consisting of the formula (IIa) and the formula (IIb) below, 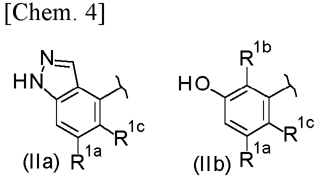
R^{1a} and R^{1b}, which are the same as or different from each other, are H, methyl, F or Cl,
R^{1c} is F, Cl, methyl or ethyl,
R² is H, halogen, optionally substituted C₁₋₃ alkyl, cyclopropyl or vinyl,
R³ is a group selected from the group consisting of the formula (III), the formula (IV), the formula (V), the formula (VI), the formula (VII), the formula (VIII), the formula (IX), the formula (X), the formula (XI), the formula (XII), the formula (XIII), the formula (XIV) and the formula (XV) below, or R³ is a saturated or unsaturated 7-membered or 8-membered bridged heterocyclic group containing one or two nitrogen atoms optionally substituted with OH, wherein when the bridged heterocyclic group is substituted with OH, OH is attached only to a carbon atom that is an atom forming a bridged heterocyclic ring, 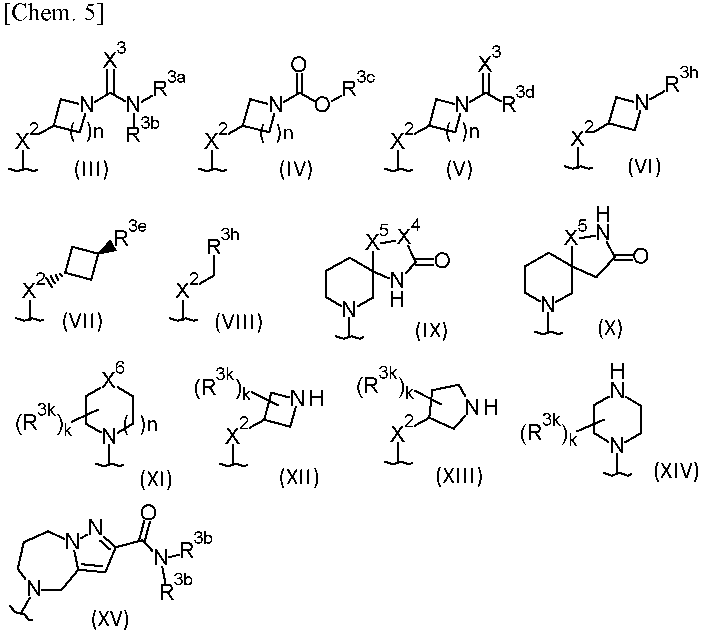
R^{3a} is -(CH₂)ₚCHR^{3f}-NR^{N1}R^{N2}, -(CH₂)ₚCHR^{3f}-OR^{3g}; a 5-membered or 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or C₁₋₃ alkyl,
R^{3c} and R^{3d} are -(CH₂)ₚCHR^{3f}-NR^{N1}R^{N2}, -(CH₂)ₚCHR^{3f}-OR^{3g}; a 4-membered to 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3e} is -O-C₂₋₃ alkylene-NR^{N1}R^{N2} or -NR^{N1}R^{N2},
R^{3f} is H, F or C₁₋₃ alkyl,
R^{3g} is H or C₁₋₃ alkyl,
R^{3h} is optionally substituted 5-membered heteroaryl containing one to four hetero atoms selected from the group consisting of oxygen, sulfur and nitrogen, optionally substituted 6-membered heteroaryl containing one to three nitrogen atoms, or cyclobutyl optionally substituted with -NR^{N1}R^{N2},
R^{N1} and R^{N2}, which are the same as or different from each other, are H or C₁₋₃ alkyl, or
R^{N1} and R^{N2}, together with the nitrogen atom to which they are attached, optionally form an optionally substituted 4-membered to 6-membered saturated heterocyclic group containing one or two hetero atoms selected from the group consisting of oxygen, sulfur and nitrogen, or
R^{3f} and R^{N1}, together with the carbon atom and the nitrogen atom to which they are attached, optionally form an optionally substituted 4-membered to 6-membered saturated heterocyclic group containing one or two hetero atoms selected from the group consisting of oxygen, sulfur and nitrogen,
each R^{3k}, which is the same as or different from each other, is a group selected from the group consisting of OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, where R^{3k} is attached only to a carbon atom that is an atom forming a ring represented by the formula (XI), the formula (XII), the formula (XIII) or the formula (XIV),
X² is -O-, -NH- or -N(C₁₋₃ alkyl)-,
X³ is O or S,
X⁴ is -CH₂- or -NH-,
X⁵ is a bond, -CH₂- or C=O,
X⁶ is -CH₂- or -O-,
n is 1 or 2,
p is 1 or 2,
k is an integer of 0 to 2,
R⁴ is optionally substituted C₁₋₆ alkyl, an optionally substituted 4-membered to 6-membered saturated heterocyclic group containing one or two hetero atoms selected from oxygen, sulfur and nitrogen, optionally substituted 5-membered heteroaryl containing one to four hetero atoms selected from oxygen, sulfur and nitrogen or optionally substituted 6-membered heteroaryl containing one to three nitrogen atoms,
X¹ is a bond, -CH₂-, -O-, -S- or -NR^{4X}-,
R^{4X} is H or C₁₋₃ alkyl,
Y is phenylene optionally substituted with a group selected from the group consisting of F and Cl or pyridinediyl,
L is -(L¹-L²-L³-L⁴-L⁵-L⁶-L⁷)-,
L¹, L², L³, L⁴, L⁵, L⁶ and L⁷, which are the same as or different from each other, are groups selected from the group consisting of a bond, -O-, -S-, -NR^{L1}-, acetylene-1,2-diyl, optionally substituted azetidinediyl, optionally substituted pyrrolidinediyl, optionally substituted piperidinediyl, optionally substituted piperazinediyl, optionally substituted diazepanediyl, optionally substituted C₁₋₃ alkylene, a saturated or unsaturated 7-membered or 8-membered bridged heterocyclic divalent group containing one or two nitrogen atoms, a saturated 7-membered to 11-membered spiroheterocyclic divalent group containing two nitrogen atoms, a saturated 8-membered to 10-membered bicycloheterocyclic divalent group containing two nitrogen atoms, optionally substituted phenylene, optionally substituted pyridinediyl, C=O, S=O and S(=O)₂,
R^{L1} is H or C₁₋₃ alkyl,
Z is a group selected from the group consisting of the formula (XVI), the formula (XVII), the formula (XVIII), the formula (XIX), the formula (XX), the formula (XXI), the formula (XXII), the formula (XXIII), the formula (XXIV), the formula (XXV), the formula (XXVI), the formula (XXVII), the formula (XXVIII), the formula (XXIX), the formula (XXX), the formula (XXXI), the formula (XXXII), the formula (XXXIII), the formula (XXXIV) and the formula (XXXV) below, 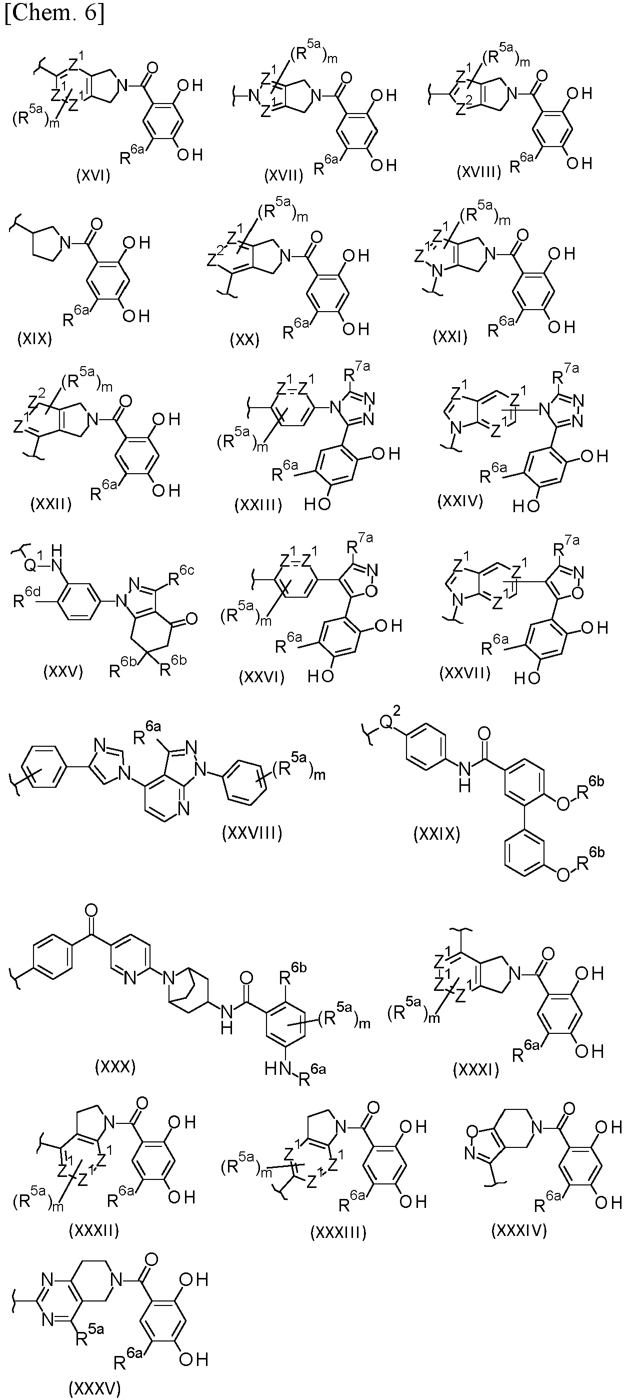
each Z¹, which is the same as or different from each other, is CH or N,
Z² is NH, NCH₃, O or S,
each R^{5a}, which is the same as or different from each other, is H, halogen, C₁₋₃ alkyl or -(C=O)NH₂,
R^{6a} is C₁₋₆ alkyl optionally substituted with F,
each R^{6b}, which is the same as or different from each other, is C₁₋₃ alkyl,
R^{6c} is C₁₋₃ alkyl optionally substituted with F,
R^{6d} is -(C-O)NR^{N3}R^{N4},
R^{N3} and R^{N4}, which are the same as or different from each other, are H or C₁₋₃ alkyl,
R^{7a} is OH or -(C=O)NR^{N5}R^{N6},
R^{N5} and R^{N6}, which are the same as or different from each other, are H, cyclopropyl or C₁₋₃ alkyl optionally substituted with F, and
m is an integer of 0 to 2.)

The present invention also relates to a compound of the formula (I) or a salt thereof and a pharmaceutical composition that contains a compound of the formula (I) or a salt thereof and one or more pharmaceutically acceptable excipients. 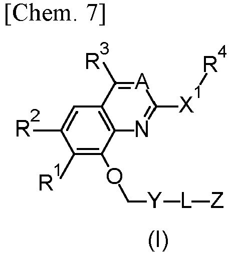 (In the formula,
A is CR^{A} or N,
R^{A} is H or C₁₋₃ alkyl,
R¹ is naphthyl optionally substituted with OH, or R¹ is a group selected from the group consisting of the formula (IIa) and the formula (IIb) below, 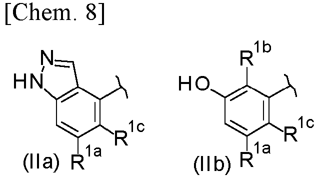
R^{1a} and R^{1b}, which are the same as or different from each other, are H, methyl, F or Cl,
R^{1c} is F, Cl, methyl or ethyl,
R² is H, halogen, optionally substituted C₁₋₃ alkyl, cyclopropyl or vinyl,
R³ is a group selected from the group consisting of the formula (III), the formula (IV), the formula (V), the formula (VI), the formula (VII), the formula (VIII), the formula (IX), the formula (X), the formula (XI), the formula (XII), the formula (XIII), the formula (XIV) and the formula (XV) below, or R³ is a saturated or unsaturated 7-membered or 8-membered bridged heterocyclic group containing one or two nitrogen atoms optionally substituted with OH, wherein when the bridged heterocyclic group is substituted with OH, OH is attached only to a carbon atom that is an atom forming a bridged heterocyclic ring, 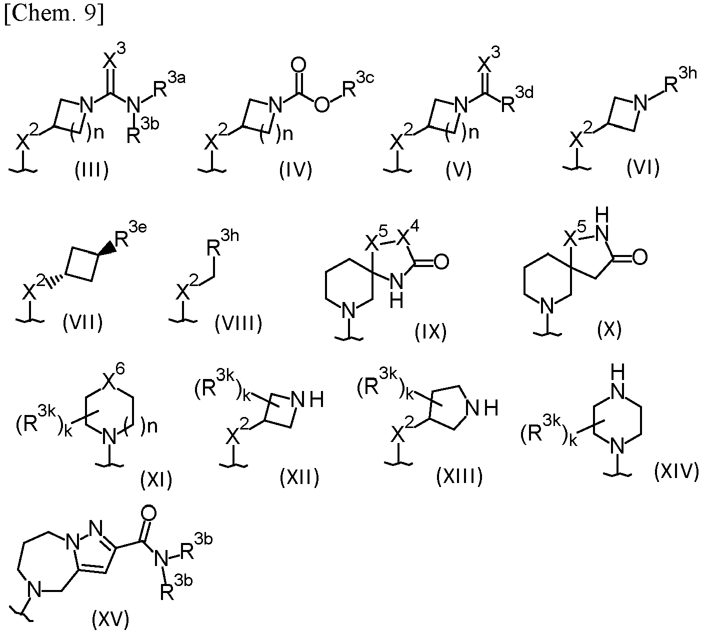
R^{3a} is -(CH₂)ₚCHR^{3f}-NR^{N1}R^{N2}, -(CH₂)ₚCHR^{3f}-OR^{3g}; a 5-membered or 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or C₁₋₃ alkyl,
R^{3c} and R^{3d} are -(CH₂)ₚCHR^{3f}-NR^{N1}R^{N2}, -(CH₂)ₚCHR^{3f}-OR^{3g}; a 4-membered to 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3e} is -O-C₂₋₃ alkylene-NR^{N1}R^{N2} or -NR^{N1}R^{N2},
R^{3f} is H, F or C₁₋₃ alkyl,
R^{3g} is H or C₁₋₃ alkyl,
R^{3h} is optionally substituted 5-membered heteroaryl containing one to four hetero atoms selected from the group consisting of oxygen, sulfur and nitrogen, optionally substituted 6-membered heteroaryl containing one to three nitrogen atoms, or cyclobutyl optionally substituted with -NR^{N1}R^{N2},
R^{N1} and R^{N2}, which are the same as or different from each other, are H or C₁₋₃ alkyl, or
R^{N1} and R^{N2}, together with the nitrogen atom to which they are attached, optionally form an optionally substituted 4-membered to 6-membered saturated heterocyclic group containing one or two hetero atoms selected from the group consisting of oxygen, sulfur and nitrogen, or
R^{3f} and R^{N1}, together with the carbon atom and the nitrogen atom to which they are attached, optionally form an optionally substituted 4-membered to 6-membered saturated heterocyclic group containing one or two hetero atoms selected from the group consisting of oxygen, sulfur and nitrogen,
each R^{3k}, which is the same as or different from each other, is a group selected from the group consisting of OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, where R^{3k} is attached only to a carbon atom that is an atom forming a ring represented by the formula (XI), the formula (XII), the formula (XIII) or the formula (XIV),
X² is -O-, -NH- or -N(C₁₋₃ alkyl)-,
X³ is O or S,
X⁴ is -CH₂- or -NH-,
X⁵ is a bond, -CH₂- or C=O,
X⁶ is -CH₂- or -O-,
provided that when R^{3c} is -(CH₂)ₚCHR^{3f}-NR^{N1}R^{N2}, X² in the formula (IV) is -O-, - NH- or -N(C₂₋₃ alkyl)-,
n is 1 or 2,
p is 1 or 2,
k is an integer of 0 to 2,
R⁴ is optionally substituted C₁₋₆ alkyl, an optionally substituted 4-membered to 6-membered saturated heterocyclic group containing one or two hetero atoms selected from oxygen, sulfur and nitrogen, optionally substituted 5-membered heteroaryl containing one to four hetero atoms selected from oxygen, sulfur and nitrogen or optionally substituted 6-membered heteroaryl containing one to three nitrogen atoms,
X¹ is a bond, -CH₂-, -O-, -S- or -NR^{4X}-,
R^{4X} is H or C₁₋₃ alkyl,
Y is phenylene optionally substituted with a group selected from the group consisting of F and Cl or pyridinediyl,
L is -(L¹-L²-L³-L⁴-L⁵-L⁶-L⁷)-,
L¹, L², L³, L⁴, L⁵, L⁶ and L⁷, which are the same as or different from each other, are groups selected from the group consisting of a bond, -O-, -S-, -NR^{L1}-, acetylene-1,2-diyl, optionally substituted azetidinediyl, optionally substituted pyrrolidinediyl, optionally substituted piperidinediyl, optionally substituted piperazinediyl, optionally substituted diazepanediyl, optionally substituted C₁₋₃ alkylene, a saturated or unsaturated 7-membered or 8-membered bridged heterocyclic divalent group containing one or two nitrogen atoms, a saturated 7-membered to 11-membered spiroheterocyclic divalent group containing two nitrogen atoms, a saturated 8-membered to 10-membered bicycloheterocyclic divalent group containing two nitrogen atoms, optionally substituted phenylene, optionally substituted pyridinediyl, C=O, S=O and S(=O)₂,
R^{L1} is H or C₁₋₃ alkyl,
Z is a group selected from the group consisting of the formula (XVI), the formula (XVII), the formula (XVIII), the formula (XIX), the formula (XX), the formula (XXI), the formula (XXII), the formula (XXIII), the formula (XXIV), the formula (XXV), the formula (XXVI), the formula (XXVII), the formula (XXVIII), the formula (XXIX), the formula (XXX), the formula (XXXI), the formula (XXXII), the formula (XXXIII), the formula (XXXIV) and the formula (XXXV) below, 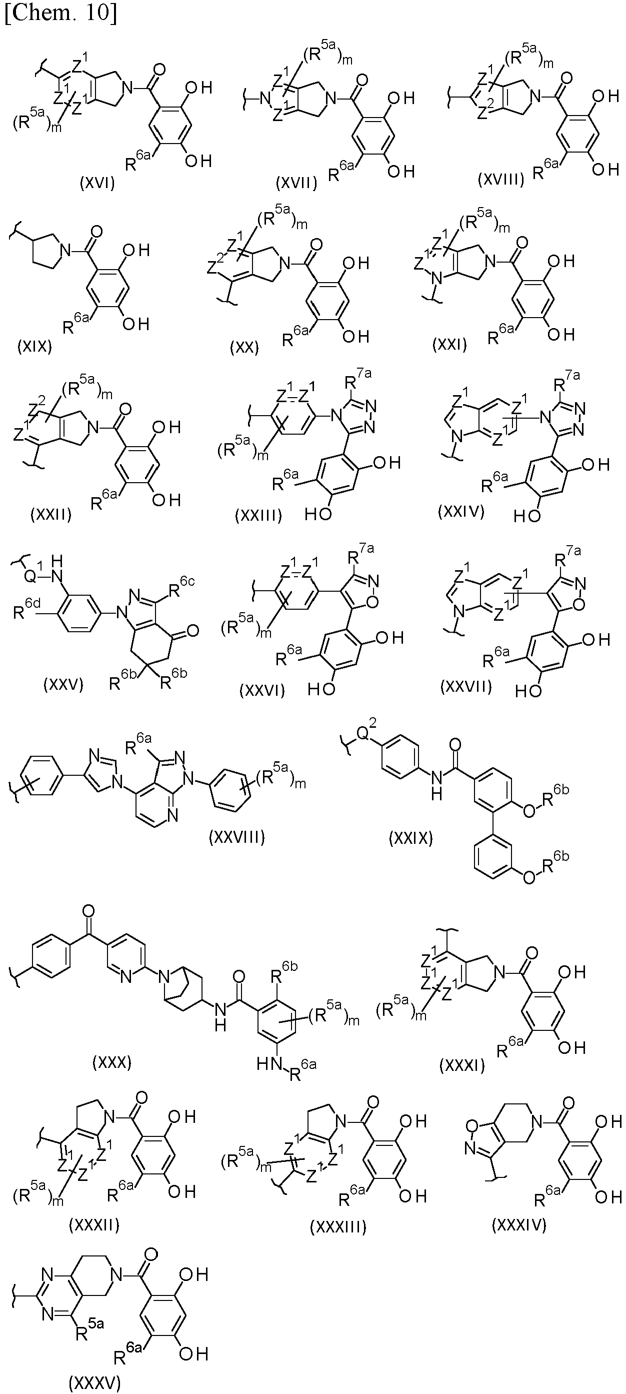
each Z¹, which is the same as or different from each other, is CH or N,
Z² is NH, NCH₃, O or S,
each R^{5a}, which is the same as or different from each other, is halogen, C₁₋₃ alkyl or - (C=O)NH₂,
R^{6a} is C₁₋₆ alkyl optionally substituted with F,
each R^{6b}, which is the same as or different from each other, is C₁₋₃ alkyl,
R^{6c} is C₁₋₃ alkyl optionally substituted with F,
R^{6d} is -(C-O)NR^{N3}R^{N4},
R^{N3} and R^{N4}, which are the same as or different from each other, are H or C₁₋₃ alkyl,
R^{7a} is OH or -(C=O)NR^{N5}R^{N6},
R^{N5} and R^{N6}, which are the same as or different from each other, are H, cyclopropyl or C₁₋₃ alkyl optionally substituted with F, and
m is an integer of 0 to 2.)

Furthermore, the compound of the formula (I) or a salt thereof is a bifunctional compound in which a ligand of a KRAS protein and a ligand of an HSP90 are bound via a Linker, and Z in the compound of the formula (I) or a salt thereof is a group capable of binding to the HSP90 protein in one embodiment. For example, reference can be made to, but not limited to, the following references.

### [Reference]

J. Med. Chem. 2020, 63, p.1798-1822

Note that, when a symbol in a chemical formula herein is used in another chemical formula, the same symbol represents the same meaning unless otherwise specified.

The present invention also relates to a pharmaceutical composition containing the compound of the formula (I) or a salt thereof and one or more pharmaceutically acceptable excipients, in one embodiment, a pharmaceutical composition for treating cancer, in particular, pancreatic cancer, in one embodiment, a pharmaceutical composition for treating mutant KRAS-positive cancer, in particular, mutant KRAS-positive pancreatic cancer, in one embodiment, a pharmaceutical composition for treating metastatic cancer, in particular, metastatic pancreatic cancer, in one embodiment, a pharmaceutical composition for treating locally advanced cancer, in particular, locally advanced pancreatic cancer, in one embodiment, a pharmaceutical composition for treating recurrent or refractory cancer, in particular, recurrent or refractory pancreatic cancer, in one embodiment, a pharmaceutical composition for treating cancer of a patient who is untreated and/or has a treatment history, in particular, pancreatic cancer of a patient who is untreated and/or has a treatment history, in one embodiment, a pharmaceutical composition for treating metastatic mutant KRAS-positive cancer, in particular, mutant KRAS-positive pancreatic cancer, in one embodiment, a pharmaceutical composition for treating locally advanced mutant KRAS-positive cancer, in particular, mutant KRAS-positive pancreatic cancer, in one embodiment, a pharmaceutical composition for treating recurrent or refractory mutant KRAS-positive cancer, in particular, mutant KRAS-positive pancreatic cancer, in one embodiment, a pharmaceutical composition for treating mutant KRAS-positive cancer, in particular, mutant KRAS-positive pancreatic cancer, of a patient who is untreated and/or has a treatment history. Note that the pharmaceutical composition for treating cancer, in particular, pancreatic cancer, and in one embodiment, mutant KRAS-positive cancer, in particular, mutant KRAS-positive pancreatic cancer, the composition containing the compound of the formula (I) or a salt thereof and one or more pharmaceutically acceptable excipients, includes a therapeutic agent containing the compound of the formula (I) or a salt thereof for cancer, in particular, pancreatic cancer, and in one embodiment, for mutant KRAS-positive cancer, in particular, mutant KRAS-positive pancreatic cancer.

The present invention also relates to use of the compound of the formula (I) or a salt thereof for the manufacture of a pharmaceutical composition for treating cancer, in particular, pancreatic cancer, in one embodiment, mutant KRAS-positive cancer, in particular, mutant KRAS-positive pancreatic cancer, in one embodiment, metastatic cancer, in particular, metastatic pancreatic cancer, in one embodiment, locally advanced cancer, in particular, locally advanced pancreatic cancer, in one embodiment, recurrent or refractory cancer, in particular, recurrent or refractory pancreatic cancer, in one embodiment, cancer of a patient who is untreated and/or has a treatment history, in particular, pancreatic cancer of a patient who is untreated and/or has a treatment history, in one embodiment, metastatic mutant KRAS-positive cancer, in particular, metastatic mutant KRAS-positive pancreatic cancer, in one embodiment, locally advanced mutant KRAS-positive cancer, in particular, locally advanced mutant KRAS-positive pancreatic cancer, in one embodiment, recurrent or refractory mutant KRAS-positive cancer, in particular, recurrent or refractory mutant KRAS-positive pancreatic cancer, in one embodiment, mutant KRAS-positive cancer of a patient who is untreated and/or has a treatment history, in particular, mutant KRAS-positive pancreatic cancer of a patient who is untreated and/or has a treatment history, to use of the compound of the formula (I) or a salt thereof for treating cancer, in particular, pancreatic cancer, in one embodiment, mutant KRAS-positive cancer, in particular, mutant KRAS-positive pancreatic cancer, to the compound of the formula (I) or a salt thereof for use in treatment of cancer, in particular, pancreatic cancer, in one embodiment, mutant KRAS-positive cancer, in particular, mutant KRAS-positive pancreatic cancer and to a method for treating cancer, in particular, pancreatic cancer, in one embodiment, mutant KRAS-positive cancer, in particular, mutant KRAS-positive pancreatic cancer, the method comprising administering an effective amount of the compound of the formula (I) or a salt thereof to a subject.

The present invention also relates to the compound of the formula (I) or a salt thereof that is a mutant KRAS protein degradation inducer and/or a mutant KRAS inhibitor, to the compound of the formula (I) or a salt thereof for use as a mutant KRAS protein degradation inducer and/or a mutant KRAS inhibitor and to a mutant KRAS protein degradation inducer and/or a mutant KRAS inhibitor containing the compound of the formula (I) or a salt thereof.

Note that the "subject" is a human or another animal that needs the treatment, and in one embodiment, the "subject" is a human who needs the prevention or treatment. Advantageous Effects of Invention

The compound of the formula (I) or a salt thereof has a degradation-inducing action on a KRAS protein and/or a KRAS inhibition activity and can be used as a therapeutic agent for cancer, in particular, pancreatic cancer, for example, mutant KRAS-positive pancreatic cancer.

### Description of Embodiments

The present invention will be described in detail below.

As used herein, "optionally substituted" means being unsubstituted or having one to five substituents. In one embodiment, the "optionally substituted" means being unsubstituted or having one to three substituents. Note that when there are multiple substituents, the substituents may be the same as or different from each other.

"C₁₋₁₂ Alkyl" is linear or branched alkyl having 1 to 12 carbon atoms, and examples thereof include methyl, ethyl, n-propyl, isopropyl, n-butyl, isobutyl, sec-butyl, tert-butyl, n-pentyl, n-hexyl, dodecyl and the like (the carbon atom numbers are described similarly hereinafter). The "C₁₋₁₂ alkyl" is ethyl or dodecyl in one embodiment.

Similarly, "C₁₋₆ alkyl" is linear or branched alkyl having 1 to 6 carbon atoms, and examples thereof include methyl, ethyl, n-propyl, isopropyl, n-butyl, isobutyl, sec-butyl, tert-butyl, n-pentyl and n-hexyl. The "C₁₋₆ alkyl" is methyl, ethyl, n-propyl, isopropyl or sec-butyl in one embodiment, methyl, ethyl, n-propyl, isopropyl or tert-butyl in one embodiment, methyl, ethyl, n-propyl, isopropyl or n-butyl in one embodiment, methyl, ethyl or n-propyl in one embodiment, methyl in one embodiment, ethyl in one embodiment or n-propyl in one embodiment.

Similarly, "C₁₋₃ alkyl" is linear or branched alkyl having 1 to 3 carbon atoms, and examples thereof include methyl, ethyl, n-propyl, isopropyl. The "C₁₋₃ alkyl" is methyl or ethyl in one embodiment, n-propyl or isopropyl in one embodiment, methyl or isopropyl in one embodiment, ethyl or isopropyl in one embodiment, methyl in one embodiment, ethyl in one embodiment, isopropyl in one embodiment or n-propyl in one embodiment.

Similarly, "C₂₋₃ alkyl" is linear or branched alkyl having two or three carbon atoms, and examples thereof include ethyl, n-propyl and isopropyl. The "C₂₋₃ alkyl" is ethyl in one embodiment, isopropyl in one embodiment or n-propyl in one embodiment.

"C₃₋₆ Cycloalkyl" is cycloalkyl having 3 to 6 carbon atoms, and examples thereof include cyclopropyl, cyclobutyl, cyclopentyl and cyclohexyl. The "C₃₋₆ cycloalkyl" is cyclobutyl, cyclopentyl or cyclohexyl in one embodiment, cyclobutyl or cyclopentyl in one embodiment, cyclopentyl or cyclohexyl in one embodiment, cyclopropyl or cyclobutyl in one embodiment, cyclopropyl in one embodiment, cyclobutyl in one embodiment, cyclopentyl in one embodiment or cyclohexyl in one embodiment.

"C₁₋₃ Alkylene" is a divalent group formed by removing the hydrogen atom from the C₁₋₃ alkyl. The "C₁₋₃ alkylene" is linear or branched C₁₋₃ alkylene, and examples thereof include methylene, ethylene, trimethylene, methylmethylene, 1,1-dimethylmethylene and the like. The "C₁₋₃ alkylene" is linear or branched C₁₋₃ alkylene in one embodiment, methylene, ethylene or trimethylene in one embodiment, methylene or ethylene in one embodiment, methylene in one embodiment or ethylene in one embodiment.

Similarly, "C₂₋₃ alkylene" is a divalent group formed by removing the hydrogen atom from the C₂₋₃ alkyl. The "C₂₋₃ alkylene" is linear or branched C₂₋₃ alkylene, ethylene or trimethylene in one embodiment, trimethylene in one embodiment or ethylene in one embodiment.

"Saturated heterocyclic group" is a saturated hydrocarbon ring group containing a hetero atom selected from the group consisting of oxygen, sulfur and nitrogen as a ring-forming atom. Further, the sulfur atom as a ring-forming atom of the saturated heterocyclic group is optionally oxidized.

Therefore, "4-membered to 6-membered saturated heterocyclic group" is a 4-membered to 6-membered saturated heterocyclic group containing a hetero atom selected from the group consisting of oxygen, sulfur and nitrogen as a ring-forming atom. The "4-membered to 6-membered saturated heterocyclic group" in one embodiment is a 4-membered to 6-membered saturated heterocyclic group containing one or two hetero atoms selected from the group consisting of oxygen, sulfur and nitrogen as ring-forming atoms. The 4-membered to 6-membered saturated heterocyclic group containing one or two hetero atoms selected from the group consisting of oxygen, sulfur and nitrogen as ring-forming atoms is a 4-membered to 6-membered saturated heterocyclic group containing one hetero atom selected from the group consisting of oxygen, sulfur and nitrogen as a ring-forming atom in one embodiment, a 5-membered or 6-membered saturated heterocyclic group containing one or two hetero atoms selected from the group consisting of oxygen, sulfur and nitrogen as ring-forming atoms in one embodiment, a 4-membered saturated heterocyclic group containing one hetero atom selected from the group consisting of oxygen, sulfur and nitrogen as a ring-forming atom in one embodiment, a 5-membered saturated heterocyclic group containing one or two hetero atoms selected from the group consisting of oxygen, sulfur and nitrogen as ring-forming atoms in one embodiment, a 6-membered saturated heterocyclic group containing one or two hetero atoms selected from the group consisting of oxygen, sulfur and nitrogen as ring-forming atoms in one embodiment, oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl or dioxothiomorpholinyl in one embodiment, oxetanyl, tetrahydrofuranyl, tetrahydropyranyl, azetidinyl, pyrrolidinyl, piperidinyl, piperazinyl, morpholinyl, thiomorpholinyl or dioxothiomorpholinyl in one embodiment, oxetanyl, tetrahydrofuranyl, tetrahydropyranyl, pyrrolidinyl, piperidinyl or morpholinyl in one embodiment, oxetanyl, tetrahydrofuranyl, tetrahydropyranyl, pyrrolidinyl or piperidinyl in one embodiment, oxetanyl, tetrahydrofuranyl or tetrahydropyranyl in one embodiment, pyrrolidinyl or piperidinyl in one embodiment, oxetanyl in one embodiment, tetrahydrofuranyl in one embodiment, tetrahydropyranyl in one embodiment, pyrrolidinyl in one embodiment, piperidinyl in one embodiment, morpholinyl in one embodiment or oxazolidinyl in one embodiment.

"Bridged heterocyclic group" is a saturated or unsaturated bridged hydrocarbon ring group containing one or two nitrogen atoms as ring-forming atoms.

Therefore, "saturated or unsaturated 7-membered or 8-membered bridged heterocyclic group" is a saturated 7-membered or 8-membered bridged heterocyclic group containing one or two nitrogen atoms as ring-forming atoms or a 7-membered or 8-membered bridged heterocyclic group having an unsaturated bond that contains one or two nitrogen atoms. The "saturated or unsaturated 7-membered or 8-membered bridged heterocyclic group" is a saturated 7-membered or 8-membered bridged heterocyclic group containing two nitrogen atoms in one embodiment, a saturated 7-membered or 8-membered bridged heterocyclic group containing two nitrogen atoms in which one of the two nitrogen atoms bonds to one hydrogen atom in one embodiment or a saturated 7-membered or 8-membered bridged heterocyclic group containing one nitrogen atom in one embodiment. Examples thereof include diazabicyclo[2.2.2]octanyl, diazabicyclo[3.2.1]octanyl, diazabicyclo[3.2.1]octenyl, diazabicyclo[3.1.1]heptanyl, diazabicyclo[2.2.1]heptanyl, diazabicyclo[2.2.1]heptenyl, azabicyclo[2.2.2]octanyl, azabicyclo[3.2.1]octanyl, azabicyclo[3.1.1]heptanyl and azabicyclo[2.2.1]heptanyl. The "saturated or unsaturated 7-membered or 8-membered bridged heterocyclic group" is diazabicyclo[2.2.2]octanyl, diazabicyclo[3.2.1]octanyl, diazabicyclo[3.2.1]oct-6-enyl, diazabicyclo[3.2.1]oct-2-enyl, diazabicyclo[3.1.1]heptanyl, diazabicyclo[2.2.1]heptanyl, diazabicyclo[2.2.1]hept-5-enyl, azabicyclo[2.2.2]octanyl, azabicyclo[3.2.1]octanyl, azabicyclo[3.1.1]heptanyl or azabicyclo[2.2.1]heptanyl in one embodiment, diazabicyclo[2.2.2]octanyl, diazabicyclo[3.2.1]octanyl, diazabicyclo[3.1.1]heptanyl, diazabicyclo[2.2.1]heptanyl or azabicyclo[3.2.1]octanyl in one embodiment, diazabicyclo[2.2.2]octanyl, diazabicyclo[3.2.1]octanyl, diazabicyclo[3.1.1]heptanyl or diazabicyclo[2.2.1]heptanyl in one embodiment, 2,5-diazabicyclo[2.2.2]octanyl, 3,8-diazabicyclo[3.2.1]octanyl, 3,6-diazabicyclo[3.1.1]heptanyl or 2,5-diazabicyclo[2.2.1]heptanyl in one embodiment, diazabicyclo[2.2.1]heptanyl in one embodiment, 2,5-diazabicyclo[2.2.1]heptanyl in one embodiment, 2,5-diazabicyclo[2.2.1]heptan-2-yl in one embodiment or azabicyclo[3.2.1]octanyl in one embodiment.

Note that the "bridged heterocyclic divalent group" is a divalent group formed by removing any one hydrogen from the "bridged heterocyclic group".

Therefore, "saturated or unsaturated 7-membered or 8-membered bridged heterocyclic divalent group" is a saturated 7-membered or 8-membered bridged heterocyclic divalent group containing one or two nitrogen atoms as ring-forming atoms or a 7-membered or 8-membered bridged heterocyclic divalent group having an unsaturated bond that contains one or two nitrogen atoms.

"Spiroheterocyclic divalent group" is a divalent group formed by removing a hydrogen atom from a saturated spirohydrocarbon ring group containing one or two nitrogen atoms as ring-forming atoms.

Therefore, "saturated 7-membered to 11-membered spiroheterocyclic divalent group" is a saturated 7-membered to 11-membered spiroheterocyclic divalent group containing one or two nitrogen atoms as ring-forming atoms. The "saturated 7-membered to 11-membered spiroheterocyclic divalent group" is a saturated 7-membered to 11-membered spiroheterocyclic divalent group containing two nitrogen atoms in one embodiment. Examples thereof include diazaspiro[3.3]heptanediyl, diazaspiro[3.4]octanediyl, diazaspiro[3.5]nonanediyl, diazaspiro[4.5]decanediyl and diazaspiro[5.5]undecanediyl.

"Bicycloheterocyclic divalent group" is a divalent group formed by removing a hydrogen atom from a saturated bicyclohydrocarbon ring group containing one or two nitrogen atoms as ring-forming atoms.

Therefore, "saturated 8-membered to 10-membered bicycloheterocyclic divalent group" is a saturated 8-membered to 10-membered bicycloheterocyclic divalent group containing one or two nitrogen atoms as ring-forming atoms. The "saturated 8-membered to 10-membered bicycloheterocyclic divalent group" is a saturated 8-membered to 10-membered bicycloheterocyclic divalent group containing two nitrogen atoms in one embodiment. Examples thereof include azabicyclo[3.3.0]octanediyl and diazabicyclo[3.3.0]octanediyl.

"Heteroaryl" is an aromatic heterocyclic group containing hetero atoms selected from the group consisting of oxygen, sulfur and nitrogen as ring-forming atoms.

Therefore, "5-membered heteroaryl" is a 5-membered aromatic heterocyclic group containing one to four hetero atoms selected from the group consisting of oxygen, sulfur and nitrogen as ring-forming atoms.

The "5-membered heteroaryl" is a 5-membered aromatic heterocyclic group containing one to three hetero atoms selected from the group consisting of oxygen, sulfur and nitrogen as ring-forming atoms in one embodiment, pyrazolyl, imidazolyl, triazolyl, tetrazolyl, oxazolyl, isoxazolyl, thiazolyl, isothiazolyl, oxadiazolyl or thiadiazolyl in one embodiment, pyrazolyl, imidazolyl, triazolyl, oxazolyl or thiazolyl in one embodiment, pyrazolyl, imidazolyl, oxazolyl or thiazolyl in one embodiment, pyrazolyl, imidazolyl, triazolyl or isoxazolyl in one embodiment, pyrazolyl, oxazolyl or thiazolyl in one embodiment, pyrazolyl, triazolyl or isoxazolyl in one embodiment, pyrazolyl or thiazolyl in one embodiment, pyrazolyl or triazolyl in one embodiment, pyrazolyl in one embodiment, imidazolyl in one embodiment, oxazolyl in one embodiment, thiazolyl in one embodiment or triazolyl in one embodiment.

"6-Membered heteroaryl" is a 6-membered aromatic heterocyclic group containing one to three nitrogen atoms as ring-forming atoms. The "6-membered heteroaryl" is pyridyl, pyrimidinyl, pyrazinyl, pyridazinyl or triazinyl in one embodiment, pyridyl or pyridazinyl in one embodiment, pyridyl or pyrimidinyl in one embodiment, pyridyl in one embodiment or pyrimidinyl in one embodiment.

"Halogen" means F, Cl, Br and I. The "halogen" is F, Cl or Br in one embodiment, F or Cl in one embodiment, F or Br in one embodiment, F in one embodiment, Cl in one embodiment or Br in one embodiment.

Substituents acceptable in "optionally substituted C₁₋₆ alkyl" and "optionally substituted C₁₋₃ alkyl" are F, OH, OCH₃, N(CH₃)₂, optionally substituted C₃₋₆ cycloalkyl, azabicyclo[3.3.0]octanyl or an optionally substituted 4-membered to 6-membered saturated heterocyclic group containing one or two hetero atoms selected from the group consisting of oxygen, sulfur and nitrogen in one embodiment, F, OH, OCH₃, N(CH₃)₂, hydroxymethyl, methoxymethyl, difluoroethyl, optionally substituted cyclopropyl, tetrahydrofuranyl, optionally substituted tetrahydropyranyl, morpholinyl, optionally substituted pyrrolidinyl, optionally substituted piperidinyl or azabicyclo[3.3.0]octanyl in one embodiment, F, OH, OCH₃, N(CH₃)₂, hydroxymethyl, methoxymethyl, optionally substituted cyclopropyl, tetrahydrofuranyl, optionally substituted tetrahydropyranyl or optionally substituted pyrrolidinyl in one embodiment, F, OH, OCH₃, N(CH₃)₂, hydroxymethyl, methoxymethyl, cyclopropyl, (hydroxymethyl)cyclopropyl, (methoxymethyl)cyclopropyl, tetrahydrofuranyl, tetrahydropyranyl, (hydroxymethyl)tetrahydropyranyl, (methoxymethyl)tetrahydropyranyl, pyrrolidinyl or methylpyrrolidinyl in one embodiment, F, OH, OCH₃, (methoxymethyl)cyclopropyl, tetrahydrofuranyl or methylpyrrolidinyl in one embodiment, F, OH or cyclopropyl in one embodiment, F, OH or OCH₃ in one embodiment, OH or OCH₃ in one embodiment, F or OCH₃ in one embodiment, OH in one embodiment, F in one embodiment or OCH₃ in one embodiment.

Substituent acceptable in "optionally substituted 5-membered heteroaryl", "optionally substituted 6-membered heteroaryl", "optionally substituted C₃₋₆ cycloalkyl", "optionally substituted pyrazolyl", "optionally substituted pyridyl", "optionally substituted pyrimidinyl", "optionally substituted phenyl" and "optionally substituted cyclopropyl" are C₁₋₃ alkyl optionally substituted with a group selected from the group consisting of OH and OCH₃, -SO₂CH₃, halogen, OH, OCH₃ or C₃₋₆ cycloalkyl in one embodiment, C₁₋₃ alkyl optionally substituted with a group selected from the group consisting of OH and OCH₃ in one embodiment, C₁₋₃ alkyl optionally substituted with OH in one embodiment, C₁₋₃ alkyl optionally substituted with OCH₃ in one embodiment, C₁₋₃ alkyl or halogen in one embodiment, methyl, ethyl, methoxymethyl or F in one embodiment or methyl, ethyl or F in one embodiment.

Substituents acceptable in "optionally substituted 4-membered to 6-membered saturated heterocyclic group", "optionally substituted pyrrolidinyl", "optionally substituted piperidinyl", "optionally substituted oxetanyl", "optionally substituted tetrahydrofuranyl" and "optionally substituted tetrahydropyranyl" are C₁₋₃ alkyl optionally substituted with a group selected from the group consisting of F, OH and OCH₃, F, OH, OCH₃, oxo or oxetanyl in one embodiment, F, OH or OCH₃ in one embodiment, OH or methyl in one embodiment, C₁₋₃ alkyl optionally substituted with a group selected from the group consisting of F, OH and OCH₃, F, oxo or oxetanyl in one embodiment, C₁₋₃ alkyl optionally substituted with a group selected from the group consisting of F, OH and OCH₃ or oxo in one embodiment, C₁₋₃ alkyl optionally substituted with a group selected from the group consisting of F, OH and OCH₃ in one embodiment, C₁₋₃ alkyl optionally substituted with F in one embodiment, C₁₋₃ alkyl optionally substituted with OH in one embodiment, C₁₋₃ alkyl optionally substituted with OCH₃ in one embodiment or C₁₋₃ alkyl in one embodiment.

Substituents acceptable in "optionally substituted azetidinediyl", "optionally substituted pyrrolidinediyl", "optionally substituted piperidinediyl", "optionally substituted piperazinediyl", "optionally substituted diazepanediyl" and "optionally substituted C₁₋₃ alkylene" are F, OH, OCH₃ or optionally substituted C₁₋₃ alkyl in one embodiment, F, OH, OCH₃, methyl, ethyl, hydroxymethyl or methoxymethyl in one embodiment or F, OH, OCH₃ or methyl in one embodiment.

Substituents acceptable in "optionally substituted phenylene" and "optionally substituted pyridinediyl" are F, Cl or optionally substituted C₁₋₃ alkyl in one embodiment, F or Cl in one embodiment or F in one embodiment.

"C₁₋₃ Alkyl optionally substituted with F" in one embodiment is methyl optionally substituted with F or ethyl optionally substituted with F. Examples thereof include methyl, ethyl, monofluoromethyl, difluoromethyl, trifluoromethyl, monofluoroethyl, difluoroethyl and trifluoroethyl. The "C₁₋₃ alkyl optionally substituted with F" is methyl, ethyl, monofluoromethyl, difluoromethyl or difluoroethyl in one embodiment, monofluoromethyl or difluoromethyl in one embodiment, monofluoromethyl or difluoroethyl in one embodiment, difluoromethyl or difluoroethyl in one embodiment, monofluoromethyl in one embodiment, difluoromethyl in one embodiment, difluoroethyl in one embodiment or 2,2-difluoroethyl in one embodiment.

"C₁₋₃ Alkyl optionally substituted with OH" in one embodiment is methyl optionally substituted with one OH or ethyl optionally substituted with one or two OH. Examples thereof include methyl, ethyl, hydroxymethyl, 1-hydroxyethyl, 2-hydroxyethyl and 1,2-dihydroxyethyl. The "C₁₋₃ alkyl optionally substituted with OH" is methyl, ethyl or hydroxymethyl in one embodiment, methyl or hydroxymethyl in one embodiment, hydroxymethyl or hydroxyethyl in one embodiment, hydroxymethyl in one embodiment or hydroxyethyl in one embodiment.

"C₁₋₃ Alkyl optionally substituted with OCH₃" in one embodiment is methyl optionally substituted with one OCH₃, ethyl optionally substituted with one or two OCH₃ or propyl optionally substituted with one to three OCH₃. Examples thereof include methyl, ethyl, methoxymethyl, 1-methoxyethyl, 2-methoxyethyl, 1,2-dimethoxyethyl and 2-methoxypropyl. The "C₁₋₃ alkyl optionally substituted with OCH₃" is methoxymethyl or methoxyethyl in one embodiment, methoxymethyl in one embodiment, methoxyethyl in one embodiment or 2-methoxypropyl in one embodiment.

"Phenylene optionally substituted with F" in one embodiment is phenylene optionally substituted with one or two F. The "phenylene optionally substituted with F" is phenylene optionally substituted with one F in one embodiment, phenylene or fluorophenylene in one embodiment, phenylene in one embodiment, 2-fluoro-1,4-phenylene in one embodiment or 3-fluoro-1,4-phenylene in one embodiment.

"Naphthyl optionally substituted with OH" in one embodiment is naphthyl optionally substituted with one OH. The "naphthyl optionally substituted with OH" is 3-hydroxy-1-naphthyl in one embodiment.

"Saturated or unsaturated 7-membered or 8-membered bridged heterocyclic group containing one or two nitrogen atoms optionally substituted with OH" in one embodiment is a saturated or unsaturated 7-membered or 8-membered bridged heterocyclic group containing one or two nitrogen atoms optionally substituted with one OH or a saturated 7-membered or 8-membered bridged heterocyclic group containing one nitrogen atom optionally substituted with one OH. Examples thereof include diazabicyclo[2.2.2]octanyl, diazabicyclo[3.2.1]octanyl, diazabicyclo[3.1.1]heptanyl, diazabicyclo[2.2.1]heptanyl, azabicyclo[3.2.1]octanyl and hydroxyazabicyclo[3.2.1]octanyl. The "saturated or unsaturated 7-membered or 8-membered bridged heterocyclic group containing one or two nitrogen atoms optionally substituted with OH" is 2,5-diazabicyclo[2.2.2]octanyl, 3,8-diazabicyclo[3.2.1]octanyl, 3,6-diazabicyclo[3.1.1]heptanyl, 2,5-diazabicyclo[2.2.1]heptanyl, 3-hydroxy-6-azabicyclo[3.2.1]octanyl or 6-hydroxy-3-azabicyclo[3.2.1]octanyl in one embodiment, diazabicyclo[2.2.1]heptanyl in one embodiment, 2,5-diazabicyclo[2.2.1]heptanyl in one embodiment, hydroxyazabicyclo[3.2.1]octanyl in one embodiment or 3-hydroxy-6-azabicyclo[3.2.1]octanyl or 6-hydroxy-3-azabicyclo[3.2.1]octanyl in one embodiment.

"KRAS Protein" is a protein encoded by a KRAS gene and represents a protein encoded by a wildtype KRAS gene and a mutant KRAS gene.

"Mutant KRAS protein" represents a protein encoded by a mutant KRAS gene.

"Mutant KRAS" is KRAS having a mutation and represents, for example, G12D mutant KRAS, G12V mutant KRAS and G12C mutant KRAS.

"G12D Mutation" represents a mutation in which the amino acid residue corresponding to the codon 12 in a wildtype protein is converted from glycine to aspartic acid.

"G12D Mutant KRAS" represents KRAS having the "G12D mutation".

"G12V Mutation" represents a mutation in which the amino acid residue corresponding to the codon 12 in a wildtype protein is converted from glycine to valine.

"G12V Mutant KRAS" represents KRAS having the "G12V mutation".

"G12C Mutation" represents a mutation in which the amino acid residue corresponding to the codon 12 in a wildtype protein is converted from glycine to cysteine.

"G12C Mutant KRAS" represents KRAS having the "G12C mutation".

"Pancreatic cancer" is a malignant tumor occurring in the pancreas. Examples thereof include pancreatic ductal carcinoma and pancreatic ductal adenocarcinoma, and the "pancreatic cancer" is pancreatic ductal carcinoma in one embodiment or pancreatic ductal adenocarcinoma in one embodiment. Moreover, the "pancreatic cancer" is metastatic pancreatic cancer in one embodiment, locally advanced pancreatic cancer in one embodiment, recurrent or refractory pancreatic cancer in one embodiment or pancreatic cancer of a patient who is untreated and/or has a treatment history in one embodiment.

"Mutant KRAS-positive pancreatic cancer" is pancreatic cancer that is positive for mutant KRAS, in particular, pancreatic cancer that is positive for G12V mutant, G12D mutant and/or G12C mutant KRAS. Examples thereof include a pancreatic cancer in which the KRAS G12V mutation, G12D mutation and/or G12C mutation occurs and a pancreatic cancer which has a high positive rate for G12V mutant, G12D mutant and/or G12C mutant KRAS. The "mutant KRAS-positive pancreatic cancer" is mutant KRAS-positive pancreatic ductal carcinoma, in particular, G12V mutant, G12D mutant and/or G12C mutant KRAS-positive pancreatic ductal carcinoma in one embodiment or mutant KRAS-positive pancreatic ductal adenocarcinoma, in particular, G12V mutant, G12D mutant and/or G12C mutant KRAS-positive pancreatic ductal adenocarcinoma in one embodiment.

Embodiments of the compound of the formula (I) or a salt thereof of the present invention are shown below.
(1-1) The compound or a salt thereof in which A is CR^{A} or N, and
   R^{A} is H or C₁₋₃ alkyl.
(1-2) The compound or a salt thereof in which A is CR^{A} or N, and R^{A} is H.
(1-3) The compound or a salt thereof in which A is N.
(1-4) The compound or a salt thereof in which A is CH.
(2-1) The compound or a salt thereof in which R¹ is naphthyl optionally substituted with OH, or R¹ is a group selected from the group consisting of the formula (IIa) and the formula (IIb) below, 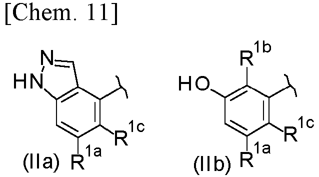
   R^{1a} and R^{1b}, which are the same as or different from each other, are H, methyl, F or Cl, and
   R^{1c} is F, Cl, methyl or ethyl.
(2-2) The compound or a salt thereof in which R¹ is the formula (IIa) below, 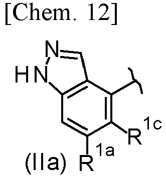
   R^{1a} is F, and
   R^{1c} is methyl.
(3-1) The compound or a salt thereof in which R² is H, halogen, optionally substituted C₁₋₃ alkyl, cyclopropyl or vinyl.
(3-2) The compound or a salt thereof in which R² is cyclopropyl.
(4-1) The compound or a salt thereof in which R³ is a group selected from the group consisting of the formula (III), the formula (IV), the formula (V), the formula (VI), the formula (VII), the formula (VIII), the formula (IX), the formula (X), the formula (XI), the formula (XII), the formula (XIII), the formula (XIV) and the formula (XV) below, or R³ is a saturated or unsaturated 7-membered or 8-membered bridged heterocyclic group containing one or two nitrogen atoms optionally substituted with OH, wherein when the bridged heterocyclic group is substituted with OH, OH is attached only to a carbon atom that is an atom forming a bridged heterocyclic ring, 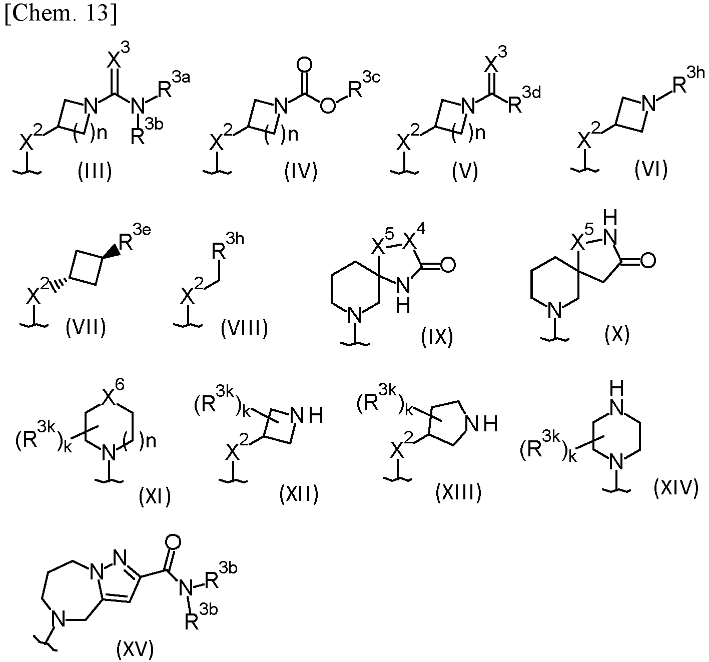
   R^{3a} is -(CH₂)ₚCHR^{3f}-NR^{N1}R^{N2}, -(CH₂)ₚCHR^{3f}-OR^{3g}; a 5-membered or 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3b} is H or C₁₋₃ alkyl,
   R^{3c} and R^{3d} are -(CH₂)ₚCHR^{3f}-NR^{N1}R^{N2}, -(CH₂)ₚCHR^{3f}-OR^{3g}; a 4-membered to 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3e} is -O-C₂₋₃ alkylene-NR^{N1}R^{N2} or -NR^{N1}R^{N2},
   R^{3f} is H, F or C₁₋₃ alkyl,
   R^{3g} is H or C₁₋₃ alkyl,
   R^{3h} is optionally substituted 5-membered heteroaryl containing one to four hetero atoms selected from the group consisting of oxygen, sulfur and nitrogen, optionally substituted 6-membered heteroaryl containing one to three nitrogen atoms, or cyclobutyl optionally substituted with -NR^{N1}R^{N2},
   R^{N1} and R^{N2}, which are the same as or different from each other, are H or C₁₋₃ alkyl, or
   R^{N1} and R^{N2}, together with the nitrogen atom to which they are attached, optionally form an optionally substituted 4-membered to 6-membered saturated heterocyclic group containing one or two hetero atoms selected from the group consisting of oxygen, sulfur and nitrogen, or
   R^{3f} and R^{N1}, together with the carbon atom and the nitrogen atom to which they are attached, optionally form an optionally substituted 4-membered to 6-membered saturated heterocyclic group containing one or two hetero atoms selected from the group consisting of oxygen, sulfur and nitrogen,
   each R^{3k}, which is the same as or different from each other, is a group selected from the group consisting of OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, where R^{3k} is attached only to a carbon atom that is an atom forming a ring represented by the formula (XI), the formula (XII), the formula (XIII) or the formula (XIV),
   X² is -O-, -NH- or -N(C₁₋₃ alkyl)-,
   X³ is O or S,
   X⁴ is -CH₂- or -NH-,
   X⁵ is a bond, -CH₂- or C=O,
   X⁶ is -CH₂- or -O-,
   n is 1 or 2,
   p is 1 or 2, and
   k is an integer of 0 to 2.
(4-2) The compound or a salt thereof in which R³ is a group selected from the group consisting of the formula (III-a), the formula (IV-a), the formula (V-a), the formula (XIII-a) and the formula (XXXVI) below, 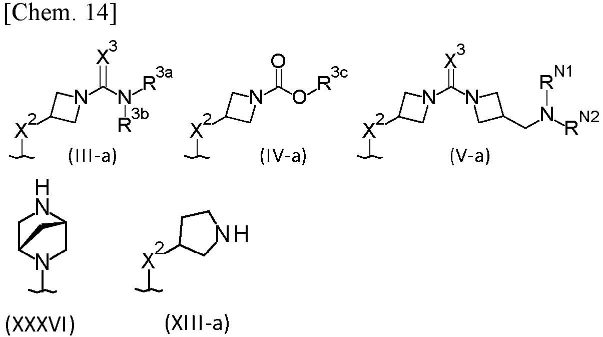
   R^{3a} is -(CH₂)ₚCHR^{3f}-NR^{N1}R^{N2},
   R^{3b} is H,
   R^{3c} is -(CH₂)ₚCHR^{3f}-NR^{N1}R^{N2} or C₄₋₆ cycloalkyl optionally substituted with - NR^{N1}R^{N2},
   R^{3f} is H,
   R^{N1} and R^{N2} are both C₁₋₃ alkyl,
   X² is -O-, -NH- or -N(C₁₋₃ alkyl)-,
   X³ is O, and
   p is 1.
(4-3) The compound or a salt thereof in which R³ is a group selected from the group consisting of the formula (III-a), the formula (IV-a), the formula (XXXVI) and the formula (XIII-a) below, 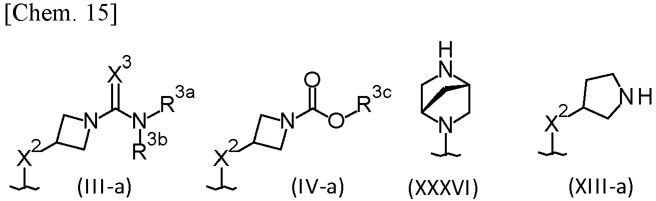
   R^{3a} is -(CH₂)ₚCHR^{3f}-NR^{N1}R^{N2},
   R^{3b} is H,
   R^{3c} is -(CH₂)ₚCHR^{3f}-NR^{N1}R^{N2},
   R^{3f} is H,
   R^{N1} and R^{N2} are both C₁₋₃ alkyl,
   X² is -O- or -N(C₁₋₃ alkyl)-,
   X³ is O, and
   p is 1.
(4-4) The compound or a salt thereof in which R³ is a group selected from the group consisting of the formula (III), the formula (IV), the formula (V), the formula (VI), the formula (VII), the formula (VIII), the formula (IX), the formula (X), the formula (XI), the formula (XII), the formula (XIII), the formula (XIV) and the formula (XV) below, or R³ is a saturated or unsaturated 7-membered or 8-membered bridged heterocyclic group containing one or two nitrogen atoms optionally substituted with OH, wherein when the bridged heterocyclic group is substituted with OH, OH is attached only to a carbon atom that is an atom forming a bridged heterocyclic ring, 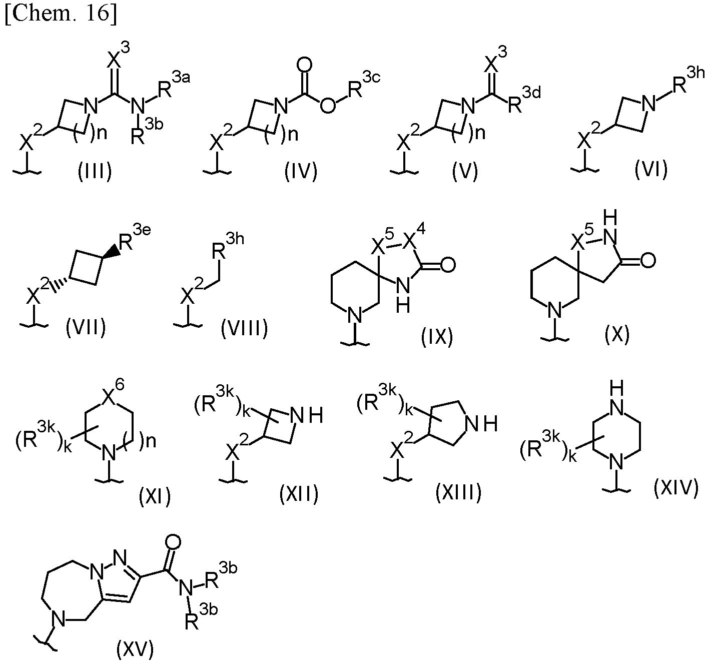
   R^{3a} is -(CH₂)ₚCHR^{3f}-NR^{N1}R^{N2}, -(CH₂)ₚCHR^{3f}-OR^{3g}; a 5-membered or 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3b} is H or C₁₋₃ alkyl,
   R^{3c} and R^{3d} are -(CH₂)ₚCHR^{3f}-NR^{N1}R^{N2}, -(CH₂)ₚCHR^{3f}-OR^{3g}; a 4-membered to 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2};or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3e} is -O-C₂₋₃ alkylene-NR^{N1}R^{N2} or -NR^{N1}R^{N2},
   R^{3f} is H, F or C₁₋₃ alkyl,
   R^{3g} is H or C₁₋₃ alkyl,
   R^{3h} is optionally substituted 5-membered heteroaryl containing one to four hetero atoms selected from the group consisting of oxygen, sulfur and nitrogen, optionally substituted 6-membered heteroaryl containing one to three nitrogen atoms, or cyclobutyl optionally substituted with -NR^{N1}R^{N2},
   R^{N1} and R^{N2}, which are the same as or different from each other, are H or C₁₋₃ alkyl, or
   R^{N1} and R^{N2}, together with the nitrogen atom to which they are attached, optionally form an optionally substituted 4-membered to 6-membered saturated heterocyclic group containing one or two hetero atoms selected from the group consisting of oxygen, sulfur and nitrogen, or
   R^{3f} and R^{N1}, together with the carbon atom and the nitrogen atom to which they are attached, optionally form an optionally substituted 4-membered to 6-membered saturated heterocyclic group containing one or two hetero atoms selected from the group consisting of oxygen, sulfur and nitrogen,
   each R^{3k}, which is the same as or different from each other, is a group selected from the group consisting of OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, where R^{3k} is attached only to a carbon atom that is an atom forming a ring represented by the formula (XI), the formula (XII), the formula (XIII) or the formula (XIV),
   X² is -O-, -NH- or -N(C₁₋₃ alkyl)-,
   X³ is O or S,
   X⁴ is -CH₂- or -NH-,
   X⁵ is a bond, -CH₂- or C=O,
   X⁶ is -CH₂- or -O-,
   provided that when R^{3c} is -(CH₂)ₚCHR^{3f}-NR^{N1}R^{N2}, X² in the formula (IV) is -O-, - NH- or -N(C₂₋₃ alkyl)-,
   n is 1 or 2,
   p is 1 or 2, and
   k is an integer of 0 to 2.
(4-4-1) The compound or a salt thereof in which R³ is a group selected from the group consisting of the formula (III), the formula (IV), the formula (V), the formula (VI), the formula (VII), the formula (VIII), the formula (IX), the formula (X), the formula (XI), the formula (XII), the formula (XIII), the formula (XIV) and the formula (XV) below, or R³ is a saturated or unsaturated 7-membered or 8-membered bridged heterocyclic group containing one or two nitrogen atoms optionally substituted with OH, wherein when the bridged heterocyclic group is substituted with OH, OH is attached only to a carbon atom that is an atom forming a bridged heterocyclic ring, 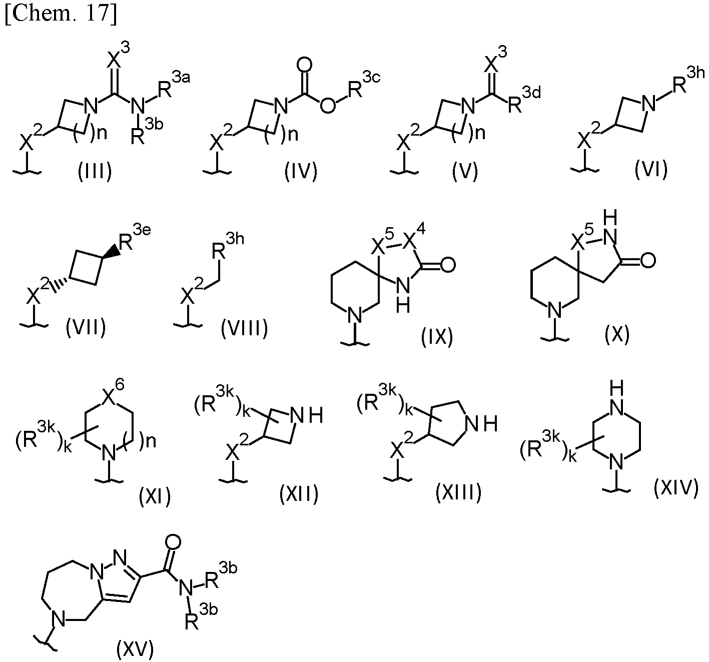
   R^{3a} is -(CH₂)ₚCHR^{3f}-NR^{N1}R^{N2}, -(CH₂)ₚCHR^{3f}-OR^{3g}; a 5-membered or 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkyl-OR^{3g}, C₁₋₃ alkyl-NR^{N1}R^{N2} and -NR^{N1}R^{N2};or C₃₋₆ cycloalkyl optionally substituted with a group selected from the group consisting of C₁₋₃ alkyl, C₁₋₃ alkyl-OR^{3g}, C₁₋₃ alkyl-NR^{N1}R^{N2}, -OR^{3g} and -NR^{N1}R^{N2},
   R^{3b} is H or C₁₋₃ alkyl,
   R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkyl-OR^{3g}, C₁₋₃ alkyl-NR^{N1}R^{N2} and -NR^{N1}R^{N2};or C₃₋₆ cycloalkyl optionally substituted with a group selected from the group consisting of C₁₋₃ alkyl, C₁₋₃ alkyl-OR^{3g}, C₁₋₃ alkyl-NR^{N1}R^{N2}, -OR^{3g} and -NR^{N1}R^{N2},
   R^{3e} is -O-C₂₋₃ alkyl-NR^{N1}R^{N2} or -NR^{N1}R^{N2},
   R^{3f} is H, F or C₁₋₃ alkyl,
   R^{3g} is H or C₁₋₃ alkyl,
   R^{3h} is optionally substituted 5-membered heteroaryl containing one to four hetero atoms selected from the group consisting of oxygen, sulfur and nitrogen, optionally substituted 6-membered heteroaryl containing one to three nitrogen atoms, or cyclobutyl optionally substituted with -NR^{N1}R^{N2},
   R^{N1} and R^{N2}, which are the same as or different from each other, are H or C₁₋₃ alkyl, or
   R^{N1} and R^{N2}, together with the nitrogen atom to which they are attached, optionally form an optionally substituted 4-membered to 6-membered saturated heterocyclic group containing one or two hetero atoms selected from the group consisting of oxygen, sulfur and nitrogen, or
   R^{3f} and R^{N1}, together with the carbon atom and the nitrogen atom to which they are attached, optionally form an optionally substituted 4-membered to 6-membered saturated heterocyclic group containing one or two hetero atoms selected from the group consisting of oxygen, sulfur and nitrogen,
   each R^{3k}, which is the same as or different from each other, is a group selected from the group consisting of OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, where R^{3k} is attached only to a carbon atom that is an atom forming a ring represented by the formula (XI), the formula (XII), the formula (XIII) or the formula (XIV),
   X² is -O-, -NH- or -N(C₁₋₃ alkyl)-,
   X³ is O or S,
   X⁴ is -CH₂- or -NH-,
   X⁵ is a bond, -CH₂- or C=O,
   X⁶ is -CH₂- or -O-,
   provided that when R^{3c} is -(CH₂)ₚCHR^{3f}-NR^{N1}R^{N2}, X² in the formula (IV) is -O-, - NH- or -N(C₂₋₃ alkyl)-,
   n is 1 or 2,
   p is 1 or 2, and
   k is an integer of 0 to 2.
(4-5) The compound or a salt thereof in which R³ is a group selected from the group consisting of the formula (III-a), the formula (IV-a), the formula (V-a), the formula (XIII-a) and the formula (XXXVI) below, 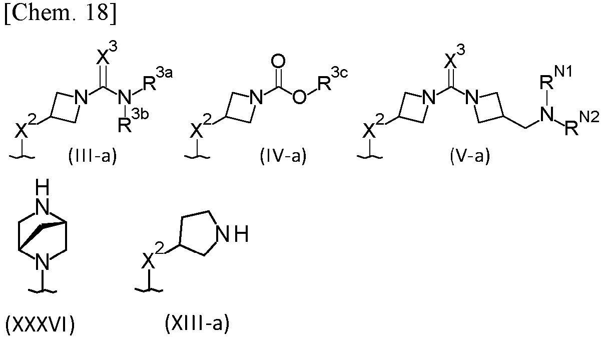
   R^{3a} is -(CH₂)ₚCHR^{3f}-NR^{N1}R^{N2},
   R^{3b} is H,
   R^{3c} is -(CH₂)ₚCHR^{3f}-NR^{N1}R^{N2} or C₄₋₆ cycloalkyl optionally substituted with - NR^{N1}R^{N2},
   R^{3f} is H,
   R^{N1} and R^{N2} are both C₁₋₃ alkyl,
   X² is -O-, -NH- or -N(C₁₋₃ alkyl)-,
   X³ is O,
   provided that when R^{3c} is -(CH₂)ₚCHR^{3f}-NR^{N1}R^{N2}, X² in the formula (IV-a) is -O-, - NH- or -N(C₂₋₃ alkyl)-, and
   p is 1.
(4-6) The compound or a salt thereof in which R³ is a group selected from the group consisting of the formula (III-a), the formula (IV-a), the formula (XIII-a) and the formula (XXXVI) below, 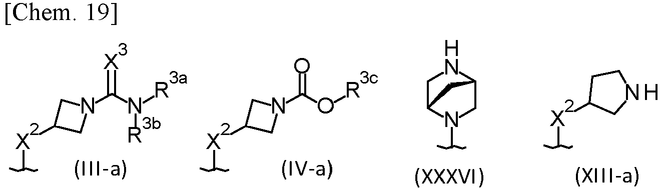
   R^{3a} is -(CH₂)ₚCHR^{3f}-NR^{N1}R^{N2},
   R^{3b} is H,
   R^{3c} is -(CH₂)ₚCHR^{3f}-NR^{N1}R^{N2},
   R^{3f} is H,
   R^{N1} and R^{N2} are both C₁₋₃ alkyl,
   X² is -O- or -N(C₁₋₃ alkyl)-,
   X³ is O,
   provided that when R^{3c} is -(CH₂)ₚCHR^{3f}-NR^{N1}R^{N2}, X² in the formula (IV) is -O- or - N(C₂₋₃ alkyl)-, and
   p is 1.
(5-1) The compound or a salt thereof in which R⁴ is optionally substituted C₁₋₆ alkyl, an optionally substituted 4-membered to 6-membered saturated heterocyclic group containing one or two hetero atoms selected from oxygen, sulfur and nitrogen, optionally substituted 5-membered heteroaryl containing one to four hetero atoms selected from oxygen, sulfur and nitrogen or optionally substituted 6-membered heteroaryl containing one to three nitrogen atoms.
(5-2) The compound or a salt thereof in which R⁴ is C₁₋₆ alkyl optionally substituted with OCH₃ or tetrahydropyranyl.
(5-3) The compound or a salt thereof in which R⁴ is C₁₋₃ alkyl optionally substituted with OCH₃ or tetrahydropyranyl.
(6-1) The compound or a salt thereof in which X¹ is a bond, -CH₂-, -O-, -S- or - NR^{4X}-, and
   R^{4X} is H or C₁₋₃ alkyl.
(6-2) The compound or a salt thereof in which X¹ is -O-.
(7-1) The compound or a salt thereof in which Y is phenylene optionally substituted with a group selected from the group consisting of F and Cl or pyridinediyl.
(7-2) The compound or a salt thereof in which Y is phenylene optionally substituted with F.
(7-3) The compound or a salt thereof in which Y is phenylene.
(8-1) The compound or a salt thereof in which L is -(L¹-L²-L³-L⁴-L⁵-L⁶-L⁷)-,
   L¹, L², L³, L⁴, L⁵, L⁶ and L⁷, which are the same as or different from each other, are groups selected from the group consisting of a bond, -O-, -S-, -NR^{L1}-, acetylene-1,2-diyl, optionally substituted azetidinediyl, optionally substituted pyrrolidinediyl, optionally substituted piperidinediyl, optionally substituted piperazinediyl, optionally substituted diazepanediyl, optionally substituted C₁₋₃ alkylene, a saturated or unsaturated 7-membered or 8-membered bridged heterocyclic divalent group containing one or two nitrogen atoms, a saturated 7-membered to 11-membered spiroheterocyclic divalent group containing two nitrogen atoms, a saturated 8-membered to 10-membered bicycloheterocyclic divalent group containing two nitrogen atoms, optionally substituted phenylene, optionally substituted pyridinediyl, C=O, S=O and S(=O)₂, and
   R^{L1} is H or C₁₋₃ alkyl.
(8-2) The compound or a salt thereof in which L is a group selected from the group consisting of the formula (XXXX-a), the formula (XXXX-b), the formula (XXXXI), the formula (XXXXII), the formula (XXXXIII), the formula (XXXXIV), the formula (XXXXV), the formula (XXXXVI), the formula (XXXXVII) and the formula (XXXXVIII) below, 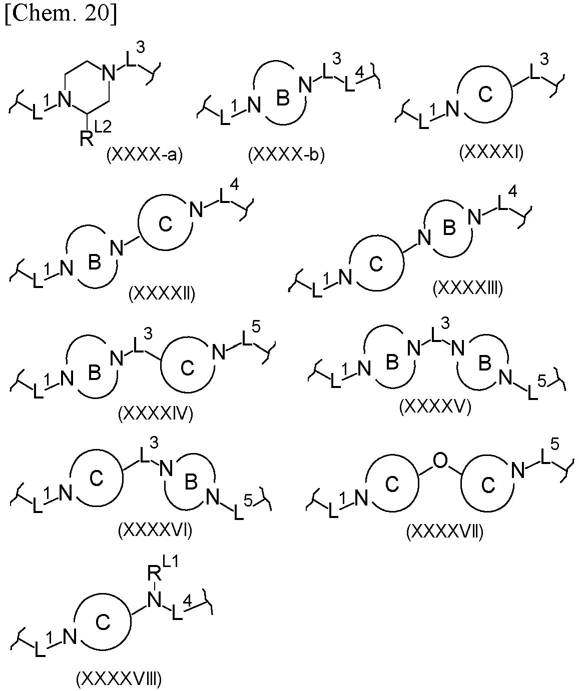
   L¹ is -(CH₂)-, C=O or S(=O)₂,
   L³, L⁴ and L⁵, which are the same as or different from each other, are a bond, -O-, C₁₋₃ alkylene or C=O,
   R^{L1} is H or C₁₋₃ alkyl,
   R^{L2} is C₁₋₃ alkyl or oxo,
   ring B is piperazine, diazepane, diazabicyclo[2.2.1]heptane, diazabicyclo[3.2.1]octane, diazaspiro[3.3]heptane, diazaspiro[3.4]octane, diazaspiro[3.5]nonane, diazaspiro[4.5]decane, diazaspiro[5.5]undecane or diazabicyclo[3.3.0]octane, and
   ring C is azetidine, pyrrolidine or piperidine.
(8-3) The compound or a salt thereof in which L is a group selected from the group consisting of the formula (XXXX-a), the formula (XXXX-b), the formula (XXXXII), the formula (XXXXIV) and the formula (XXXXVIII) below, 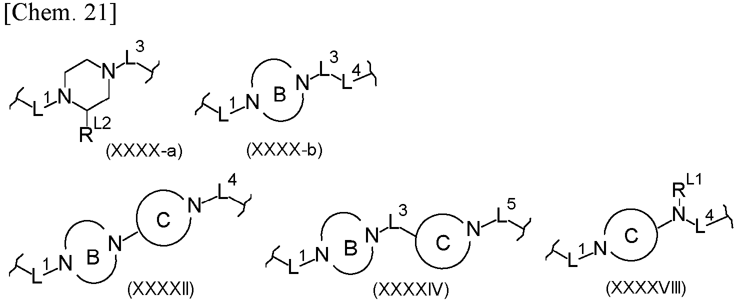
   L¹ is -(CH₂)- or C=O,
   L³, L⁴ and L⁵, which are the same as or different from each other, are a bond, -O-, C₁₋₃ alkylene or C=O,
   R^{L1} and R^{L2} are C₁₋₃ alkyl,
   ring B is piperazine, diazepane, diazabicyclo[3.2.1]octane, diazaspiro[3.5]nonane or diazabicyclo[3.3.0]octane, and
   ring C is azetidine or piperidine.
(8-4) The compound or a salt thereof in which L is a group selected from the group consisting of the formula (XXXX), the formula (XXXX-a), the formula (XXXXI), the formula (XXXXII), the formula (XXXXIII), the formula (XXXXIV), the formula (XXXXV), the formula (XXXXVI), the formula (XXXXVII) and the formula (XXXXVIII) below, 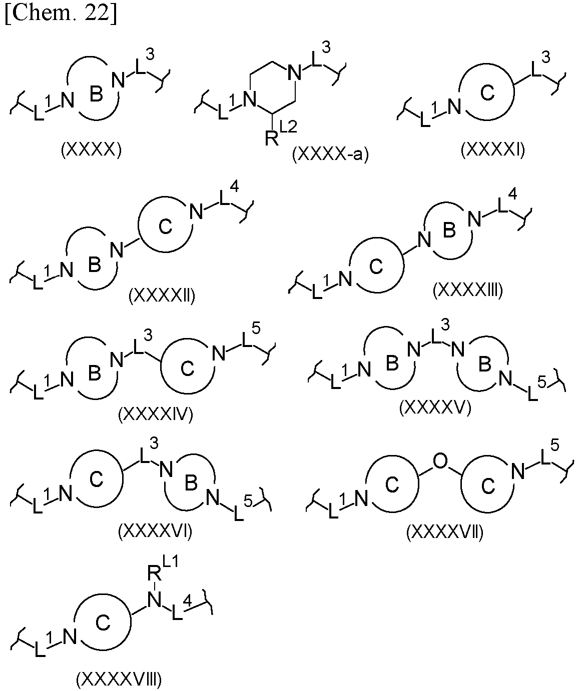
   L¹ is -(CH₂)-, C=O or S(=O)₂,
   L³, L⁴ and L⁵, which are the same as or different from each other, are -(CH₂)- or C=O,
   R^{L1} is H or C₁₋₃ alkyl,
   R^{L2} is C₁₋₃ alkyl or oxo,
   ring B is piperazine, diazepane, diazabicyclo[2.2.1]heptane, diazabicyclo[3.2.1]octane, diazaspiro[3.3]heptane, diazaspiro[3.4]octane, diazaspiro[3.5]nonane, diazaspiro[4.5]decane, diazaspiro[5.5]undecane or diazabicyclo[3.3.0]octane, and
   ring C is azetidine, pyrrolidine or piperidine.
(8-5) The compound or a salt thereof in which L is a group selected from the group consisting of the formula (XXXX), the formula (XXXX-a), the formula (XXXXI), the formula (XXXXII), the formula (XXXXIV) and the formula (XXXXVIII) below, 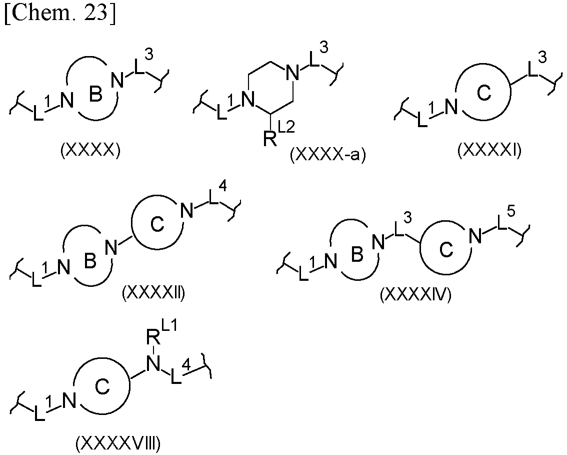
   L¹, L³, L⁴ and L⁵, which are the same as or different from each other, are -(CH₂)- or C=O,
   R^{L1} and R^{L2} are C₁₋₃ alkyl,
   ring B is piperazine, diazepane, diazabicyclo[3.2.1]octane, diazaspiro[3.5]nonane or diazabicyclo[3.3.0]octane, and
   ring C is azetidine or piperidine.
(9-1) The compound or a salt thereof in which Z is a group selected from the group consisting of the formula (XVI), the formula (XVII), the formula (XVIII), the formula (XIX), the formula (XX), the formula (XXI), the formula (XXII), the formula (XXIII), the formula (XXIV), the formula (XXV), the formula (XXVI), the formula (XXVII), the formula (XXVIII), the formula (XXIX), the formula (XXX), the formula (XXXI), the formula (XXXII), the formula (XXXIII), the formula (XXXIV) and the formula (XXXV) below, 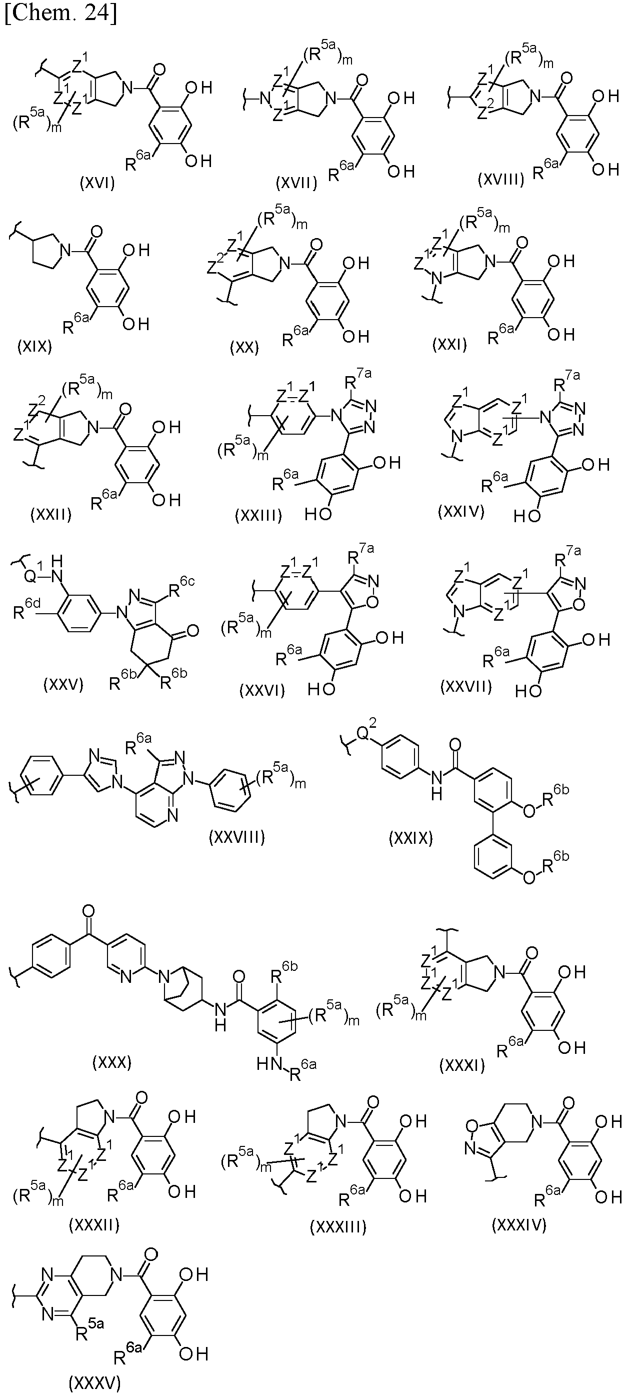
   each Z¹, which is the same as or different from each other, is CH or N,
   Z² is NH, NCH₃, O or S,
   each R^{5a}, which is the same as or different from each other, is H, halogen, C₁₋₃ alkyl or -(C=O)NH₂,
   R^{6a} is C₁₋₆ alkyl optionally substituted with F,
   each R^{6b}, which is the same as or different from each other, is C₁₋₃ alkyl,
   R^{6c} is C₁₋₃ alkyl optionally substituted with F,
   R^{6d} is -(C-O)NR^{N3}R^{N4},
   R^{N3} and R^{N4}, which are the same as or different from each other, are H or C₁₋₃ alkyl,
   R^{7a} is OH or -(C=O)NR^{N5}R^{N6},
   R^{N5} and R^{N6}, which are the same as or different from each other, are H, cyclopropyl or C₁₋₃ alkyl optionally substituted with F, and
   m is an integer of 0 to 2.
(9-2) The compound or a salt thereof in which Z is a group selected from the group consisting of the formula (XVI-a), the formula (XXIII-a), the formula (XIX) and the formula (XXXV) below, 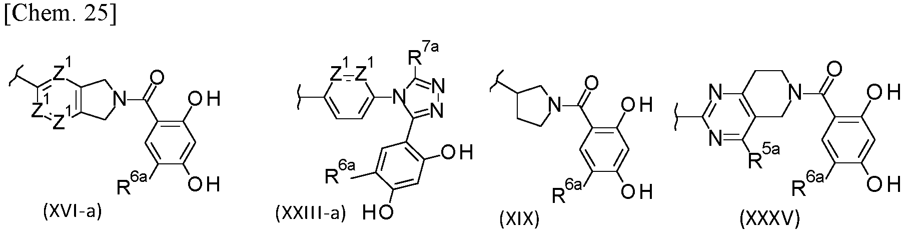
   each Z¹ is CH,
   R^{5a} is H,
   R^{6a} is C₁₋₃ alkyl,
   R^{7a} is OH or -(C=O)NR^{N5}R^{N6},
   R^{N5} is H, and
   R^{N6} is C₁₋₃ alkyl.
(9-3) The compound or a salt thereof in which Z is a group selected from the group consisting of the formula (XVI-a), the formula (XXIII-a), the formula (XIX) and the formula (XXXV) below, 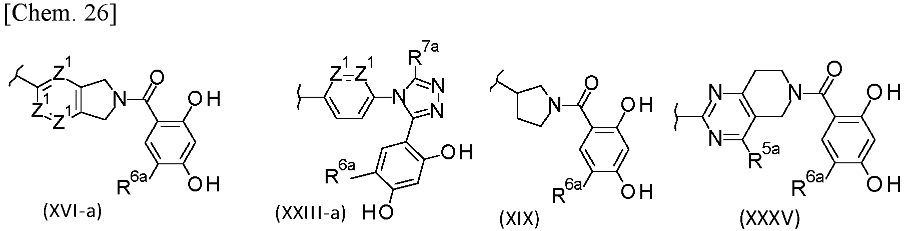
   each Z¹ is CH,
   R^{5a} is H,
   R^{6a} is isopropyl, and
   R^{7a} is OH.
(9-4) The compound or a salt thereof in which Z is a group selected from the group consisting of the formula (XVI), the formula (XVII), the formula (XVIII), the formula (XIX), the formula (XX), the formula (XXI), the formula (XXII), the formula (XXIII), the formula (XXIV), the formula (XXV), the formula (XXVI), the formula (XXVII), the formula (XXVIII), the formula (XXIX), the formula (XXX), the formula (XXXI), the formula (XXXII), the formula (XXXIII), the formula (XXXIV) and the formula (XXXV) below, 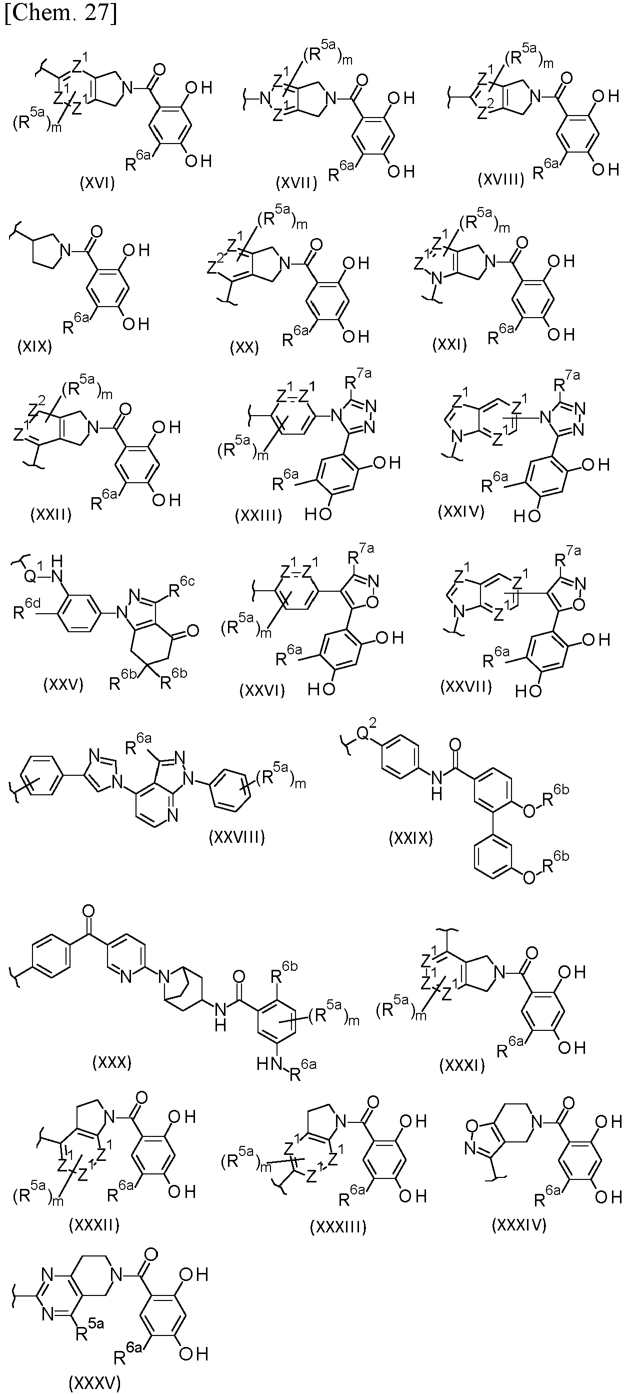
   each Z¹, which is the same as or different from each other, is CH or N,
   Z² is NH, NCH₃, O or S,
   each R^{5a}, which is the same as or different from each other, is halogen, C₁₋₃ alkyl or - (C=O)NH₂,
   R^{6a} is C₁₋₆ alkyl optionally substituted with F,
   each R^{6b}, which is the same as or different from each other, is C₁₋₃ alkyl,
   R^{6c} is C₁₋₃ alkyl optionally substituted with F,
   R^{6d} is -(C-O)NR^{N3}R^{N4},
   R^{N3} and R^{N4}, which are the same as or different from each other, are H or C₁₋₃ alkyl,
   R^{7a} is OH or -(C=O)NR^{N5}R^{N6},
   R^{N5} and R^{N6}, which are the same as or different from each other, are H, cyclopropyl or C₁₋₃ alkyl optionally substituted with F, and
   m is an integer of 0 to 2.
(9-5) The compound or a salt thereof in which Z is a group selected from the group consisting of the formula (XVI-a) and the formula (XXIII-a) below, 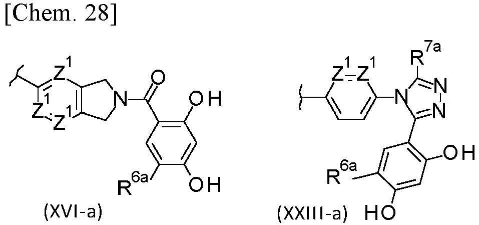
   each Z¹ is CH,
   R^{6a} is C₁₋₃ alkyl,
   R^{7a} is OH or -(C=O)NR^{N5}R^{N6},
   R^{N5} is H, and
   R^{N6} is C₁₋₃ alkyl.
(9-6) The compound or a salt thereof in which Z is a group selected from the group consisting of the formula (XVI-a) and the formula (XXIII-a),
   each Z¹ is CH,
   R^{6a} is isopropyl, and
   R^{7a} is OH.
(10) The compound or a salt thereof which is a combination of any two or more of the embodiments compatible with each other described in (1-1) to (9-6) above.

Specific examples of the combination described in (10) above include the following embodiments.
(11-1) The compound of the formula (I) or a salt thereof which is the combination of the embodiments of (1-1), (2-1), (3-1), (4-1), (5-1), (6-1), (7-1), (8-1) and (9-1) above.
(11-2) The compound of the formula (I) or a salt thereof which is the combination of the embodiments of (1-2), (2-2), (3-2), (4-2), (5-2), (6-2), (7-2), (8-2) and (9-2) above.
(11-3) The compound of the formula (I) or a salt thereof which is the combination of the embodiments of (1-2), (2-2), (3-2), (4-3), (5-3), (6-2), (7-3), (8-3) and (9-3) above.
(11-4) The compound of the formula (I) or a salt thereof which is the combination of the embodiments of (1-1), (2-1), (3-1), (4-4), (5-1), (6-1), (7-1), (8-1) and (9-4) above.
(11-5) The compound of the formula (I) or a salt thereof which is the combination of the embodiments of (1-2), (2-2), (3-2), (4-5), (5-2), (6-2), (7-2), (8-4) and (9-5) above.
(11-6) The compound of the formula (I) or a salt thereof which is the combination of the embodiments of (1-3), (2-2), (3-2), (4-6), (5-3), (6-2), (7-3), (8-5) and (9-6) above.

The compound of the formula (I) may have tautomers or geometrical isomers depending on the type of the substituent. In this specification, the compound of the formula (I) is sometimes described only as one of isomers, but the present invention includes isomers other than the above one and includes separated isomers or mixtures thereof.

In addition, the compound of the formula (I) may have an asymmetric carbon atom or an axial chirality and may have diastereomers based on them. The present invention includes separated diastereomers of the compound of the formula (I) or mixtures thereof.

Furthermore, the present invention also includes a pharmaceutically acceptable prodrug of the compound represented by the formula (I). The pharmaceutically acceptable prodrug is a compound having a group that can be converted into an amino group, a hydroxy group, a carboxyl group or the like by solvolysis or under physiological conditions. Examples of a prodrug-forming group include groups described in Prog. Med., 1985, 5, p.2157-2161 and "Pharmaceutical Research and Development", Vol. 7, Molecular Design, Hirokawa Shoten, 1990, p.163-198.

In addition, the salt of the compound of the formula (I) is a pharmaceutically acceptable salt of the compound of the formula (I) and may be an acid addition salt or a salt formed with a base depending on the type of the substituent. Examples thereof include salts shown in P. Heinrich Stahl, Handbook of Pharmaceutical Salts Properties, Selection, and Use, Wiley-VCH, 2008. Specific examples include an acid addition salt with an inorganic acid, such as hydrochloric acid, hydrobromic acid, hydroiodic acid, sulfuric acid, nitric acid or phosphoric acid, or with an organic acid, such as formic acid, acetic acid, propionic acid, oxalic acid, malonic acid, succinic acid, fumaric acid, maleic acid, lactic acid, malic acid, mandelic acid, tartaric acid, dibenzoiltartaric acid, ditoluoyltartaric acid, citric acid, methanesulfonic acid, ethanesulfonic acid, benzenesulfonic acid, p-toluenesulfonic acid, aspartic acid or glutamic acid, a salt with an inorganic metal, such as sodium, potassium, magnesium, calcium or aluminum, a salt with an organic base, such as methylamine, ethylamine or ethanolamine, a salt with various amino acids and amino acid derivatives, such as acetylleucine, lysine or ornithine, an ammonium salt and the like.

Furthermore, the present invention also includes various hydrates, solvates and crystal polymorphism substances of the compound of the formula (I) and a salt thereof.

The present invention also includes all the compounds of the formula (I) or salts thereof which are labeled with one or more pharmaceutically acceptable radioactive or nonradioactive isotopes. Examples of suitable isotopes used for isotopic labeling of the compound of the present invention include isotopes of hydrogen (²H, ³H and the like), carbon (¹¹C, ¹³C, ¹⁴C and the like), nitrogen (¹³N, ¹⁵N and the like), oxygen (¹⁵O, ¹⁷O, ¹⁸O and the like), fluorine (¹⁸F and the like), chlorine (³⁶Cl and the like), iodine (¹²³I, ¹²⁵I and the like) and sulfur (³⁵S and the like).

The isotope-labeled compound of the invention of the present application can be used for research and the like such as research on tissue distribution of drugs and/or substrates. For example, radioactive isotopes such as tritium (³H) and carbon 14 (¹⁴C) can be used for this purpose due to the easiness of labeling and the convenience of detection.

Substitution by a heavier isotope, for example, substitution of hydrogen by deuterium (2H), is therapeutically advantageous through the improvement of metabolic stability in some cases (for example, increase in the *in vivo* half-life, decrease in the required dose or decrease in the interaction between drugs).

Substitution by a positron-emitting isotope (11C, 18F, 15O, 13N or the like) can be used in a positron emission tomography (PET) test for testing occupancy of a substrate receptor.

The isotope-labeled compound of the present invention can be generally produced by a conventional method known to a person skilled in the art or by the same production methods as in the Examples or the Production Examples and the like using suitable reagents which are labeled with an isotope in place of unlabeled reagents.

### (Production method)

The compound of the formula (I) and a salt thereof can be produced by applying various known synthetic methods using characteristics based on the basic structure or the type of substituent thereof. Here, depending on the type of functional group, it is sometimes effective as a production technique to substitute the functional group with an appropriate protective group (a group that can be easily converted to the functional group) in the process from a raw material to an intermediate. Examples of the protective group include protective groups described in P. G. M. Wuts and T. W. Greene, "Greene's Protective Groups in Organic Synthesis", 5th edition, John Wiley & Sons Inc., 2014 and the like, and a group appropriately selected from the protective groups is used depending on the reaction conditions. In such a method, a reaction is carried out with the protective group introduced, and then the protective group is removed, as required, whereby a desired compound can be obtained.

In addition, a prodrug of the compound of the formula (I) can be produced similarly to the protective group by introducing a specific group in the process from a raw material to an intermediate or by further performing the reaction using the resulting compound of the formula (I). This reaction can be performed by applying a method known to a person skilled in the art, such as common esterification, amidation and dehydration.

Typical methods for producing the compound of the formula (I) will be described below. The production methods can also be carried out with reference to a reference attached to the description. Note that the production method of the present invention is not limited to the examples described below.

In this specification, the following abbreviations are sometimes used.

DMF: N,N-dimethylformamide, DMAc: N,N-dimethylacetamide, THF: tetrahydrofuran, MeCN: acetonitrile, MeOH: methanol, EtOH: ethanol, iPrOH: isopropyl alcohol, tBuOH: tert-butyl alcohol, DOX: 1,4-dioxane, DMSO: dimethyl sulfoxide, TEA: triethylamine, DIPEA: N,N-diisopropylethylamine, tBuOK: potassium tert-butoxide, PdCl₂(dppf)·CH₂Cl₂: [1,1'-bis(diphenylphosphino)ferrocene]palladium(II) dichloride·dichloromethane adduct, Pd/C: palladium on carbon, PyBOP: (benzotriazol-1-yloxy)tripyrrolidinophosphonium hexafluorophosphate, iPr₂O: diisopropyl ether, HATU: 1-[bis(dimethylamino)methylene]-1H-1,2,3-triazolo[4,5-b]pyridinium 3-oxide hexafluorophosphate, DABCO: 1,4-diazabicyclo[2.2.2]octane, TfOH: trifluoromethanesulfonic acid, COMU: N-[({[(1Z)-1-cyano-2-ethoxy-2-oxoethylidene]amino}oxy)(morpholin-4-yl)methylene]-N-methylmethanaminium hexafluorophosphate, NMM: N-methylmorpholin, CDI: 1,1'carbodiimidazole, NMO: N-methylmorpholine N-oxide, LHMDS: lithium bis(trimethylsilyl)amide, NHMDS: (sodium bis(trimethylsilyl)amide, DMEDA: N,N'-dimethylethylenediamine, Triton B: benzyltrimethylammonium hydroxide, Xantphos: 4,5-bis(diphenylphosphino)-9,9-dimethylxanthene, Ruphos: 2-dicyclohexylphosphino-2',6'-diisopropoxybiphenyl, Xphos: 2-dicyclohexylphosphino-2',4',6'-triisopropylbiphenyl, BINAP: 2,2'-bis(diphenylphosphino)-1,1'-binaphthyl.

### (Production Method 1)

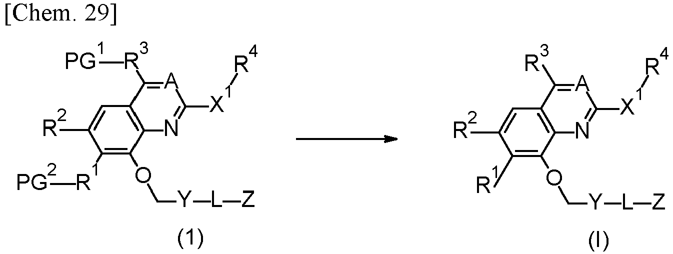

(In the formula, PG¹ represents a protective group of NH or OH contained in R³, and PG² represents a protective group of NH or OH contained in R¹ or a hydrogen atom. Note that -R³ - in PG¹-R³- represents a divalent group or a divalent group formed by removing hydrogen of NH or OH from R³, and -R¹- in PG²-R¹- represents a divalent group formed by removing hydrogen of NH or OH from R¹. The same shall apply hereinafter.)

The compound of the formula (I) can be obtained by subjecting a compound (1) to deprotection reaction conditions under acidic conditions. Here, examples of the protective group which can be removed under acidic conditions include a tert-butoxycarbonyl group, a triphenylmethyl group, a tetrahydro-2H-pyran-2-yl group, a methoxymethyl group, a dimethylmethanediyl group, a tert-butylsulfinyl group and the like.

This reaction is performed by stirring the compound (1) using a deprotection reagent in an equivalent amount or an excess equivalent amount to the compound (1) in a solvent inactive for the reaction under cooling to under reflux with heat, generally for 0.1 hours to 5 days. Examples of the deprotection reagent used here include, but are not particularly limited to, acids such as hydrogen chloride (DOX solution), trifluoroacetic acid, methanesulfonic acid, phosphoric acid, p-toluenesulfonic acid and trifluoromethanesulfonic acid and a mixture thereof. In particular, when the deprotection reagent is trifluoroacetic acid, performing the deprotection reaction in the presence of a cation scavenger, such as triisopropylsilane, is sometimes advantageous for smoothly promoting the reaction.

Examples of the solvent used here include, but are not particularly limited to, an alcohol, such as MeOH or EtOH, a halogenated hydrocarbon, such as dichloromethane, 1,2-dichloromethane or chloroform, an ether, such as diethyl ether, THF, DOX or dimethoxyethane, DMF, DMSO, MeCN, TfOH or water and a mixture thereof.

By selecting a protective group, deprotection can also be performed by a catalytic hydrogenation reaction or under basic conditions. Examples of the protective group which can be removed by a catalytic hydrogenation reaction include a benzyl group, a p-methoxybenzyl group, a benzyloxycarbonyl group and the like. Moreover, deprotection can also be performed with a fluoride ion source such as tetra-n-butylammonium fluoride. Examples of the protective group include a tert-butyl(dimethyl)silyl group, a (trimethylsilyl)ethoxymethyl group and the like. Examples of the protective group which can be removed under basic conditions include an acetyl group, a trifluoroacetyl group, a benzoyl group and the like. Moreover, the deprotection can also be performed in stages by selecting protective groups which can be removed under different deprotection conditions as PG¹ and PG².

For example, the following can be referred as a reference about this reaction.

Wuts (P. G. M. Wuts) and Greene (T. W. Greene), "Greene's Protective Groups in Organic Synthesis, (5th edition, 2014)".

Note that, when the compound (1) as a raw material has an axial chirality, a stereoisomer which is obtained by once separating the compound (1) may be used for this reaction.

### (Raw Material Synthesis 1)

This production method shows a synthesis method of the compound (1)-1 and the compound (1)-2 included in the compound (1), which is a raw material of Production Method 1.

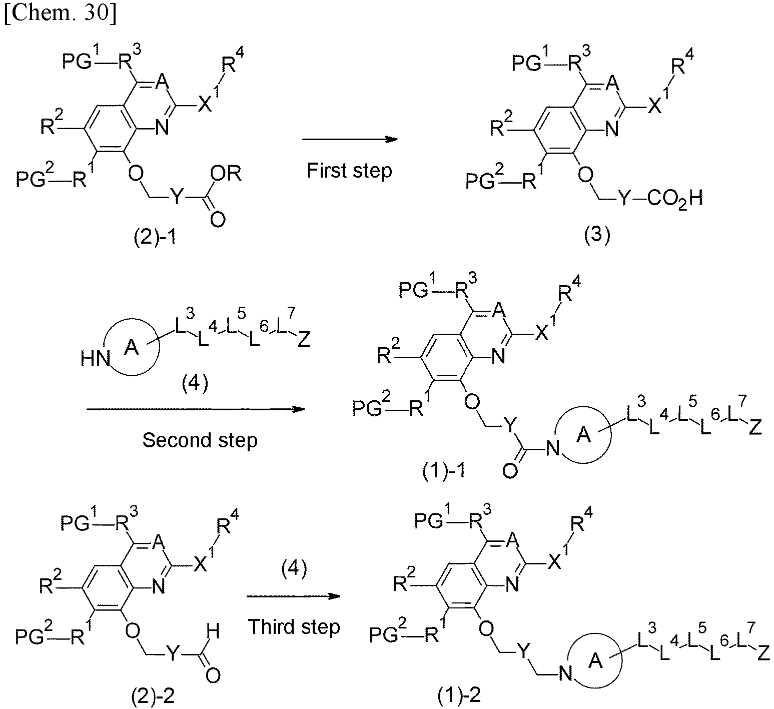

(In the formula, ring A represents piperazine optionally substituted with C₁₋₃ alkyl, ring B or ring C. R represents a C₁₋₃ alkyl group. The same shall apply hereinafter.)

### (First step)

This step is a step of producing a compound (3) by hydrolysis of the compound (2)-1 contained in the raw material compound (2).

This reaction is performed by stirring the compound (2)-1 and a hydrolysis reagent in an equivalent amount or an excess equivalent amount to the compound (2)-1 in a solvent inactive for the reaction under cooling to under reflux with heat, generally for 1 hours to 5 days. Examples of the hydrolysis reagent used here include, but are not particularly limited to, an aqueous sodium hydroxide solution, an aqueous potassium hydroxide solution, an aqueous lithium hydroxide solution, trimethyltin hydroxide and the like. Examples of the solvent include, but are not particularly limited to, an alcohol, such as methanol, ethanol or n-propanol, an ether-based solvent, such as tetrahydrofuran, diethyl ether or 1,4-dioxane, a halogenated hydrocarbon, such as dichloromethane, 1,2-dichloroethane or chloroform, water and a mixture thereof.

### (Second step)

This step is a method for producing a compound (1)-1 by an amidation reaction of the compound (3) and the compound (4).

In this reaction, the compound (3) and the compound (4) are used in an equivalent amount or with one in an excess equivalent amount, and the mixture of the compounds is stirred in the presence of a condensing agent, in a solvent inactive for the reaction, from under cooling to under heating, preferably at -20°C to 60°C, generally for 0.1 hours to 5 days. Examples of the solvent include, but are not particularly limited to, an aromatic hydrocarbon, such as toluene, an ether, such as THF or DOX, a halogenated hydrocarbon, such as dichloromethane, an alcohol, N,N-dimethylformamide, DMSO, ethyl acetate, MeCN and a mixture thereof. Examples of the condensing agent include HATU, PyBOP, 1-(3-dimethylaminopropyl)-3-ethylcarbodiimide or the hydrochloride thereof, N,N'-dicyclohexylcarbodiimide (DCC), CDI, diphenylphosphoryl azide (DPPA) and the like. Use of an additive (for example, 1-hydroxybenzotriazole) is sometimes preferred for the reaction. Performing the reaction in the presence of an organic base, such as TEA, DIPEA and NMM, or an inorganic base, such as potassium carbonate, sodium carbonate and potassium hydroxide, is sometimes advantageous for smoothly promoting the reaction.

Alternatively, a method in which the compound (3) is converted into a reactive derivative, which is then subjected to an acylation reaction, can be used. Examples of the reactive derivative of a carboxylic acid include an acid halogenation product obtained by a reaction with a halogenating agent, such as phosphorus oxychloride and thionyl chloride, a mixed acid anhydride obtained by a reaction with isobutyl chloroformate or the like, an active ester obtained by condensation with 1-hydroxybenzotriazole or the like. The reaction of the reactive derivative and the compound (4) can be performed in a solvent inactive for the reaction, such as a halogenated hydrocarbon, an aromatic hydrocarbon and an ether, from under cooling to under heating, preferably at -20°C to 120°C.

### [Reference]

S. R. Sandler and W. Karo, "Organic Functional Group Preparations", 2nd edition, Vol. 1, Academic Press Inc., 1991

The Chemical Society of Japan, "Jikken Kagaku Koza (Courses in Experimental Chemistry) (5th edition)", Vol. 16 (2005) (Maruzen)

### (Third step)

This step is a step of producing a compound (1)-2 by a reductive amination reaction of the compound (2)-2 contained in the raw material compound (2) and the compound (4).

This reaction is performed by stirring the compound (2)-2 and the compound (4) in an equivalent amount or with one in an excess equivalent amount in the presence of a reducing agent and acetic acid, in a solvent inactive for the reaction, from under ice-bath cooling to room temperature, generally for 0.1 hour to 5 days. Examples of the reducing agent used here include, but are not particularly limited to, sodium triacetoxyborohydride (NaBH(OAc)₃), 2-picoline borane, sodium cyanoborohydride (NaBH₃CN) and the like. Examples of the solvent used here include, but are not particularly limited to, a halogenated hydrocarbon, such as dichloromethane, dichloroethane or chloroform, an ether-based solvent, such as tetrahydrofuran, diethyl ether or 1,4-dioxane, an alcohol-based solvent, such as methanol or ethanol, acetonitrile and the like.

### (Raw Material Synthesis 2)

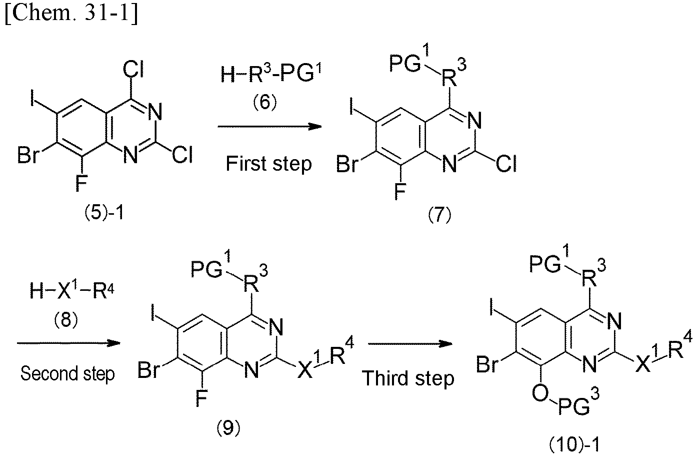

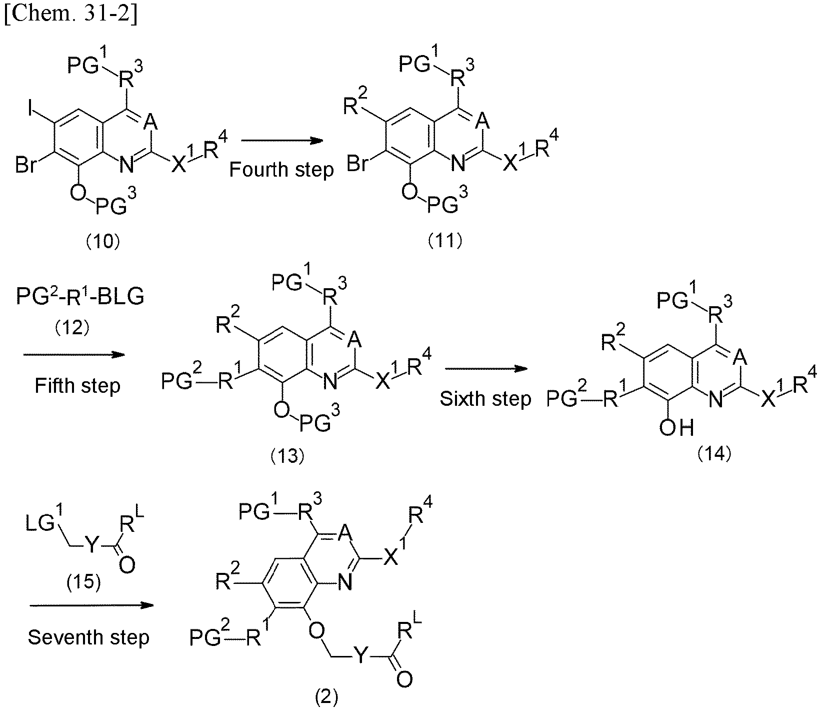

(In the formulae, PG³ represents a protective group of OH, LG¹ represents a leaving group, and BLG represents a boronic acid group, a boronic acid group protected with a protective group of boronic acid such as a boronic acid pinacol ester group or a trifluoroboric acid salt group (hereinafter sometimes described as a boronic acid group or the like). Examples of the leaving group shown here include Cl, Br, a methanesulfonyloxy group, p-toluenesulfonyloxy group and the like. R^{L} represents OR or H.)

This production method is a first method for producing a raw material compound (2).

### (First step)

This step is a method for producing a compound (7) by an ipso substitution reaction of a compound (5)-1 and a compound (6).

In this reaction, the compound (5)-1 and the compound (6) are used in an equal amount or with one compound thereof in an excess amount, and the mixture of the compounds is stirred in a solvent inactive for the reaction or with no solvent, from under cooling to under reflux with heat, preferably at 0°C to 80°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, DMF, DMAc, DMSO, ethyl acetate, MeCN and a mixture thereof. Performing the reaction in the presence of an organic base, such as TEA, DIPEA, N-methylmorpholine (NMM), 1,4-diazabicyclo[2.2.2]octane (DABCO) or tBuOK, or an inorganic base, such as sodium hydride, potassium carbonate, sodium carbonate or cesium carbonate, is sometimes advantageous for smoothly promoting the reaction.

Moreover, the compound (7) can be produced by a catalytic hydrogenation reaction of the compound obtained by a Mizoroki-Heck reaction of the compound (5)-1 and the compound (6).

### (Second step)

This step is a method for producing a compound (9) by an ipso substitution reaction of the compound (7) and a compound (8).

The reaction conditions are the same as in the first step of the Raw Material Synthesis 2.

Moreover, the compound (9) can be produced by the Negishi coupling of a compound in which a hydrogen atom of the compound (8) is converted to halogen and the compound (7).

### (Third step)

This step is a method for producing a compound (10)-1 by an ipso substitution reaction of the compound (9) and PG³-OH.

Examples of the PG³-OH used here include benzyl alcohol, p-methoxybenzyl alcohol and 1-phenylethanol.

The reaction conditions are the same as in the first step of the Raw Material Synthesis 2.

### (Fourth step)

This step is a method for producing a compound (11) by a Suzuki-Miyaura coupling reaction of the compound (10) including both of the compound (10)-1 obtained in the third step of this synthesis method and the compound (10)-2 obtained in (Raw Material Synthesis 9) described below and a boronic acid derivative composed of a R²-boronic acid group or the like. Examples of the boronic acid group or the like used here include, but are not particularly limited to, a boronic acid group, a boronic acid ester group, a boronic acid pinacol ester group, a triol borate salt group and a trifluoroboric acid salt group.

In this reaction, the compound (10) and the boronic acid derivative composed of the R²-boronic acid group or the like are used in an equal amount or with one compound thereof in an excess amount, and the mixture of the compounds is stirred in a solvent inactive for the reaction, in the presence of a base and a palladium catalyst, from at room temperature to under reflux with heat, preferably at 20°C to 140°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, an alcohol, such as MeOH, EtOH, isopropyl alcohol, butanol or amyl alcohol, DMF, DMSO, MeCN, 1,3-dimethylimidazolidin-2-one, water and a mixture thereof. Examples of the base include inorganic bases, such as tripotassium phosphate, sodium carbonate, potassium carbonate, sodium hydroxide and barium hydroxide. Examples of the palladium catalyst include tetrakis(triphenylphosphine)palladium, bis(triphenylphosphine)palladium(II) dichloride, [1,1'-bis(diphenylphosphino)ferrocene]palladium(II) dichloride·dichloromethane additive, (1E,4E)-1,5-diphenylpenta-1,4-dien-3-one/palladium (3:2), (2-dicyclohexylphosphino-2',6'-diisopropoxy-1,1'-biphenyl)[2-(2'-amino-1,1'-biphenyl)]palladium(II) methanesulfonate, palladium(II) acetate and the like. Performing the reaction in the presence of a ligand, such as dicyclohexyl(2',6'-dimethoxybiphenyl-2-yl)phosphine, dicyclohexyl(2',6'-diisopropoxy-[1,1'-biphenyl]-2-yl)phosphine or 1,1'-bis(diphenylphosphino)ferrocene is sometimes advantageous for smoothly promoting the reaction. In addition, heating the mixture by microwave irradiation is sometimes advantageous for smoothly promoting the reaction.

### [Reference]

J. Am. Chem. Soc., 2005, 127, p.4685-4696
Org. Lett. 2011, 13, p.3948-3951
Org. Lett. 2012, 14, p. 1278-1281

The compound (11) (where R² is hydrogen) can be produced by a dehydrogenation reaction of the compound (10) with a Pd catalyst and a reducing agent.

### [Reference]

J. Org. Chem., 1977, 42, p.3491-3494
Tetrahedron Letters 2013, 54, 5207-5210

### (Fifth step)

This step is a method for producing a compound (13) by a Suzuki-Miyaura coupling reaction of the compound (11) and a compound (12).

The reaction conditions are the same as in the fourth step of the Raw Material Synthesis 2.

When the compound (13) has an axial chirality, the compound (13) is obtained as a mixture of stereoisomers, and each stereoisomer can be isolated by separation using a common separation operation, for example, ODS column chromatography or silica gel column chromatography.

### (Sixth step)

This step is a method for producing a compound (14) by deprotection by a catalytic hydrogenation reaction of the compound (13).

This reaction can be performed by stirring the compound (13) under hydrogen atmosphere, from under normal pressure to under increased pressure, in a solvent inactive for the reaction, such as MeOH, EtOH or ethyl acetate, in the presence of a metal catalyst, from under cooling to under heating, preferably at room temperature, for 1 hour to 5 days. As the metal catalyst, a palladium catalyst, such as Pd/C or palladium black, a platinum catalyst, such as a platinum plate or platinum oxide, a nickel catalyst, such as reduced nickel or Raney nickel, or the like is used.

### (Seventh step)

This step is a method for producing the compound (2) by a reaction of the compound (14) and a compound (15).

This reaction is performed by reacting a mixture of the compound (14) and the compound (15) in an equal amount or with one compound thereof in an excess amount in the presence of a base, in a solvent inactive for the reaction, from under cooling to under reflux with heat, preferably at 0°C to 80°C, generally for 0.1 hours to 5 days. The solvent used here is not particularly limited, and examples thereof include an aromatic hydrocarbon, such as benzene, toluene or xylene, an alcohol, such as MeOH or EtOH, an ether, such as diethyl ether, THF, DOX or 1,2-dimethoxyethane, a halogenated hydrocarbon, such as dichloromethane, 1,2-dichloroethane or chloroform, DMF, DMSO, ethyl acetate, MeCN and a mixture thereof. Examples of the base include, but are not particularly limited to, an organic base, for example, such as TEA, DIPEA, 1,8-diazabicyclo[5.4.0]-7-undecene, n-butyllithium or tBuOK, and an inorganic base, such as sodium hydroxide, sodium carbonate, potassium carbonate, cesium carbonate or sodium hydride. Performing the reaction in the presence of a phase transfer catalyst, such as tetra-n-butylammonium chloride, is sometimes advantageous.

For example, the following can be referred as a reference about this reaction.

The Chemical Society of Japan, "Jikken Kagaku Koza (Courses in Experimental Chemistry)", 5th edition, Vol. 14, Maruzen, 2005

The compound (2) sometimes has an axial chirality and is obtained as a mixture of stereoisomers, and each stereoisomer can be isolated by subjecting the compound (2) in which PG² is a protective group or a compound obtained by subjecting the compound (2) to a deprotection reaction to separation using a common separation operation, for example, ODS column chromatography or silica gel column chromatography.

The reaction conditions for the deprotection reaction used here are the same as in the step described in the Production Method 1.

Moreover, the compound (15) in which LG¹ is halogen can be produced by halogenation of a compound in which the moiety corresponding to LG¹ is a hydroxy group. Examples of the halogenating agent used here include, but are not particularly limited to, thionyl chloride, phosphorus oxychloride, hydrobromic acid, phosphorus tribromide and the like.

For example, the following can be referred as a reference about this reaction.

The Chemical Society of Japan, "Jikken Kagaku Koza (Courses in Experimental Chemistry)", 5th edition, Vol. 13, Maruzen, 2004

The compound (15) in which LG¹ is a sulfonyloxy group can be produced by sulfonylation of a compound in which the moiety corresponding to LG¹ is a hydroxy group in the presence of a base. Examples of the sulfonylating reagent used here include, but are not particularly limited to, for example, methanesulfonylchloride, p-toluenesulfonylchloride, methanesulfonic anhydride and the like. Examples of the base include, but are not particularly limited to, for example, TEA, DIPEA, pyridine, tetramethylethylenediamine and the like.

For example, the following can be referred as a reference about this reaction.
Synthesis 1999, 9, p.1633-1636

In this step, the compound (2) can be produced by a Mitsunobu reaction of a compound in which the moiety corresponding to LG¹ of the compound (15) is a hydroxy group and the compound (14).

For example, the following can be referred as a reference about the Mitsunobu reaction here.
Chem. Asian J. 2007, 2, p.1340-1355

### (Raw Material Synthesis 3)

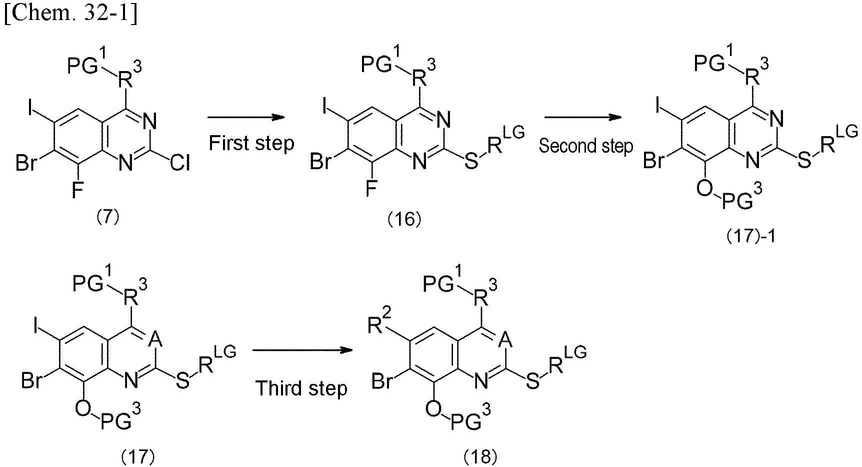

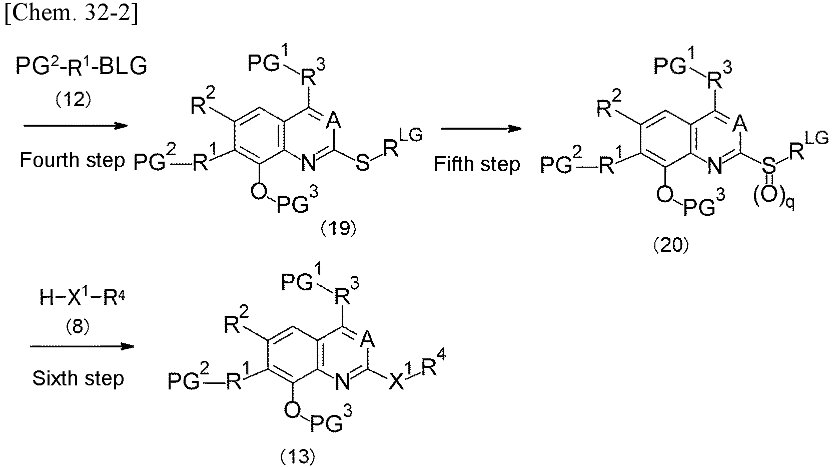

(In the formulae, R^{LG} represents a C₁₋₁₂ alkyl group, and q represents 1 or 2.)

This production method is a second method for producing the raw material compound (13).

### (First step)

This step is a method for producing a compound (16) by an ipso substitution reaction of the compound (7) and R^{LG}-SH. Examples of the R^{LG}-SH used here include C₁₋₁₂ alkylthiols, for example, ethanethiol and dodecanethiol.

The reaction conditions are the same as in the first step of the Raw Material Synthesis 2.

### (Second step)

This step is a method for producing a compound (17)-1 by an ipso substitution reaction of the compound (16) and PG³-OH. Examples of the PG³-OH used here include benzyl alcohol, p-methoxybenzyl alcohol and 1-phenylethanol.

The reaction conditions are the same as in the first step of the Raw Material Synthesis 2.

### (Third step)

This step is a method for producing a compound (18) by a Suzuki-Miyaura coupling reaction of the compound (17) including both of the compound (17)-1 obtained in the second step of this synthesis method and the compound (17)-2 obtained in (Raw Material Synthesis 8) described below and a boronic acid derivative composed of a R²-boronic acid group or the like.

The reaction conditions are the same as in the fourth step of the Raw Material Synthesis 2.

The compound (18) (where R² is hydrogen) can be produced by a dehydrogenation reaction of the compound (17) with a Pd catalyst and a reducing agent.

### [Reference]

J. Org. Chem., 1977, 42, p.3491-3494
Tetrahedron Letters 2013, 54, 5207-5210

### (Fourth step)

This step is a method for producing a compound (19) by a Suzuki-Miyaura coupling reaction of the compound (18) and a compound (12).

The reaction conditions are the same as in the fourth step of the Raw Material Synthesis 2.

The compound (19) sometimes has an axial chirality and is obtained as a mixture of stereoisomers, and each stereoisomer can be isolated by subjecting the compound (19) in which PG¹⁹ is a protective group or a compound obtained by subjecting the compound (19) to a deprotection reaction to separation using a common separation operation, for example, ODS column chromatography or silica gel column chromatography.

The reaction conditions for the deprotection reaction used here are the same as in the step described in the Production Method 1.

Examples of the protective group of PG² which is subsequently converted include a tetrahydro-2H-pyran-2-yl group and the like.

### (Fifth step)

This step is a method for producing a compound (20) by an oxidation reaction of the compound (19).

In this reaction, the compound (19) is treated with an oxidant in an equal amount or in an excess amount in a solvent inactive for the reaction, from under cooling to under heating, preferably at -20°C to 80°C, generally for 0.1 hours to 3 days. In this reaction, oxidation with m-chloroperbenzoic acid, perbenzoic acid, peracetic acid, sodium hypochlorite or hydrogen peroxide is suitably used. Examples of the solvent include an aromatic hydrocarbon, an ether, a halogenated hydrocarbon, such as dichloromethane, DMF, DMSO, ethyl acetate, MeCN and a mixture thereof. Other examples of the oxidant include cumene hydroperoxide, Oxone, active manganese dioxide, chromic acid, potassium permanganate, sodium periodate and the like.

### [Reference]

The Chemical Society of Japan, "Jikken Kagaku Koza (Courses in Experimental Chemistry)", 5th edition, Vol. 17, Maruzen, 2004

### (Sixth step)

This step is a method for producing the compound (13) by an ipso substitution reaction of the compound (20) and a compound (8).

The reaction conditions are the same as in the first step of the Raw Material Synthesis 2.

When the compound (13) has an axial chirality, the compound (13) is obtained as a mixture of stereoisomers, and each stereoisomer can be isolated by separation using a common separation operation, for example, ODS column chromatography or silica gel column chromatography.

### (Raw Material Synthesis 4)

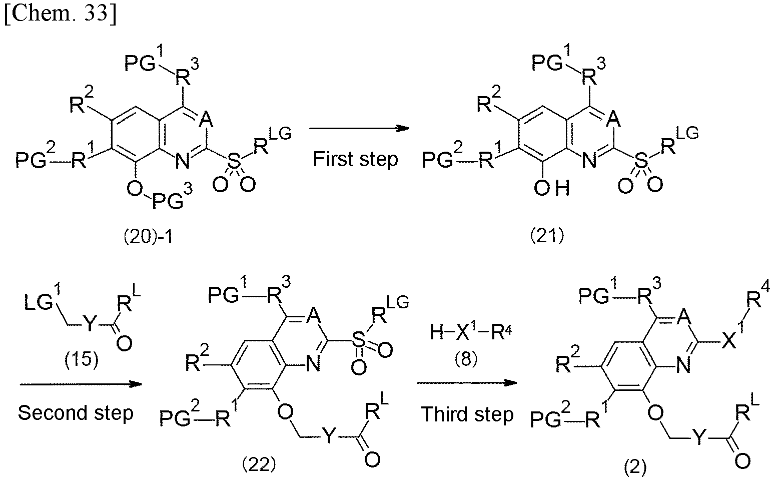

The production method is the second method for producing a raw material compound (2).

### (First step)

This step is a method for producing a compound (21) by deprotection by a catalytic hydrogenation reaction of the compound (20)-1.

The reaction conditions are the same as in the sixth step of the Raw Material Synthesis 2.

### (Second step)

This step is a method for producing a compound (22) by a reaction of the compound (21) and a compound (15).

The reaction conditions are the same as in the seventh step of the Raw Material Synthesis 2.

### (Third step)

This step is a method for producing the compound (2) by an ipso substitution reaction of the compound (22) and a compound (8).

The reaction conditions are the same as in the first step of the Raw Material Synthesis 2.

The compound (2) sometimes has an axial chirality and is obtained as a mixture of stereoisomers, and each stereoisomer can be isolated by subjecting the compound (2) in which PG² is a protective group or a compound obtained by subjecting the compound (2) to a deprotection reaction to separation using a common separation operation, for example, ODS column chromatography or silica gel column chromatography.

The reaction conditions for the deprotection reaction used here are the same as in the step described in the Production Method 1.

### (Raw Material Synthesis 5)

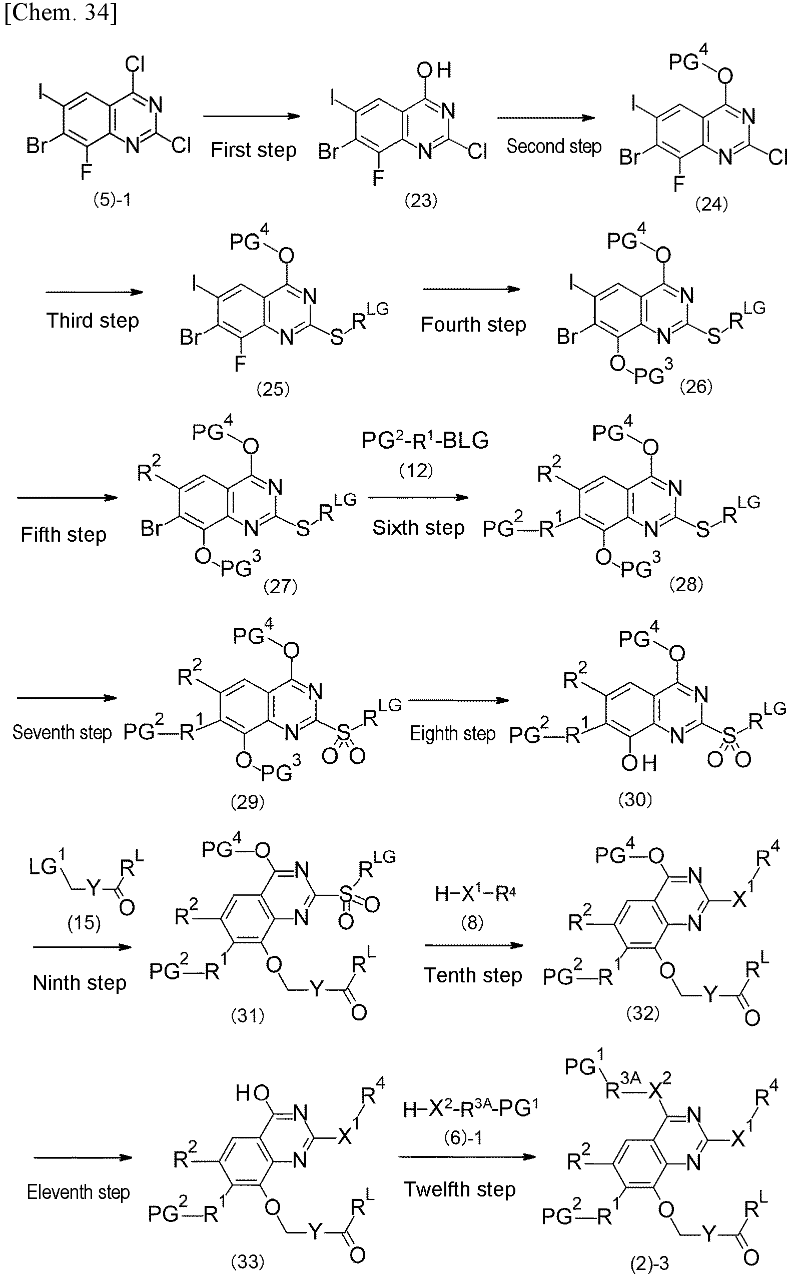

(In the formula, PG⁴ represents a tert-butyl group, and R^{3A} represents a substituent that is attached to X² represented by the formula (III), the formula (IV), the formula (V), the formula (VI), the formula (VII), the formula (VIII), the formula (XII) or the formula (XIII). In the formula, PG¹ represents a protective group of NH or OH contained in R^{3A}.)

The production method is a method for producing a compound (2)-3 included in the raw material compound (2).

### (First step)

This step is a method for producing a compound (23) by hydrolysis of the compound (5)-1.

This reaction is performed by stirring the compound (5)-1 under cooling to under reflux with heat, generally for 0.1 hours to 5 days. Examples of the solvent used here include, but are not particularly limited to, an alcohol, acetone, DMF, THF and the like. In addition, a mixed solvent of the above solvent and water is sometimes suitable for the reaction. Examples of the hydrolysis reagent include, but are not particularly limited to, an aqueous sodium hydroxide solution, an aqueous potassium hydroxide solution and the like.

For example, the following can be referred as a reference about this reaction.

The Chemical Society of Japan, "Jikken Kagaku Koza (Courses in Experimental Chemistry) (5th edition)", Vol. 16 (2005) (Maruzen)
Angew. Chem. Int. Ed. 2005, 44, p.1378-1382.

### (Second step)

This step is a method for producing a compound (24) by protection of a hydroxy group of the compound (23) with the tert-butyl group.

This reaction is performed by stirring the compound (23) under cooling to under reflux with heat, generally for 0.1 hours to 5 days. Examples of the solvent used here include, but are not particularly limited to, an ether, such as THF or DOX, a halogenated hydrocarbon, such as dichloromethane, tBuOH, DMF and the like. Examples of the tert-butyl protection reagent include, but are not particularly limited to, isobutene, 2-tert-butyl-1,3-diisopropylisourea and the like.

Moreover, the compound (24) can be produced by a dehydration condensation reaction of the compound (23) and tBuOH.

For example, the following can be referred as a reference about this reaction.
P. G. M. Wuts and T. W. Greene, "Greene's Protective Groups in Organic Synthesis", 5th edition, John Wiley & Sons Inc., 2014
Org. Lett., 2012, 14, 17, p.4678-4681

### (Third step)

This step is a method for producing a compound (25) by an ipso substitution reaction of the compound (24) and R^{LG}-SH.

The reaction conditions are the same as in the first step of the Raw Material Synthesis 3.

### (Fourth step)

This step is a method for producing a compound (26) by an ipso substitution reaction of the compound (25) and PG³-OH.

The reaction conditions are the same as in the third step of the Raw Material Synthesis 2.

### (Fifth step)

This step is a method for producing a compound (27) by a Suzuki-Miyaura coupling reaction of the compound (26) and a boronic acid derivative composed of a R²-boronic acid group or the like.

The reaction conditions are the same as in the fourth step of the Raw Material Synthesis 2.

The compound (27) (where R² is hydrogen) can be produced by a dehydrogenation reaction of the compound (26) with a Pd catalyst and a reducing agent.

### [Reference]

J. Org. Chem., 1977, 42, p.3491-3494
Tetrahedron Letters 2013, 54, 5207-5210

### (Sixth step)

This step is a method for producing a compound (28) by a Suzuki-Miyaura coupling reaction of the compound (27) and a compound (12).

The reaction conditions are the same as in the fifth step of the Raw Material Synthesis 2.

### (Seventh step)

This step is a method for producing a compound (29) by an oxidation reaction of the compound (28).

The reaction conditions are the same as in the fifth step of the Raw Material Synthesis 3.

When the compound (29) has an axial chirality, the compound (29) may be obtained as a mixture of stereoisomers, and each stereoisomer can be isolated by separation using a common separation operation, for example, ODS column chromatography or silica gel column chromatography or by fractional crystallization.

In addition, the compound (29) sometimes converts PG² to another protective group, followed by a deprotection reaction, so that the compound (29) can be deprotected under different conditions from a protective group PG¹ to be introduced later.

The reaction conditions for the deprotection reaction used here are the same as in the step described in the Production Method 1.

Examples of the protective group of PG² which is subsequently converted include a tetrahydro-2H-pyran-2-yl group and the like.

For example, the following can be referred as a reference about this reaction.
P. G. M. Wuts and T. W. Greene, "Greene's Protective Groups in Organic Synthesis", 5th edition, John Wiley & Sons Inc., 2014

### (Eighth step)

This step is a method for producing a compound (30) by deprotection by a catalytic hydrogenation reaction of the compound (29).

The reaction conditions are the same as in the sixth step of the Raw Material Synthesis 2.

### (Ninth step)

This step is a method for producing a compound (31) by a reaction of the compound (30) and a compound (15).

The reaction conditions are the same as in the seventh step of the Raw Material Synthesis 2.

### (Tenth step)

This step is a method for producing the compound (32) by an ipso substitution reaction of the compound (31) and a compound (8).

The reaction conditions are the same as in the sixth step of the Raw Material Synthesis 3.

### (Eleventh step)

This step is a method for producing a compound (33) by subjecting the compound (32) to a deprotection reaction.

The reaction conditions are the same as in the step described in the Production Method 1.

### (Twelfth step)

This step is a method for producing a compound (2)-3 by a reaction of the compound (33) and a compound (6)-1.

In this reaction, the compound (33) and the compound (6)-1 are used in an equal amount or with one in an excess amount, and the mixture of the compounds is stirred in the presence of a condensing agent, in a solvent inactive for the reaction, from under cooling to under heating, preferably at -20°C to 60°C, generally for 0.1 hours to 5 days. Examples of the solvent include an aromatic hydrocarbon, such as toluene, an ether, such as THF or DOX, a halogenated hydrocarbon, such as dichloromethane, an alcohol, DMF, DMSO, ethyl acetate, MeCN and a mixture thereof. Examples of the condensing agent include PyBOP, HATU, CDI and the like. Performing the reaction in the presence of an organic base, such as TEA, DIPEA or NMM, or an inorganic base, such as potassium carbonate, sodium carbonate or cesium carbonate, is sometimes advantageous for smoothly promoting the reaction.

### (Raw Material Synthesis 6)

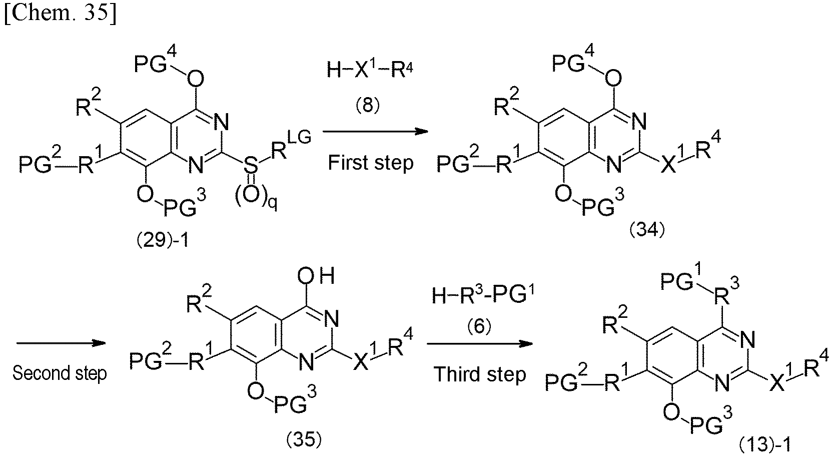

The production method is a method for producing a compound (13)-1 included in the raw material compound (13).

This step is a method for producing a compound (34) by an ipso substitution reaction of a compound (29)-1 and a compound (8).

The reaction conditions are the same as in the sixth step of the Raw Material Synthesis 3.

### (Second step)

This step is a method for producing a compound (35) by subjecting the compound (34) to a deprotection reaction.

The reaction conditions are the same as in the step described in the Production Method 1.

### (Third step)

This step is a method for producing the compound (13)-1 by a reaction of the compound (35) and a compound (6).

The reaction conditions are the same as in the twelfth step of the Raw Material Synthesis 5.

### (Raw Material Synthesis 7)

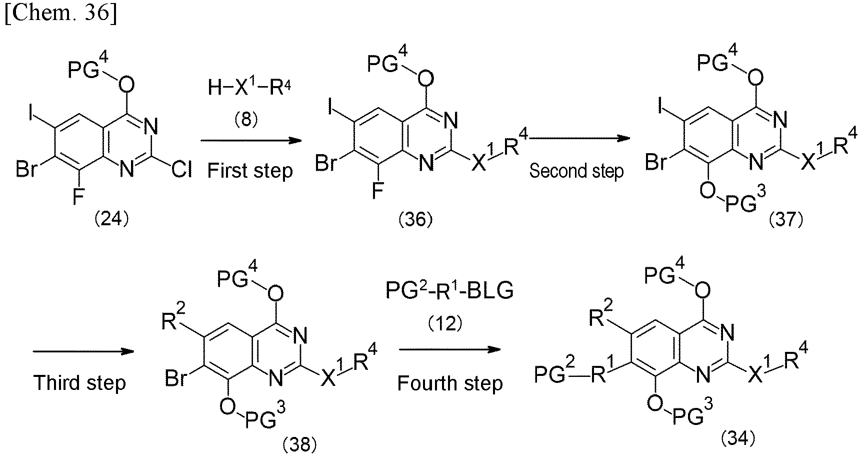

This production method is a second method for producing the raw material compound (34).

### (First step)

This step is a method for producing the compound (36) by an ipso substitution reaction of the compound (24) and a compound (8).

The reaction conditions are the same as in the second step of the Raw Material Synthesis 2.

### (Second step)

This step is a method for producing a compound (37) by an ipso substitution reaction of the compound (36) and PG³-OH.

The reaction conditions are the same as in the third step of the Raw Material Synthesis 2.

### (Third step)

This step is a method for producing a compound (38) by a Suzuki-Miyaura coupling reaction of the compound (37) and a boronic acid derivative composed of a R²-boronic acid group or the like.

The reaction conditions are the same as in the fourth step of the Raw Material Synthesis 2.

The compound (38) (where R² is hydrogen) can be produced by a dehydrogenation reaction of the compound (37) with a Pd catalyst and a reducing agent.

### [Reference]

J. Org. Chem., 1977, 42, p.3491-3494
Tetrahedron Letters 2013, 54, 5207-5210

### (Fourth step)

This step is a method for producing a compound (34) by a Suzuki-Miyaura coupling reaction of the compound (38) and a compound (12).

The reaction conditions are the same as in the fifth step of the Raw Material Synthesis 2.

### (Raw Material Synthesis 8)

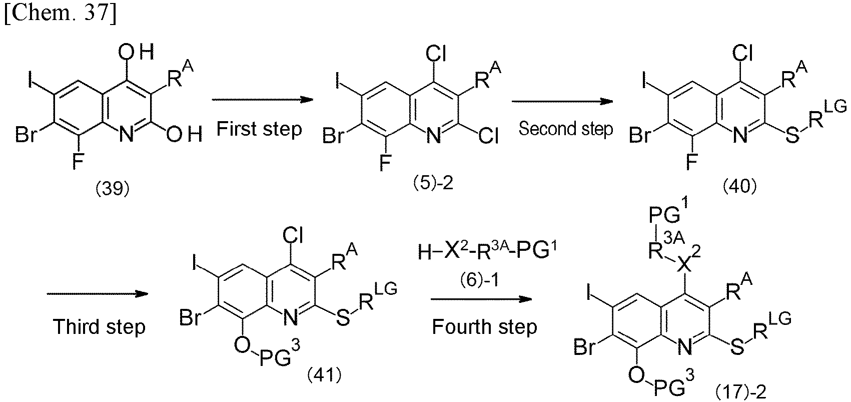

The production method is a method for producing a compound (17)-2 included in the raw material compound (17).

### (First step)

This step is a method for producing a compound (5)-2 by a chlorination reaction of the compound (39).

This reaction is performed by stirring a mixture of the compound (39) and a chlorinating agent in an equal amount or with one in an excess amount in a solvent inactive for the reaction or with no solvent, from under cooling to under reflux with heat, preferably at 60°C to under reflux with heat, generally for 0.1 hours to 5 days. Examples of the solvent used here include, but are not particularly limited to, an aromatic hydrocarbon, such as toluene, an ether, such as THF or DOX, a halogenated hydrocarbon, such as dichloromethane, and the like. Examples of the chlorinating agent include phosphorus oxychloride, thionyl chloride the like. Performing the reaction in the presence of an organic base, such as TEA, DIPEA or NMM, is sometimes advantageous for smoothly promoting the reaction.

### (Second step)

This step is a method for producing a compound (40) by an ipso substitution reaction of the compound (5)-2 and R^{LG}-SH.

The reaction conditions are the same as in the first step of the Raw Material Synthesis 3.

### (Third step)

This step is a method for producing a compound (41) by an ipso substitution reaction of the compound (40) and PG³-OH.

The reaction conditions are the same as in the third step of the Raw Material Synthesis 2.

### (Fourth step)

This step is a method for producing the compound (17)-2 by an ipso substitution reaction of the compound (41) and the compound (6)-1.

The reaction conditions are the same as in the first step of the Raw Material Synthesis 2.

### (Raw Material Synthesis 9)

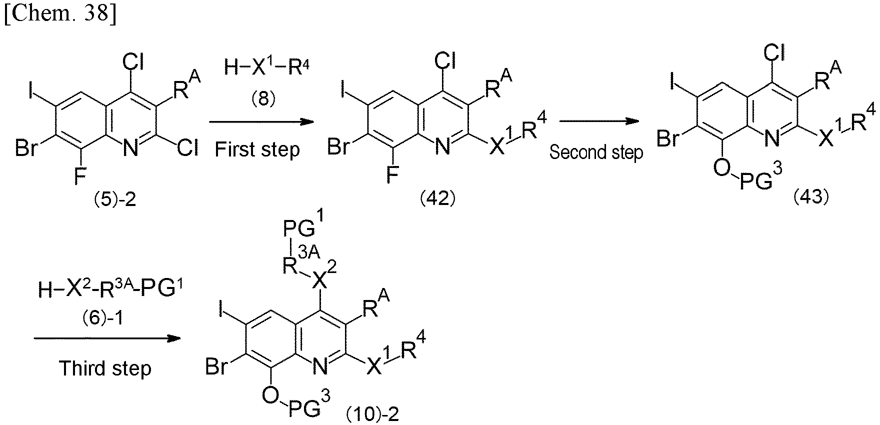

The production method is a method for producing a compound (10)-2 included in the raw material compound (10).

### (First step)

This step is a method for producing a compound (42) by an ipso substitution reaction of a compound (5)-2 and a compound (8).

The reaction conditions are the same as in the second step of the Raw Material Synthesis 2.

Moreover, the compound (42) can be produced by the Negishi coupling of a compound in which a hydrogen atom of the compound (8) is converted to halogen and the compound (5)-2.

### (Second step)

This step is a method for producing a compound (43) by an ipso substitution reaction of the compound (42) and PG³-OH.

The reaction conditions are the same as in the third step of the Raw Material Synthesis 2.

### (Third step)

This step is a method for producing the compound (10)-2 by an ipso substitution reaction of the compound (43) and the compound (6)-1.

The reaction conditions are the same as in the first step of the Raw Material Synthesis 2.

The compound of the formula (I) is isolated and purified as a free compound, a salt, hydrate, solvate or crystal polymorphous substance thereof or a substance in the amorphous solid form. A salt of the compound of the formula (I) can also be produced by subjecting the compound to a salt formation reaction which is an ordinary method.

The isolation and purification are performed by applying a common chemical operation, such as extraction, fractional crystallization or various types of fraction chromatography.

Various types of isomers can be produced by selecting an appropriate raw material compound or can be separated using a difference in physiochemical properties between the isomers. For example, an optical isomer can be obtained by a general optical resolution method of a racemate (for example, fractional crystallization for inducing a racemate to a diastereomer salt with an optically active base or acid, chromatography using a chiral column or the like or the like) and can also be produced from an appropriate optically active raw material compound.

In addition, the compound of the formula (I) or an intermediate thereof sometimes has an axial chirality and are obtained as a mixture of stereoisomers, and each stereoisomer can be isolated by separation using a common separation operation, for example, octadecylsilyl (ODS) column chromatography or silica gel column chromatography, or by fractional crystallization.

The pharmacological activities of the compound of the formula (I) was confirmed by the following tests.

Test Example 1: Evaluation of KRAS degradation activity on human G12D mutant KRAS-positive pancreatic cancer line AsPC-1 (CRL-1682; ATCC)

The KRAS degradation activity of test compounds was evaluated by measuring the expression levels of KRAS G12D by Cell ELISA.

AsPC-1 cells were seeded at 20 µL per well on 384-well plates (from Greiner bioone) to give 2.0 × 10⁴ cells per well. As for the cell culture conditions, RPMI 1640 (from Sigma-Aldrich) medium containing 10% fetal bovine serum (from Cytiva) was used in the presence of 5% CO₂ at 37°C.

The next day, the test compounds (10 points having final concentrations in the range of 10 µM to 0.3 nM) and DMSO (from FUJIFILM Wako Pure Chemical Corporation), which was the solvent for the test compounds, as a negative control were diluted 500-fold with a fresh medium and were added at 20 µL per well. The cells were cultured overnight.

The next day, the culture supernatant was removed, and 4% paraformaldehyde phosphate buffer (from FUJIFILM Wako Pure Chemical Corporation) was added at 20 µL per well. The plates were allowed to stand for 30 minutes at room temperature to thus immobilize the cells. Then, the supernatant was removed, and 0.1% Triton X-100 (from Amersham Biosciences)-containing Phosphate buffered saline (PBS; from FUJIFILM Wako Pure Chemical Corporation) was added at 20 µL per well. After allowing to stand for 10 minutes at room temperature, the supernatant was removed. PBS was added at 25 µL per well, and the supernatant was removed to thus wash each well. The washing was performed twice in total. Next, the supernatant was removed, and 0.5% sodium dodecyl sulfate (SDS; from Invitrogen)-containing PBS was added at 20 µL per well. After allowing to stand at room temperature for 10 minutes, the supernatant was removed by a centrifugal operation (using a centrifugal dehydrator machine, the supernatant was removed by the same method hereinafter). PBS was added at 25 µL per well, and the supernatant was removed to thus wash each well. The washing was performed twice in total. The supernatant was removed, and blocking solution (Intercept Blocking Buffer; from Li-COR Biosciences) was added at 20 µL per well. After allowing to stand for 30 minutes at room temperature, the supernatant was removed, and a solution obtained by diluting anti-β-actin antibody (Anti-beta Actin antibody [mAbcam 8226]-Loading Control; from abcam) 1,000-fold with blocking solution and a solution obtained by diluting anti-Ras (G12D Mutant Specific) antibody (Ras (G12D Mutant Specific) (D8H7) Rabbit mAb #14429; from Cell Signaling Technology) and anti-β-actin antibody (from abcam) 1,000-fold with blocking solution were respectively added to positive control wells and the other wells at 15 µL per well as a primary antibody. The plates were allowed to stand overnight at 4°C.

The next day, the supernatant was removed. PBS was added at 25 µL per well, and the supernatant was removed to thus wash each well. The washing was performed twice in total. The supernatant was removed, and a solution obtained by diluting Donkey antiMouse IgG H&L (IRDye 680RD) (from Li-COR Biosciences) and Goat anti-Rabbit IgG H&L (IRDye 800CW) (from Li-COR Biosciences) 1,000-fold with blocking solution was added at 15 µL per well as a secondary antibody. After allowing to stand for 1 hour at room temperature, the supernatant was removed. PBS was added at 25 µL per well, and the supernatant was removed to thus wash each well. The washing was performed twice in total. After removing the supernatant, the plates were dried with air at room temperature for 2 hours or more, and the 700 nm and 800 nm fluorescent signals were measured with Aerius (from Li-COR Biosciences).

After the signaling value of RAS measured at a fluorescence wavelength of 800 nm was corrected with the signaling value of β-actin measured at a fluorescence wavelength of 700 nm, with the signaling value at the time of addition of DMSO taken as 0% and with the signaling value when stained only with the anti-β-actin antibody taken as 100%, the degradation rate at 10 µM, the highest concentration evaluated, was calculated. The results for some test compounds of the formula (I) are shown in tables below.

In the tables presented below, Ex represents Example No.

**[Table 1]**

| Ex | % degradation | Ex | % degradation |
|---|---|---|---|
| 1 | 43 | 10 | 37 |
| 2 | 44 | 11 | 51 |
| 3 | 46 | 12 | 20 |
| 4 | 15 | 13 | 57 |
| 5 | 46 | 14 | 58 |
| 6 | 25 | 15 | 17 |
| 7 | 20 | 16 | 30 |
| 8 | 53 | 17 | 37 |
| | | 18 | 20 |

Test Example 2: Evaluation of non-anchorage-dependent cell growth inhibitory effect on human G12D mutant KRAS-positive pancreatic cancer line AsPC-1

The non-anchorage-dependent cell growth inhibitory effect of test compounds was evaluated by spheroid 3D cell culture.

AsPC-1 cells were seeded on low-cell-adhesive round bottom 384-well plates (PrimeSurface: from Sumitomo Bakelite) at 36 µL/well to give 5 x 10² cells per well. As for the cell culture conditions, RPMI 1640 medium containing 10% fetal bovine serum was used in the presence of 5% CO₂ at 37°C.

The next day, the test compounds (6 points having final concentrations in the range of 10 µM to 30 nM) and DMSO, which was the solvent for the test compounds, as a negative control were diluted 100-fold with a fresh medium and were added at 4 µL per well. After culturing in the presence of 5% CO₂ at 37°C for 6 days, CellTiter Glo 2.0 (from Promega) was added at 20 µL per well. After stirring with a plate mixer (from FINEPCR) at normal temperature for 1 hour, the luminescent signals were measured with ARVO X3 (from PerkinElmer).

With the signaling value in treatment with DMSO taken as 100% and with the signaling value in the medium alone without cells taken as 0%, the 50% inhibitory concentration values (IC₅₀) were calculated by Sigmoid-Emax nonlinear regression analysis. The results for some test compounds of the formula (I) are shown in tables below.

In the tables presented below, Ex represents Example No.

**[Table 2]**

| Ex | IC₅₀ (µM) | Ex | IC₅₀ (µM) |
|---|---|---|---|
| 1 | 3.3 | 10 | 3.7 |
| 2 | 1.9 | 11 | 2.9 |
| 3 | 2.8 | 12 | 3.5 |
| 4 | 5.1 | 13 | 2.0 |
| 5 | 2.2 | 14 | 1.2 |
| 6 | 3.6 | 15 | 3.9 |
| 8 | 3.1 | 17 | 2.6 |
| 9 | 3.4 | 18 | 7.4 |
| | | 19 | 2.3 |

Test Example 3: Evaluation of inhibitory effect on KRAS G12D/SOS/c-Raf complex formation

Using human recombinant KRAS G12D, SOS and c-Raf proteins, the inhibitory effect of test compounds on the formation of the complex of the proteins was examined by the time-resolved fluorescence resonance energy transfer (TR-FRET) method.

Biotinylated AviTag-KRAS G12D (amino acid region of 1-185, GDP) (2.5 µL; 400 nM) and test compounds dissolved in an assay buffer (50 mM HEPES [from Jena], 150 mM NaCl [from Nacalai Tesque], 5 mM MgCl₂ [from Thermo Fisher Scientific], 0.05% Tween 20 [from Sigma-Aldrich], pH 7.0) were added to 384-well plates (from Corning) in a liquid volume of 2.5 µL at 40,000 nM to 40 nM. Son of Sevenless (SOS) (amino acid region of 564-1049, 2.5 µL; 1.3 µM) and c-Raf (amino acid region of 51-131) GST (2.5 µL; 130 nM) containing GTP (from Sigma-Aldrich; 2 µM) were added to the plates, and the plates were allowed to stand for 1 hour at room temperature. Then, a mixture liquid (10 µL) of LANCE Ulight-anti-GST (from PerkinElmer; 120 nM) and LANCE Eu-W1024 labeled Streptoavidin (from PerkinElmer; 100 ng/mL) was added, and the 620-nm and 665-nm fluorescence intensities were measured using EnVision 2104 (from PerkinElmer) under the conditions of an excitation wavelength of 337 nm. After standardizing the values with the fluorescence intensity at a reference wavelength of 620 nm, the 50% inhibitory concentrations (IC₅₀) were calculated by Sigmoid-Emax nonlinear regression analysis with the signaling value of the solvent treatment taken as 0% inhibition and with the signaling value without the addition of GTP taken as 100% inhibition.

As a result of the above tests, G12D mutant KRAS degradation activity was observed for some compounds of the formula (I) (Test Example 1). Moreover, inhibitory effect on complex formation (G12D mutant KRAS inhibitory effect) was observed (Test Example 3). Furthermore, cell growth inhibitory effect on a human G12D mutant KRAS-positive pancreatic cancer line was observed for some compounds of the formula (I) (Test Example 2). Therefore, the compounds of the formula (I) can be used for the treatment of cancer, in particular, pancreatic cancer, for example, G12D mutant KRAS-positive pancreatic cancer, and the like.

A pharmaceutical composition that contains one or two or more compounds of the formula (I) or salts thereof as active ingredients can be prepared by a usually used method using an excipient usually used in the art, namely, a pharmaceutical excipient, a pharmaceutical carrier or the like.

The administration may be either oral administration with a tablet, pill, capsule, granule, powder, liquid or other agent or parenteral administration with an intraarticular, intravenous, intramuscular or other injection, a transmucosal agent, an inhalant or the like.

As a solid composition for oral administration, a tablet, powder, granular or other agent is used. In such a solid composition, one or two or more active ingredients are mixed with at least one inactive excipient. The composition may contain an inactive additive, for example, a lubricant, a disintegrator, a stabilizer or a dissolution aid, according to an ordinary method. A tablet or pill may be coated with a sugar coating or a film soluble in the stomach or intestine, as needed.

Liquid compositions for oral administration include a pharmaceutically acceptable emulsion, solution, suspension, syrup, elixir agent and the like and contain a generally used inactive diluent, for example, purified water or EtOH. The liquid composition may contain, in addition to the inactive diluent, an adjuvant, such as a solubilizer, a wetting agent or a suspending agent, a sweetening agent, a flavor, an aromatic or a preservative.

The injection agents for parenteral administration include a sterile aqueous or nonaqueous solution, suspension or emulsion agent. Examples of the aqueous solvent include distilled water for injection or physiological saline. An example of the nonaqueous solvent is an alcohol, such as EtOH. Such a composition may further contain an isotonizing agent, a preservative, a wetting agent, an emulsifier, a dispersant, a stabilizer or a dissolution aid. These are sterilized, for example, by filtration through a bacteria keeping filter, incorporation of a microbicide or irradiation. In addition, such a composition can be produced as a sterile solid composition, which is dissolved or suspended in sterile water or a sterile solvent for injection before use.

The transmucosal agent, such as an inhalant or a transnasal agent, is used in a solid, liquid or semi-solid form and can be produced according to a conventionally known method. For example, a known excipient and in addition, a pH modifier, a preservative, a surfactant, a lubricant, a stabilizer, a thickener or the like may be appropriately added. The administration can be performed using an appropriate device for inhalation or insufflation. For example, the agent can be administered using a known device, such as a metering and administering inhalation device, or an atomizer, as a compound alone or a powder of a mixture formulated, or as a solution or a suspension in combination with a medically acceptable carrier. A dry powder inhaler or the like may be for a single administration or multiple administrations, and dry powder or powder-containing capsule can be used. Alternatively, the agent may be used in a form of a pressurized aerosol spray or the like using an appropriate ejection agent, for example, a suitable gas, such as a chlorofluoroalkane or carbon dioxide.

In the case of a common oral administration, the daily dose is appropriately about 0.001 to 100 mg/kg body weight, preferably 0.1 to 30 mg/kg body weight, further preferably 0.1 to 10 mg/kg body weight, and the dose is given once or is divided into two to four times in a day. In the case of intravenous administration, the daily dose is appropriately about 0.0001 to 10 mg/kg body weight and is given once or is divided into multiple times in a day. In addition, the daily dose of a transmucosal agent is about 0.001 to 100 mg/kg body weight and is given once or is divided into multiple times in a day. The dose is appropriately decided depending on the individual case taking the symptom, age, sex and the like into account.

Depending on the route of administration, dosage form, site of administration and types of excipient and additive, the pharmaceutical composition of the present invention contains 0.01 to 100% by weight, in one embodiment, 0.01 to 50% by weight, of one or more compound of the formula (I) or salts thereof which are active ingredients.

The compounds of the formula (I) can be used in combination with various therapeutic agents or preventive agents for a disease to which the compounds of the formula (I) are considered to have an effectiveness. The combination use may be simultaneous administration or separate administration either sequential or with a desired interval. A simultaneous administration preparation may be a formulated agent or may be separately formulated.

### Examples

The production method of the compounds of the formula (I) will be described in further detail below based on Examples. Note that the present invention is not to be limited to the compounds described in the following Examples. The production methods of raw material compounds are also shown in the Production Examples. The production methods of the compounds of the formula (I) are not limited only to the production methods of specific Examples described below, and the compounds of the formula (I) can also be produced by a combination of the production methods or a method that is obvious to a person skilled in the art.

Note that, in this specification, a compound is sometimes named using naming software, such as ACD/Name (registered trademark, Advanced Chemistry Development, Inc.).

For the purpose of convenience, the concentration mol/L is shown as M. For example, 1 M aqueous sodium hydroxide solution means an aqueous sodium hydroxide solution of 1 mol/L.

The "amorphous solid form" described in this specification includes both a form showing no peak in the powder X-ray diffraction (XRD) pattern and a form having a low crystallinity.

### Production Example 1

A mixture of 7-bromo-2,4-dichloro-8-fluoro-6-iodoquinazoline (100 g), DOX (1000 mL) and THF (500 mL) was cooled with ice bath, and then DIPEA (240 mL) and tert-butyl (1S,4S)-2,5-diazabicyclo[2.2.1]heptane-2-carboxylate (48 g) were added. The mixture was stirred at room temperature overnight. Water was added to the reaction mixture, and the mixture was extracted with ethyl acetate. The organic layer was washed with an aqueous sodium chloride solution, was dried over anhydrous magnesium sulfate and was then concentrated under reduced pressure until the total amount of the solution became about 400 mL. A mixed solvent (hexane/ethyl acetate = 4/1, 1000 mL) was added to the resulting solution, and the mixture was stirred at room temperature. The precipitated solid was filtered to give tert-butyl (1S,4S)-5-(7-bromo-2-chloro-8-fluoro-6-iodoquinazolin-4-yl)-2,5-diazabicyclo[2.2.1]heptane-2-carboxylate (123 g) as a solid.

### Production Example 2

To a mixture of tert-butyl (1S,4S)-5-(7-bromo-2-chloro-8-fluoro-6-iodoquinazolin-4-yl)-2,5-diazabicyclo[2.2.1]heptane-2-carboxylate (38.4 g) and CH₂Cl₂ (500 mL) were added ethanethiol (5.3 mL) and DABCO (10.6 g) with stirring at room temperature, and the mixture was stirred under an argon atmosphere at room temperature for 17 hours. Ethanethiol (1 mL) was added, and the mixture was further stirred for 6 hours at room temperature. Then, DABCO (1.5 g) was added thereto and stirred for 3 days at room temperature, and cesium carbonate (2.1 g) was added thereto and stirred at room temperature for 22 hours. Ice water was poured into the reaction mixture, and the mixture was extracted twice with CHCl₃. The combined organic layer was dried over anhydrous magnesium sulfate and was concentrated under reduced pressure, thus obtaining tert-butyl (1S,4S)-5-[7-bromo-2-(ethylsulfanyl)-8-fluoro-6-iodoquinazolin-4-yl]-2,5-diazabicyclo[2.2.1]heptane-2-carboxylate (43.2 g) as a foam-like solid.

### Production Example 3

To a mixture of tert-butyl (1S,4S)-5-[7-bromo-2-(ethylsulfanyl)-8-fluoro-6-iodoquinazolin-4-yl]-2,5-diazabicyclo[2.2.1]heptane-2-carboxylate (40.2 g), dehydrated THF (400 mL) and (1S)-1-phenylethane-1-ol (10 mL), which were sufficiently stirred under cooling in an ice/MeOH bath, was added tBuOK (15.3 g), and under an argon atmosphere, the mixture was stirred under cooling for 30 minutes and at room temperature for 30 minutes. Ice and saturated aqueous ammonium chloride solution were added to the reaction mixture and the mixture was extracted twice with ethyl acetate. The combined organic layer was washed with saturated aqueous sodium chloride solution, was dried over anhydrous magnesium sulfate and was concentrated under reduced pressure, thus obtaining tert-butyl (1S,4S)-5-{7-bromo-2-(ethylsulfanyl)-6-iodo-8-[(1S)-1-phenylethoxy]quinazolin-4-yl}-2,5-diazabicyclo[2.2.1]heptane-2-carboxylate (45.94 g) as a foam-like solid.

### Production Example 4

A mixture of tert-butyl (1S,4S)-5-{7-bromo-2-(ethylsulfanyl)-6-iodo-8-[(1S)-1-phenylethoxy]quinazolin-4-yl}-2,5-diazabicyclo[2.2.1]heptane-2-carboxylate (45.93 g), cyclopropylboronic acid (9.8 g), tripotassium phosphate (55 g), PdCl₂(dppf)·CH₂Cl₂ (5.4 g), MeCN (360 mL), DOX (240 mL) and water (120 mL) was degassed and then purged with argon gas, and the mixture was stirred at 90°C for 5 hours under an argon atmosphere. The reaction mixture which was allowed to cool was concentrated under reduced pressure to about half the volume, and saturated aqueous sodium hydrogen carbonate solution (100 mL) and water (300 mL) were poured into the residue and extracted twice with ethyl acetate. After the combined organic layer was washed with saturated aqueous sodium chloride solution, thiol-modified silica gel (about 10 g) and basic silica gel (about 10 g) were added, and the mixture was stirred at room temperature for 30 minutes. The insoluble materials were removed by filtration while washing with ethyl acetate, and the filtrate was concentrated under reduced pressure. The residue was purified by silica gel column chromatography (hexane/ethyl acetate), thus obtaining tert-butyl (1S,4S)-5-{7-bromo-6-cyclopropyl-2-(ethylsulfanyl)-8-[(1S)-1-phenylethoxy]quinazolin-4-yl}-2,5-diazabicyclo[2.2.1]heptane-2-carboxylate (27.74 g) as a foam-like solid.

### Production Example 5

A mixture of tert-butyl (1S,4S)-5-{7-bromo-6-cyclopropyl-2-(ethylsulfanyl)-8-[(1S)-1-phenylethoxy]quinazolin-4-yl}-2,5-diazabicyclo[2.2.1]heptane-2-carboxylate (17.7 g), 6-fluoro-5-methyl-4-(4,4,5,5-tetramethyl-1,3,2-dioxaborolan-2-yl)-2-(triphenylmethyl)-2H-indazole (19.6 g), palladium(II) acetate (0.7 g), dicyclohexyl(2',6'-diisopropoxy-[1,1'-biphenyl]-2-yl)phosphine (2.7 g), barium hydroxide (14.6 g), DOX (500 mL) and water (50 mL) was degassed and then purged with argon gas, and the mixture was stirred at 50°C for 6 hours under an argon atmosphere. The reaction suspension which was allowed to cool was filtered through celite (registered trademark) pad while washing with ethyl acetate. After the filtrate was concentrated to about 1/4 under reduced pressure, water was poured, and the mixture was extracted twice with ethyl acetate. The combined organic layer was washed with saturated aqueous sodium chloride solution, and then dried over anhydrous magnesium sulfate. The solution was concentrated under reduced pressure, and the residue was purified twice by silica gel column chromatography (basic silica gel, hexane/ethyl acetate), thus obtaining tert-butyl (1S,4S)-5-{6-cyclopropyl-2-(ethylsulfanyl)-7-[6-fluoro-5-methyl-2-(triphenylmethyl)-2H-indazol-4-yl]-8-[(1S)-1-phenylethoxy]quinazolin-4-yl}-2,5-diazabicyclo[2.2.1]heptane-2-carboxylate (about 4.5:1 diastereomeric mixture derived from axial chirality, 20.64 g) as a foam-like solid.

### Production Example 6

To a mixture of tert-butyl (1S,4S)-5-{6-cyclopropyl-2-(ethylsulfanyl)-7-[6-fluoro-5-methyl-2-(triphenylmethyl)-2H-indazol-4-yl]-8-[(1S)-1-phenylethoxy]quinazolin-4-yl}-2,5-diazabicyclo[2.2.1]heptane-2-carboxylate (about 4.5:1 diastereomeric mixture derived from axial chirality, 20.63 g) and EtOH (250 mL) was added 4-methylbenzene-1-sulfonic acid monohydrate (4.8 g) with stirring at room temperature, and the mixture was stirred at room temperature for 1 hour under an argon atmosphere. Ice and saturated aqueous sodium hydrogen carbonate solution were poured into the reaction mixture, and the resulting white insoluble materials were dissolved in ethyl acetate and extracted twice with ethyl acetate. The combined organic layer was washed with saturated aqueous sodium chloride solution, was dried over anhydrous magnesium sulfate and was then concentrated under reduced pressure. The residue was purified by silica gel column chromatography (hexane/ethyl acetate), thus obtaining tert-butyl (1S,4S)-5-{(7M)-6-cyclopropyl-2-(ethylsulfanyl)-7-(6-fluoro-5-methyl-1H-indazol-4-yl)-8-[(1S)-1-phenylethoxy]quinazolin-4-yl}-2,5-diazabicyclo[2.2.1]heptane-2-carboxylate (single diastereomer derived from axial chirality, 11.17 g), which was a low-polar fraction, as a foam-like solid and tert-butyl (1S,4S)-5-{(7P)-6-cyclopropyl-2-(ethylsulfanyl)-7-(6-fluoro-5-methyl-1H-indazol-4-yl)-8-[(1S)-1-phenylethoxy]quinazolin-4-yl}-2,5-diazabicyclo[2.2.1]heptane-2-carboxylate (single diastereomer derived from axial chirality, 1.92 g), which was a high-polar fraction, as a foam-like solid.

### Production Example 7

To a mixture of tert-butyl (1S,4S)-5-{(7M)-6-cyclopropyl-2-(ethylsulfanyl)-7-(6-fluoro-5-methyl-1H-indazol-4-yl)-8-[(1S)-1-phenylethoxy]quinazolin-4-yl}-2,5-diazabicyclo[2.2.1]heptane-2-carboxylate (11.1 g) and THF (150 mL) were added 3,4-dihydro-2H-pyran (10 mL) and 4-methylbenzene-1-sulfonic acid monohydrate (0.7 g) with stirring at room temperature, and the mixture was stirred at room temperature for 14 hours under an argon atmosphere. Another 3,4-dihydro-2H-pyran (5 mL) and 4-methylbenzene-1-sulfonic acid monohydrate (0.3 g) were added, and the mixture was stirred at room temperature for another 5 hours. After TEA (2 mL) was added to quench the reaction, the mixture was concentrated under reduced pressure. The residue was purified by silica gel column chromatography (hexane/ethyl acetate), thus obtaining tert-butyl (1S,4S)-5-{(7M)-6-cyclopropyl-2-(ethylsulfanyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (11.15 g) as a foam-like solid.

### Production Example 8

To a mixture of tert-butyl (1S,4S)-5-{(7M)-6-cyclopropyl-2-(ethylsulfanyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (11.14 g) and CH₂Cl₂ (170 mL) was added m-chloroperbenzoic acid (about 30% water content, 7.9 g) under ice-bath cooling, and the mixture was stirred at room temperature for 1.5 hours under an argon atmosphere. Ice, saturated aqueous sodium thiosulfate solution and saturated aqueous sodium hydrogen carbonate solution were added, and the mixture was stirred at room temperature for about 20 minutes. Then, the reaction solution was diluted with CH₂Cl₂ and separated into layers. The organic layer was dried over anhydrous magnesium sulfate and was concentrated under reduced pressure. The residue was purified by silica gel column chromatography (hexane/ethyl acetate), thus obtaining tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (9.02 g) as a foam-like solid.

### Production Example 9

To a mixture of tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (4.5 g) and MeOH (100 mL) was added 10% Pd/C (52% water content, 1 g), and the mixture was stirred at room temperature overnight under a hydrogen atmosphere at normal pressure. Celite (registered trademark) was added, and the mixture was filtered while washing with ethyl acetate and CHCl₃. Toluene (about 20 mL) was added to the filtrate, and the mixture was concentrated under reduced pressure. MeOH (80 mL) and 10% Pd/C (52% water content, 1.4 g) were added to the resulting solid, and the mixture was stirred at room temperature for 3 hours under a hydrogen atmosphere at normal pressure. Celite (registered trademark) was added, and the mixture was filtered while washing with ethyl acetate and CHCl₃. The filtrate was concentrated under reduced pressure, and the residue was purified by silica gel column chromatography (hexane/ethyl acetate), thus obtaining tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-hydroxyquinazolin-4-yl}-2,5-diazabicyclo[2.2.1]heptane-2-carboxylate (3.42 g) as a foam-like solid.

### Production Example 10

To a mixture of tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-hydroxyquinazolin-4-yl}-2,5-diazabicyclo[2.2.1]heptane-2-carboxylate (3.01 g), DMF (30 mL) and methyl 4-(chloromethyl)benzoate (1 g) was added cesium carbonate (4 g) with stirring at room temperature, and the mixture was stirred under an argon atmosphere at room temperature overnight. Ice water and saturated aqueous ammonium chloride solution were added to the reaction mixture, and the mixture was extracted with ethyl acetate twice. The combined organic layer was washed with saturated aqueous sodium chloride solution, was dried over anhydrous magnesium sulfate and was concentrated under reduced pressure. The resulting residue was purified by silica gel column chromatography (basic silica gel, hexane/ethyl acetate), thus obtaining tert-butyl (1S,4S)-5-[(7M)-6-cyclopropyl-2-(ethanesulfonyl)-7-[6-fluoro-5-methyl-1-(oxan-2-yl)-1H-indazol-4-yl]-8-{[4-(methoxycarbonyl)phenyl]methoxy}quinazolin-4-yl]-2, 5-diazabicyclo[2.2.1]heptane-2-carboxylate (3.32 g) as a foam-like solid.

### Production Example 11

To a mixture of tert-butyl (1S,4S)-5-[(7M)-6-cyclopropyl-2-(ethanesulfonyl)-7-[6-fluoro-5-methyl-1-(oxan-2-yl)-1H-indazol-4-yl]-8-{[4-(methoxycarbonyl)phenyl]methoxy}quinazolin-4-yl]-2, 5-diazabicyclo[2.2.1]heptane-2-carboxylate (200 mg), tetrahydro-2H-pyran-4-ol (32 mg) and THF (4 mL) was added tBuOK (56 mg) under cooling in an ice/MeOH bath, and the mixture was stirred at the same temperature for 30 minutes under an argon atmosphere. Ice water and saturated aqueous ammonium chloride solution were added to the reaction mixture, and the mixture was extracted with ethyl acetate twice. The combined organic layer was washed with saturated aqueous sodium chloride solution, was dried over anhydrous magnesium sulfate and was concentrated under reduced pressure. The residue was purified by silica gel column chromatography (basic silica gel, hexane/ethyl acetate), thus obtaining tert-butyl (1S,4S)-5-{(7M)-6-cyclopropyl-7-[6-fluoro-5-methyl-1-(oxan-2-yl)-1H-indazol-4-yl]-8-{[4-(methoxycarbonyl)phenyl]methoxy}-2-[(oxan-4-yl)oxy]quinazolin-4-yl}-2,5-diazabicyclo[2.2.1]heptane-2-carboxylate (144 mg) as a foam-like solid.

### Production Example 12

To a mixture of tert-butyl (1S,4S)-5-{(7M)-6-cyclopropyl-7-[6-fluoro-5-methyl-1-(oxan-2-yl)-1H-indazol-4-yl]-8-{[4-(methoxycarbonyl)phenyl]methoxy}-2-[(oxan-4-yl)oxy]quinazolin-4-yl}-2,5-diazabicyclo[2.2.1]heptane-2-carboxylate (142 mg), MeOH (1 mL) and THF (1 mL) was added aqueous sodium hydroxide solution (1 M, 1 mL) under ice-bath cooling, and the mixture was stirred at room temperature for 6 hours. Under ice-bath cooling, hydrochloric acid (1M, 1 mL) was added, and the mixture was extracted twice with CHCl₃/MeOH (9/1). The combined organic layer was dried over anhydrous sodium sulfate. The solvent was removed under reduced pressure to give 4-[({(7M)-4-[(1S,4S)-5-(tert-butoxycarbonyl)-2,5-diazabicyclo[2.2.1]heptan-2-yl]-6-cyclopropyl-7-[6-fluoro-5-methyl-1-(oxan-2-yl)-1H-indazol-4-yl]-2-[(oxan-4-yl)oxy]quinazolin-8-yl}oxy)methyl]benzoic acid (132 mg) as a solid.

### Production Example 13

In THF (400 mL), 7-bromo-2,4-dichloro-8-fluoro-6-iodoquinazoline (40 g) was suspended, aqueous sodium hydroxide solution (1M, 190 mL) was added dropwise under ice-bath cooling so that the internal temperature was 10°C or lower, and the mixture was stirred for 2 hours. The reaction solution was poured at once into an Erlenmeyer flask containing hydrochloric acid (1M, 190 mL) and ice water (about 900 g), and the mixture was stirred for about 30 minutes at room temperature (until the ice melted). The insoluble materials were filtered while washing with water and were dried under reduced pressure, thus obtaining 7-bromo-2-chloro-8-fluoro-6-iodoquinazolin-4-ol (33.56 g) as a solid.

### Production Example 14

Under nitrogen flow, to a mixture of 7-bromo-2-chloro-8-fluoro-6-iodoquinazolin-4-ol (24.6 g) and THF (260 mL), which was heated to 60°C, was added 2-tert-butyl-1,3-diisopropylisourea (73.4 g) dropwise over 15 minutes. The mixture was stirred at the same temperature for 2.5 hours. After allowing to cool to room temperature, the colorless solid was separated by filtration while washing with THF (about 500 mL). The filtrate was concentrated, MeOH (210 mL) was added to the resulting solid, and the mixture was suspended and washed by stirring at room temperature for 1 hour. The solid was filtered with MeOH (100 mL) to give 7-bromo-4-tert-butoxy-2-chloro-8-fluoro-6-iodoquinazoline (23.2 g) as a solid.

### Production Example 15

Ethanethiol (3.6 mL) and DABCO (7.7 g) were added to a CH₂Cl₂ (200 mL) suspension of 7-bromo-4-tert-butoxy-2-chloro-8-fluoro-6-iodoquinazoline (21 g) at room temperature, and under an argon atmosphere, the mixture was stirred at room temperature overnight. The reaction was quenched with water under ice-bath cooling. CHCl₃ was added, the organic layer and the aqueous layer were separated, and the aqueous layer was extracted with CHCl₃ three times.

The collected organic layer was dried over anhydrous sodium sulfate, was filtered and was concentrated under reduced pressure, thus obtaining 7-bromo-4-tert-butoxy-2-(ethylsulfanyl)-8-fluoro-6-iodoquinazoline (23.4 g) as a solid.

### Production Example 16

To a THF (400 mL) solution of 7-bromo-4-tert-butoxy-2-(ethylsulfanyl)-8-fluoro-6-iodoquinazoline (32 g) and (1S)-1-phenylethan-1-ol (11 mL) was added tBuOK (10 g) under ice-bath cooling, and the mixture was stirred under ice-bath cooling for 1 hour under an argon atmosphere. The reaction was quenched with saturated aqueous ammonium chloride solution under ice-bath cooling. Water and ethyl acetate were added, the organic layer and the aqueous layer were separated, and the organic layer was washed with saturated aqueous sodium chloride solution, was dried over anhydrous sodium sulfate and was filtered. The filtrate was concentrated under reduced pressure. The residue was purified by silica gel column chromatography (hexane/ethyl acetate), thus obtaining 7-bromo-4-tert-butoxy-2-(ethylsulfanyl)-6-iodo-8-[(1S)-1-phenylethoxy]quinazoline (36.6 g) as an oil.

### Production Example 17

At room temperature, 7-bromo-4-tert-butoxy-2-(ethylsulfanyl)-6-iodo-8-[(1S)-1-phenylethoxy]quinazoline (36.6 g), cyclopropylboronic acid (7.5 g), PdCl₂(dppf)·CH₂Cl₂(7.6 g), tripotassium phosphate (53 g), MeCN (440 mL) and water (80 mL) were mixed and were stirred at 90°C for 4 hours under an argon atmosphere. After the temperature was returned to room temperature, the reaction solution was diluted with ethyl acetate and water. The organic layer and the aqueous layer were separated, and the organic layer was washed with saturated aqueous sodium chloride solution, was dried over anhydrous sodium sulfate and was filtered. The filtrate was concentrated under reduced pressure. The residue was purified by silica gel column chromatography (hexane/ethyl acetate), thus obtaining 7-bromo-4-tert-butoxy-6-cyclopropyl-2-(ethylsulfanyl)-8-[(1S)-1-phenylethoxy]quinazoline (22.9 g) as an oil.

### Production Example 18

To 7-bromo-4-tert-butoxy-6-cyclopropyl-2-(ethylsulfanyl)-8-[(1S)-1-phenylethoxy]quinazoline (14.21 g) were added 6-fluoro-5-methyl-4-(4,4,5,5-tetramethyl-1,3,2-dioxaborolan-2-yl)-2-(triphenylmethyl)-2H-indazole (19.3 g), palladium(II) acetate (0.67 g), dicyclohexyl(2',6'-diisopropoxy-[1,1'-biphenyl]-2-yl)phosphine (2.67 g), anhydrous barium hydroxide (14.6 g) and DOX (500 mL)/water (100 mL), degassing and argon gas substitution operations were performed several times. Then, the mixture was heated and stirred at 50°C overnight under an argon atmosphere. The reaction suspension which was allowed to cool was filtered through celite (registered trademark) pad while washing with ethyl acetate, and the gray insoluble materials were removed by filtration. After the filtrate was concentrated to about 1/4 under reduced pressure, water was added, and the mixture was extracted twice with ethyl acetate. The collected organic layer was washed with saturated aqueous sodium chloride solution, was dried over anhydrous magnesium sulfate and was concentrated under reduced pressure. The resulting residue was purified by silica gel column chromatography (hexane/ethyl acetate), thus obtaining 4-tert-butoxy-6-cyclopropyl-2-(ethylsulfanyl)-7-[6-fluoro-5-methyl-2-(triphenylmethyl)-2H-indazol-4-yl]-8-[(1S)-1-phenylethoxy]quinazoline (about 3.3:1 diastereomeric mixture derived from axial chirality, 16.44 g) as a solid.

### Production Example 19

Under nitrogen flow, 4-tert-butoxy-6-cyclopropyl-2-(ethylsulfanyl)-7-[6-fluoro-5-methyl-2-(triphenylmethyl)-2H-indazol-4-yl]-8-[(1S)-1-phenylethoxy]quinazoline (about 3.3:1 diastereomeric mixture derived from axial chirality, 33.71 g) was dissolved in CH₂Cl₂ (500 mL), and m-chloroperbenzoic acid (about 30% water content, 22.4 g) was added under ice-bath cooling (internal temperature: 5 to 10°C). The mixture was stirred for 2 hours at room temperature. Under ice-bath cooling, an aqueous solution (300 mL) of sodium thiosulfate pentahydrate (11 g) and saturated aqueous sodium hydrogen carbonate solution (300 mL) were poured into the reaction solution, and the mixture was stirred for 30 minutes at room temperature and was then extracted with ethyl acetate twice. The combined organic layer was washed with saturated aqueous sodium hydrogen carbonate solution and saturated aqueous sodium chloride solution, was dried over anhydrous magnesium sulfate and was filtered. The filtrate was concentrated under reduced pressure, thus obtaining 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (diastereomeric mixture derived from axial chirality). iPrOH (1000 mL) was added to the resulting residue, and the mixture was stirred at room temperature overnight. The resulting solid was filtered, was washed with iPrOH and was dried under reduced pressure, thus obtaining 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (about 1:1 diastereomeric mixture derived from axial chirality, 11.52 g) as a solid. The filtrate was concentrated under reduced pressure, thus obtaining the desired (7M)-4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (single diastereomer, 23.29 g) as a solid.

### Production Example 23

To a mixture of methyl 4-[({(7M)-4-tert-butoxy-6-cyclopropyl-7-[6-fluoro-5-methyl-2-(triphenylmethyl)-2H-indazol-4-yl]-2-[(2S)-2-methoxypropoxy]quinazolin-8-yl}oxy)methyl]benzoate (2.51 g) and THF (30 mL) were added 3,4-dihydro-2H-pyran (2.7 mL) and 4-methylbenzene-1-sulfonic acid monohydrate (270 mg) at room temperature, and the mixture was stirred at 60°C for 2 hours. The reaction solution was concentrated under reduced pressure, and the resulting residue was purified by silica gel column chromatography (basic silica gel, CHCl₃/MeOH), thus obtaining methyl 4-[({(7M)-6-cyclopropyl-7-[6-fluoro-5-methyl-1-(oxan-2-yl)-1H-indazol-4-yl]-4-hydroxy-2-[(2S)-2-methoxypropoxy]quinazolin-8-yl}oxy)methyl]benzoate (1.79 g) as a foam-like solid.

### Production Example 24

Under nitrogen atmosphere, to a THF (2 mL) solution of methyl 4-[({(7M)-6-cyclopropyl-7-[6-fluoro-5-methyl-1-(oxan-2-yl)-1H-indazol-4-yl]-4-hydroxy-2-[(2S)-2-methoxypropoxy]quinazolin-8-yl}oxy)methyl]benzoate (100 mg) were added cesium carbonate (85 mg) and PyBOP (120 mg) at room temperature in this order, and the mixture was stirred at room temperature for 1 hour. Tert-butyl (3S)-3-(methylamino)pyrrolidine-1-carboxylate (76 mg) and cesium carbonate (100 mg) were added at room temperature, and the mixture was stirred at 60°C for 2 hours. After the mixture was allowed to cool to room temperature, ethyl acetate was added to the reaction solution, and the mixture was filtered through celite (registered trademark) pad. The filtrate was concentrated, and the resulting residue was purified silica gel chromatography (hexane/ethyl acetate), thus obtaining tert-butyl (3S)-3-[{(7M)-6-cyclopropyl-7-[6-fluoro-5-methyl-1-(oxan-2-yl)-1H-indazol-4-yl]-8-{[4-(methoxycarbonyl)phenyl]methoxy}-2-[(2S)-2-methoxypropoxy]quinazolin-4-yl}(methyl)amino]pyrrolidine-1-carboxylate (123 mg) as an oil.

### Production Example 29

To a mixture of methyl 4-[({(7M)-6-cyclopropyl-4-[(1-{[2-(dimethylamino)ethyl]carbamoyl}azetidin-3-yl)oxy]-7-[6-fluoro-5-methyl-1-(oxan-2-yl)-1H-indazol-4-yl]-2-[(2S)-2-methoxypropoxy]quinazolin-8-yl}oxy)methyl]benzoate (45 mg) and dichloroethane (2 mL) was added trimethyltin hydroxide (50 mg) at room temperature, and the mixture was stirred at 80°C for 16 hours. After the mixture was allowed to cool to room temperature, trimethyltin hydroxide (50 mg) was added, and the mixture was stirred at 80°C for 48 hours. After the mixture was allowed to cool to room temperature, hydrochloric acid (1 M) was added, and the mixture was extracted twice with CHCl₃/MeOH (9/1). The combined organic layer was washed with hydrochloric acid (1 M), was dried over anhydrous sodium sulfate, and was filtered. The filtrate was concentrated under reduced pressure, thus obtaining 4-[({(7M)-6-cyclopropyl-4-[(1-{[2-(dimethylamino)ethyl]carbamoyl}azetidin-3-yl)oxy]-7-[6-fluoro-5-methyl-1-(oxan-2-yl)-1H-indazol-4-yl]-2-[(2S)-2-methoxypropoxy]quinazolin-8-yl}oxy)methyl]benzoic acid (74 mg, purity: about 60%) as a solid.

### Production Example 30

To a mixture of 2-amino-4-bromo-3-fluoro-5-iodobenzoic acid (10 g) and N-methyl-2-pyrrolidone (20 mL) was added trimethyl orthoacetate (10.5 mL), and the mixture was stirred at 110°C for 12 hours. After the mixture was cooled to room temperature, MeOH (20 mL) was added, and the resulting solid was filtered while washing with ice-cooled methanol (about 60 mL) and was dried under reduced pressure, thus obtaining 2-acetamido-4-bromo-3-fluoro-5-iodobenzoic acid methyl ester (6.67 g) as a solid.

### Production Example 31

Under an argon atmosphere, to a mixture of lithium bis(trimethylsilyl)amide (1.3 M THF solution, 100 mL) and THF (150 mL) was added 2-acetamido-4-bromo-3-fluoro-5-iodobenzoic acid methyl ester (18 g) portionwise under cooling in a water bath. The mixture was stirred at 40°C for 1 hour and was then cooled to room temperature, water was added, and the mixture was washed twice with ethyl acetate. Under ice-bath cooling, the aqueous layer was acidified with hydrochloric acid (1M, 140 mL) and ice water and was stirred for a while. The precipitated solid was filtered and was dried under reduced pressure. The resulting solid was filtered while washing with methanol and was dried under reduced pressure, thus obtaining 7-bromo-8-fluoro-4-hydroxy-6-iodoquinoline -2(1H)-one (13.6 g) as a solid.

### Production Example 32

Under an argon atmosphere, phosphoryl chloride (24 mL) was added to 7-bromo-8-fluoro-4-hydroxy-6-iodoquinoline -2(1H)-one (5 g), and under ice-bath cooling DIPEA (7 mL) was slowly added. The mixture was stirred at 110°C for 1 hour. The reaction solution was cooled to room temperature and was dried at room temperature under reduced pressure. Ice water was added to the residue, and the mixture was stirred for 30 minutes. The precipitated solid was filtered, was washed with water and was then dried under reduced pressure, thus obtaining 7-bromo-2,4-dichloro-8-fluoro-6-iodoquinoline (5.03 g).

### Production Example 33

Under an argon atmosphere, to a mixture of 7-bromo-2,4-dichloro-8-fluoro-6-iodoquinoline (6.4 g) and DMAc (70 mL) was added DABCO (1.8 g), and the mixture was stirred at 40°C for 2 hours. Ethanethiol (1.4 mL) was added, and the mixture was stirred at 60°C for 1 hour. After the mixture was cooled to room temperature, water was added, and the mixture was stirred for 5 minutes. The resulting solid was filtered and was dried under reduced pressure. The resulting solid was purified by silica gel column chromatography (hexane/ethyl acetate), thus obtaining 7-bromo-4-chloro-2-(ethylsulfanyl)-8-fluoro-6-iodoquinoline (5.76 g) as a solid.

### Production Example 34

Under an argon atmosphere, a mixture prepared by adding tBuOK (1.39 g) to a dehydrated THF (40 mL) solution of (1S)-1-phenylethan-1-ol (1.56 mL) and stirring at room temperature for 30 minutes was added dropwise to a mixture of 7-bromo-4-chloro-2-(ethylsulfanyl)-8-fluoro-6-iodoquinoline (5.5 g) and dehydrated THF (40 mL) under ice-bath cooling over 20 minutes, and the mixture was stirred at the same temperature for 10 minutes. Separately, under an argon atmosphere, a mixture prepared by adding tBuOK (415 mg) to a dehydrated THF (10 mL) solution of (1S)-1-phenylethan-1-ol (0.45 mL) and stirring at room temperature for 10 minutes was slowly added dropwise to the above reaction mixture under ice-bath cooling, and the mixture was stirred at the same temperature for 10 minutes. Saturated aqueous ammonium chloride solution, ice and ethyl acetate were added to divide the mixture into layers, and the aqueous layer was extracted with ethyl acetate twice. The combined organic layer was washed with saturated aqueous sodium chloride solution and was dried over anhydrous magnesium sulfate. The insoluble materials were removed by filtration, and the filtrate was concentrated under reduced pressure. The residue was purified by silica gel column chromatography (hexane/ethyl acetate), thus obtaining 7-bromo-4-chloro-2-(ethylsulfanyl)-6-iodo-8-[(1S)-1-phenylethoxy]quinoline (4.9 g) as an oil. Slightly impure fractions were concentrated, thus obtaining 7-bromo-4-chloro-2-(ethylsulfanyl)-6-iodo-8-[(1S)-1-phenylethoxy]quinoline (1.56 g) as an oil.

### Production Example 35

Under an argon atmosphere, a mixture prepared by adding tBuOK (1.59 g) to a DMAc (20 mL) solution of tert-butyl (3 S)-3-hydroxypyrrolidine-1-carboxylate (2.7 g) and stirring at room temperature for 10 minutes was added dropwise to a mixture of 7-bromo-4-chloro-2-(ethylsulfanyl)-6-iodo-8-[(1S)-1-phenylethoxy]quinoline (6.46 g) and DMAc (40 mL) under ice-bath cooling, and the mixture was stirred at the same temperature for 30 minutes. Saturated aqueous ammonium chloride solution, ice and ethyl acetate were added, and the mixture was stirred to divide the mixture into layers. The aqueous layer was extracted with ethyl acetate twice. The combined organic layer was washed with saturated aqueous sodium chloride solution, was dried over anhydrous magnesium sulfate and was concentrated under reduced pressure. The residue was purified by silica gel column chromatography (hexane/ethyl acetate), thus obtaining tert-butyl (3S)-3-({7-bromo-2-(ethylsulfanyl)-6-iodo-8-[(1S)-1-phenylethoxy]quinolin-4-yl}oxy)pyrrolidine-1-carboxylate (4.6 g) as a solid.

### Production Example 36

Under an argon atmosphere, to a mixture of tert-butyl (3S)-3-({7-bromo-2-(ethylsulfanyl)-6-iodo-8-[(1S)-1-phenylethoxy]quinolin-4-yl}oxy)pyrrolidine-1-carboxylate (1.38 g), cyclopropylboronic acid (255 mg), PdCl₂(dppf)·CH₂Cl₂ (162 mg), MeCN (15 mL) and water (3 mL) was added tripotassium phosphate (1.52 g), and the mixture was stirred at 90°C for 6 hours. After the mixture was allowed to cool to room temperature, ethyl acetate and water were added. Two layers were separated, and the aqueous layer was extracted with ethyl acetate. The combined organic layer was washed with saturated aqueous sodium chloride solution and was dried over anhydrous magnesium sulfate. The insoluble materials were removed by filtration, and the filtrate was concentrated under reduced pressure. The residue was purified by silica gel column chromatography (hexane/ethyl acetate), thus obtaining tert-butyl (3S)-3-({7-bromo-6-cyclopropyl-2-(ethylsulfanyl)-8-[(1S)-1-phenylethoxy]quinolin-4-yl}oxy)pyrrolidine-1-carboxylate (589 mg) as a foam-like solid.

### Production Example 37

Under an argon atmosphere, a mixture of tert-butyl (3S)-3-({7-bromo-6-cyclopropyl-2-(ethylsulfanyl)-8-[(1S)-1-phenylethoxy]quinolin-4-yl}oxy)pyrrolidine-1-carboxylate (2.71 g), 6-fluoro-5-methyl-4-(4,4,5,5-tetramethyl-1,3,2-dioxaborolan-2-yl)-2-(triphenylmethyl)-2H-indazole (3.44 g), dicyclohexyl(2',6'-diisopropoxy-[1,1'-biphenyl]-2-yl)phosphine (531 mg), palladium(II) acetate (106 mg), and anhydrous barium hydroxide (2.29 g) were suspended in DOX (110 mL) and water (22 mL), the suspension was stirred at 50°C for 15 minutes. 6-fluoro-5-methyl-4-(4,4,5,5-tetramethyl-1,3,2-dioxaborolan-2-yl)-2-(triphenylmethyl)-2H-indazole (1.6 g) was added, and the mixture was further stirred at 50°C for 15 minutes. After the mixture was cooled to room temperature, ethyl acetate and water were added, and the insoluble materials were removed by filtration through celite (registered trademark) pad. The two layers of filtrate were separated, and the aqueous layer was extracted with ethyl acetate. The combined organic layer was washed with saturated aqueous sodium chloride solution and was dried over anhydrous magnesium sulfate. After separating the drying agent by filtration, the filtrate was concentrated under reduced pressure, and the resulting residue was purified by silica gel column chromatography (basic silica gel, hexane/ethyl acetate), thus obtaining tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (about 5:1 diastereomeric mixture derived from axial chirality, 3.88 g) as an oil.

To tert-butyl (3S)-3-({6-cyclopropyl-2-(ethylsulfanyl)-7-[6-fluoro-5-methyl-2-(triphenylmethyl)-2H-indazol-4-yl]-8-[(1 S)-1-phenylethoxy]quinolin-4-yl}oxy)pyrrolidine-1-carboxylate (about 5:1 diastereomeric mixture derived from axial chirality, 100 mg) was added MeOH (2 mL), and the mixture was heated to 70°C to dissolve. The mixture was stirred at room temperature overnight and was further stirred for 3 days. Solid precipitation (pale yellow suspension) was observed. The solid was filtered and was washed with a small amount of MeOH, thus obtaining tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (about 1:1 diastereomeric mixture derived from axial chirality, 21 mg) as a powder. The filtrate was concentrated, thus obtaining the desired tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (single diastereomer derived from axial chirality, 70 mg) as a solid.

Similarly, to tert-butyl (3 S)-3-({ 6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (about 5:1 diastereomeric mixture derived from axial chirality, 2.95 g) was added EtOH (6 mL), and the mixture was heated to 70°C to dissolve. The powder of the diastereomeric mixture derived from axial chirality (obtained above) was added in a trace amount at room temperature, and the mixture was stirred at room temperature overnight. The precipitated solid was filtered and was washed with a small amount of EtOH, thus obtaining tert-butyl (3S)-3-({6-cyclopropyl-2-(ethanesulfonyl)-7-(6-fluoro-5-methyl-2-(triphenylmethyl)-2H-indazol-4-yl]-8-[(1S)-1-phenylethoxy]quinolin-4-yl}oxy)pyrrolidine-1-carboxylate (about 1:1 diastereomeric mixture derived from axial chirality, 699 mg) as a colorless powder. The filtrate was concentrated, thus obtaining the desired tert-butyl (3S)-3-[{6-cyclopropyl-2-(ethylsulfanyl)-7-[6-fluoro-5-methyl-2-(triphenylmethyl)-2H-indazol-4-yl]-8-[(1 S)-1-phenylethoxy]quinolin-4-yl}oxy)pyrrolidine-1-carboxylate (single diastereomer derived from axial chirality, 2.33 g) as a foam-like solid.

### Production Example 38

Under an argon atmosphere, to a CH₂Cl₂ (30 mL) solution of tert-butyl (3S)-3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (2.70 g) was added m-chloroperbenzoic acid (about 30% water content, 1.6 g) under ice-bath cooling, and the mixture was stirred at the same temperature for 1 hour. Under ice-bath cooling, an aqueous solution (10 mL) containing sodium thiosulfate pentahydrate (1.7 g) and saturated aqueous sodium hydrogen carbonate solution (40 mL) were added, and the mixture was stirred at room temperature for 30 minutes. Then, the reaction mixture was extracted with CHCl₃. The organic layer was dried over anhydrous magnesium sulfate and was concentrated under reduced pressure. The residue was purified by silica gel column chromatography (hexane/ethyl acetate), thus obtaining tert-butyl (3 S)-3-({ 6-cyclopropyl-2-(ethanesulfonyl)-7-[6-fluoro-5-methyl-2-(triphenylmethyl)-2H-indazol-4-yl]-8-[(1S)-1-phenylethoxy]quinolin-4-yl}oxy)pyrrolidine-1-carboxylate (2.68 g) as a foam-like solid.

### Production Example 39

Under nitrogen atmosphere, to a solution in MeOH (15 mL) and THF (15 mL) of tert-butyl (3S)-3-({6-cyclopropyl-2-(ethanesulfonyl)-7-[6-fluoro-5-methyl-2-(triphenylmethyl)-2H-indazol-4-yl]-8-[(1 S)-1-phenylethoxy]quinolin-4-yl}oxy)pyrrolidine-1-carboxylate (1.3 g) were added 10% Pd/C (50% water content, 300 mg) and sodium hydrogen carbonate (600 mg), and then the mixture was stirred at room temperature for 4 hours under a hydrogen atmosphere at normal pressure. The reaction mixture overlayed with nitrogen and was then filtered through celite (registered trademark) pad using MeOH, and the filtrate was concentrated under reduced pressure. Water was added to the residue, and the mixture was extracted twice with ethyl acetate. The combined organic layer was washed with saturated aqueous sodium chloride solution, was dried over anhydrous magnesium sulfate and was concentrated under reduced pressure, thus obtaining tert-butyl (3S)-3-({6-cyc1opropyl-2-(ethanesulfonyl)-7-[6-fluoro-5-methyl-2-(triphenylmethyl)-2H-indazol-4-yl]-8-hydroxyquinolin-4-yl}oxy)pyrrolidine-1-carboxylate (1.16 g) as a foam-like solid.

### Production Example 40

To a mixture of tert-butyl (3S)-3-({6-cyclopropyl-2-(ethanesulfonyl)-7-[6-fluoro-5-methyl-2-(triphenylmethyl)-2H-indazol-4-yl]-8-hydroxyquinolin-4-yl}]oxy)pyrrolidine-1-carboxylate (1.16 g), methyl 4-(chloromethyl)benzoate (375 mg) and DMF (12 mL) was added cesium carbonate (2 g) at room temperature, and the mixture was stirred at room temperature for 6 hours. Ethyl acetate and water were added to the reaction mixture, two layers were separated, and the aqueous layer was extracted with ethyl acetate. The combined organic layer was washed with water and saturated aqueous sodium chloride solution and was dried over anhydrous magnesium sulfate. The insoluble materials were removed by filtration, and the filtrate was concentrated under reduced pressure. Then, the resulting residue was purified by silica gel column chromatography (hexane/ethyl acetate), thus obtaining tert-butyl (3S)-3-({6-cyclopropyl-2-(ethanesulfonyl)-7-[6-fluoro-5-methyl-2-(triphenylmethyl)-2H-indazol-4-yl]-8-{[4-(methoxycarbonyl)phenyl]methoxy}quinolin-4-yl}]oxy}pyrrolidine-1-carboxylate (1.22 g) as a foam-like solid.

### Production Example 41

To a THF (10 mL) solution of tert-butyl (3S)-3-{[6-cyclopropyl-2-(ethanesulfonyl)-7-[6-fluoro-5-methyl-2-(triphenylmethyl)-2H-indazol-4-yl]-8-{[4-(methoxycarbonyl)phenyl]methoxy}quinolin-4-yl]oxy}pyrrolidine-1-carboxylate (835 mg) and (2S)-2-methoxypropan-1-ol (97 µL) was added tBuOK (114 mg) under ice-bath cooling, and the mixture was stirred at the same temperature for 2 hours. Ice water and aqueous ammonium chloride solution were added to the reaction solution to quench the reaction, and the mixture was extracted with ethyl acetate twice. The combined organic layer was washed with saturated aqueous sodium chloride solution, was dried over anhydrous magnesium sulfate and was concentrated under reduced pressure. The resulting residue was purified by silica gel column chromatography (hexane/ethyl acetate), thus obtaining tert-butyl (3S)-3-({6-cyclopropyl-7-[6-fluoro-5-methyl-2-(triphenylmethyl)-2H-indazol-4-yl]-8-{[4-(methoxycarbonyl)phenyl]methoxy}-2-[(2S)-2-methoxypropoxy]quinolin-4-yl}]oxy}pyrrolidine-1-carboxylate (374 mg) as a foam-like solid.

### Production Example 42

To a solution in MeOH (2 mL) and THF (2 mL) of tert-butyl (3S)-3-({6-cyclopropyl-7-[6-fluoro-5-methyl-2-(triphenylmethyl)-2H-indazol-4-yl]-8-{[4-(methoxycarbonyl)phenyl]methoxy}-2-[(2S)-2-methoxypropoxy]quinolin-4-yloxy)pyrrolidine-1-carboxylate (250 mg) was added aqueous sodium hydroxide solution (1 M, 1.2 mL) at room temperature, and the mixture was stirred at room temperature for 6 hours. Under ice-bath cooling, ethyl acetate and saturated aqueous ammonium chloride solution were added, and the organic layer was extracted, was washed with saturated aqueous sodium chloride solution, was dried over anhydrous sodium sulfate and was concentrated under reduced pressure. iPr₂O was added to the resulting residue, and the precipitated solid was filtered and washed with iPr₂O to give 4-{[(4-{[(3S)-1-(tert-butoxycarbonyl)pyrrolidin-3-yl]oxy}-6-cyclopropyl-7-[6-fluoro-5-methyl-2-(triphenylmethyl)-2H-indazol-4-yl]-2-[(2S)-2-methoxypropoxy]quinolin-8-yl}oxy)methyl]benzoic acid (216 mg) as a solid.

### Production Example 43

To a mixture of 4-bromo-6-fluoro-1H-indazole (235 g), TEA (183 mL) and CH₂Cl₂ (1880 mL) was added 1,1',1"-(chloromethanetriyl)tribenzene (335 g) at room temperature, and the mixture was stirred at 25°C for 16 hours. The reaction mixture was poured into ice water (1.5 L), and the organic layer and the aqueous layer were separated. The aqueous layer was extracted with CH₂Cl₂ (400 mL) three times. The combined organic layer was dried over anhydrous sodium sulfate. Then, the insoluble materials were removed by filtration, and the filtrate was concentrated under reduced pressure. Petroleum ether (550 mL) was added to the resulting residue for trituration (0°C, 2 hours), and then 4-bromo-6-fluoro-2-(triphenylmethyl)-2H-indazole (508.98 g) was obtained as a solid by collecting by filtration and drying under reduced pressure.

### Production Example 44

To a mixture of 4-bromo-6-fluoro-2-(triphenylmethyl)-2H-indazole (100 g) and 2-methyltetrahydrofuran (1000 mL) was added lithium diisopropylamide (2 M THF solution, 214.28 mL) at -78°C under nitrogen atmosphere, and the mixture was stirred at -78°C for 2.5 hours. Methyl iodide (26.68 mL) was added at -78°C, and the mixture was stirred at 25°C for 2.5 hours. Water (2000 mL) was added to quench the reaction, and the mixture was extracted with ethyl acetate (800 mL) twice. The combined organic layer was dried over anhydrous sodium sulfate. Then, the insoluble materials were removed by filtration, and the filtrate was concentrated under reduced pressure. Ethyl acetate (50 mL)/petroleum ether (50 mL) were added to the resulting residue for trituration, and then 4-bromo-6-fluoro-5-methyl-2-(triphenylmethyl)-2H-indazole (81 g) was obtained as a solid by collecting by filtration and drying under reduced pressure.

### Production Example 45

To a mixture of 4-bromo-6-fluoro-5-methyl-2-(triphenylmethyl)-2H-indazole (100 g), 4,4,4',4',5,5,5',5'-octamethyl-2,2'-bi-1,3,2-dioxaborolane (61.42 g), triphenylphosphine (10.57 g), potassium acetate (59.34 g) and DOX (1000 mL) was added palladium acetate (4.52 g) under nitrogen atmosphere at room temperature. After the reaction mixture was degassed and filled with nitrogen gas three times each, the mixture was stirred at 100°C for 12 hours under nitrogen atmosphere. After cooling, water (1500 mL) was added, and the mixture was extracted with ethyl acetate (900 mL) three times. The combined organic layer was dried over anhydrous sodium sulfate, and then the insoluble materials were removed by filtration. Activated carbon (50 g) was added to the resulting solution, and the solution was stirred at 20°C for 1 hour and filtered while washing with ethyl acetate (50 mL) three times. The filtrate was concentrated. MeOH (200 mL) was added to the resulting residue for trituration, and 6-fluoro-5-methyl-4-(4,4,5,5-tetramethyl-1,3,2-dioxaborolan-2-yl)-2-(triphenylmethyl)-2H-indazole (110 g) was obtained as a solid by collecting by filtration and drying under reduced pressure.

### Production Example 48

To a mixture of tert-butyl (1S,4S)-5-{(7M)-6-cyclopropyl-7-[6-fluoro-5-methyl-1-(oxan-2-yl)-1H-indazol-4-yl]-8-hydroxy-2-[(oxan-4-yl)oxy]quinazolin-4-yl}-2,5-diazabicyclo[2.2.1]heptane-2-carboxylate (200 mg),4-(chloromethyl)benzaldehyde (60 mg) and DMF (4 mL) was added cesium carbonate (160 mg), and the mixture was stirred at room temperature for 2 hours. Ethyl acetate and water were added, two layers were separated, and the aqueous layer was extracted with ethyl acetate. The combined organic layer was washed with water and saturated aqueous sodium chloride solution, was dried over anhydrous sodium sulfate and was filtered. The filtrate was concentrated under reduced pressure. The residue was purified by silica gel chromatography (CHCl₃/MeOH), thus obtaining tert-butyl (1S,4S)-5-{(7M)-6-cyclopropyl-7-[6-fluoro-5-methyl-1-(oxan-2-yl)-1H-indazol-4-yl]-8-[(4-formylphenyl)methoxy]-2-[(oxan-4-yl)oxy]quinazolin-4-yl}-2,5-diazabicyclo[2.2.1]heptane-2-carboxylate (165 mg) as an oil.

### Production Example 49

A mixture of tert-butyl (1S,45)-5-{(7M)-6-cyclopropyl-7-[6-fluoro-5-methyl-1-(oxan-2-yl)-1H-indazol-4-yl]-8-[(4-formylphenyl)methoxy]-2-[(oxan-4-yl)oxy]quinazolin-4-yl}-2,5-diazabicyclo[2.2.1]heptane-2-carboxylate (60 mg), {{2-[2,4-dihydroxy-5-(propan-2-yl)benzoyl]-2,3-dihydro-1H-isoindol-5-yl}(piperazin-1-yl)methanone monohydrochloride (55 mg), MeOH (1 mL) and acetic acid (0.1 mL) was stirred at room temperature for 30 minutes. Then, 2-methylpyridine borane (15 mg) was added, and the mixture was stirred at room temperature for 2 hours. Hydrochloric acid (1 M) was added at room temperature, and the mixture was stirred at room temperature for 15 minutes. Then, water and saturated aqueous sodium hydrogen carbonate solution were added, and the mixture was extracted twice with CHCl₃/MeOH (9/1). The organic layer was dried over anhydrous sodium sulfate and was then filtered and was concentrated under reduced pressure. The residue was purified by silica gel column chromatography (CHCl₃/MeOH), thus obtaining tert-butyl (1S,4S)-5-{(7M)-6-cyclopropyl-8-({4-[(4-{2-[2,4-dihydroxy-5-(propan-2-yl)benzoyl]-2,3-dihydro-1H-isoindole-5-carbonyl}piperazin-1-yl)methyl]phenyl}methoxy)-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (20 mg) as a solid.

### Production Example 50

To a mixture of 4-[({(7M)-4-[(1 S,4S)-5-(tert-butoxycarbonyl)-2,5-diazabicyclo[2.2.1]heptan-2-yl]-6-cyclopropyl-7-[6-fluoro-5-methyl-1-(oxan-2-yl)-1H-indazol-4-yl]-2-[(oxan-4-yl)oxy]quinazolin-8-yl}oxy)methyl]benzoic acid (60 mg), {2-[2,4-dihydroxy-5-(propan-2-yl)benzoyl]-2,3-dihydro-1H-isoindol-5-yl}(piperazin-1-yl)methanone monohydrochloride (38 mg), DIPEA (50 µL) and DMF (1 mL) was added HATU (35 mg) under ice-bath cooling, and the mixture was stirred at room temperature for 1 hour. Water and saturated aqueous sodium hydrogen carbonate solution were added to the reaction solution, and the mixture was extracted with ethyl acetate. The organic layer was washed twice with saturated aqueous sodium chloride solution, was dried over anhydrous sodium sulfate and was then concentrated under reduced pressure. The residue was purified by silica gel column chromatography (CHCl₃/MeOH), thus obtaining tert-butyl (1S,4S)-5-{(7M)-6-cyclopropyl-8-{[4-(4-{2-[2,4-dihydroxy-5-(propan-2-yl)benzoyl]-2,3-dihydro-1H-isoindole-5-carbonyl}piperazine-1-carbonyl)phenyl}methoxy}-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (74 mg) as an oil.

### Production Example 67

To a mixture of 2-[2,4-dihydroxy-5-(propan-2-yl)benzoyl]-2,3-dihydro-1H-isoindole-5-carboxylate (100 mg), tert-butyl piperazine-1-carboxylate (65 mg), DIPEA(100 µL) and DMF(1 mL) was added HATU (35 mg) under ice-bath cooling, and the mixture was stirred at room temperature for 4 hours. Ethyl acetate, water and saturated aqueous sodium hydrogen carbonate solution were added, and the aqueous layer was separated. The organic layer was washed twice with saturated aqueous sodium chloride solution, was dried over anhydrous sodium sulfate and was then filtered. The filtrate was concentrated under reduced pressure. The residue was purified by silica gel column chromatography (CHCl₃/MeOH), thus obtaining tert-butyl 4-{2-[2,4-dihydroxy-5-(propan-2-yl)benzoyl]-2,3-dihydro-1H-isoindole-5-carbonyl}piperazine-1-carboxylate (147 mg) as an oil.

### Production Example 70

To a mixture of tert-butyl 4-{2-[2,4-dihydroxy-5-(propan-2-yl)benzoyl]-2,3-dihydro-1H-isoindole-5-carbonyl}piperazine-1-carboxylate (145 mg) and CH₂Cl₂ (2 mL) was added hydrogen chloride (4 M DOX solution, 1 mL) under ice-bath cooling, and the mixture was stirred at room temperature for 2 hours. The reaction mixture was concentrated under reduced pressure to give {2-[2,4-dihydroxy-5-(propan-2-yl)benzoyl]-2,3-dihydro-1H-isoindol-5-yl}(piperazin-1-yl)methanone monohydrochloride (136 mg) as a solid.

### Production Example 73

To a CH₂Cl₂ (2 mL) solution of tert-butyl (2S)-2-methylpiperazine-1-carboxylate (90 mg) and 2-[2,4-bis(benzyloxy)-5-(propan-2-yl)benzoyl]-2,3-dihydro-1H-isoindole-5-carbaldehyde (120 mg) was added acetic acid (10 µL) at room temperature, and the mixture was stirred for 30 minutes. Then, sodium triacetoxyborohydride (100 mg) was added, and the mixture was stirred at room temperature for 2 hours. Water and saturated aqueous sodium hydrogen carbonate solution were added at room temperature, and the mixture was stirred for a while and was extracted with CHCl₃. The organic layer was dried over anhydrous magnesium sulfate, was filtered and was concentrated under reduced pressure. The residue was purified by silica gel column chromatography (hexane/ethyl acetate), thus obtaining tert-butyl (2S)-4-({2-[2,4-bis(benzyloxy)-5-(propan-2-yl)benzoyl]-2,3-dihydro-1H-isoindol-5-yl}methyl)-2-methylpiperazine-1-carboxylate (159 mg) as an oil.

### Production Example 81

To tert-butyl (2S)-4-({2-[2,4-bis(benzyloxy)-5-(propan-2-yl)benzoyl]-2,3-dihydro-1H-isoindol-5-yl}methyl)-2-methylpiperazine-1-carboxylate (70 mg) were added trifluoroacetic acid (2 mL) and trifluoromethanesulfonic acid (0.2 mL) at room temperature, and the mixture was stirred at 60°C for 2 hours. The mixture was concentrated under reduced pressure, and the residue was purified by silica gel column chromatography (basic silica gel, CHCl₃/MeOH), thus obtaining [2,4-dihydroxy-5-(propan-2-yl)phenyl](5-{[(3S)-3-methylpiperazin-1-yl]methyl}-1,3-dihydro-2H-isoindol-2-yl)methanone (58 mg) as a solid.

### Production Example 86

A solution in trifluoroacetic acid (10 mL) and trifluoromethanesulfonic acid (1 mL) of tert-butyl 4-({2-[2,4-bis(benzyloxy)-5-(propan-2-yl)benzoyl]-2,3-dihydro-1H-isoindol-5-yl}methyl)piperazine-1-carboxylate (2.80 g) was stirred at 60°C for 12 hours. The solution was concentrated under reduced pressure, and the resulting crude product was purified by reverse phase HPLC (40% aqueous MeCN solution/0.1% hydrochloric acid), thus obtaining [2,4-dihydroxy-5-(propan-2-yl)phenyl]{5-[(piperazin-1-yl)methyl]-1,3-dihydro-2H-isoindol-2-yl}methanone n trifluoromethanesulfonate (376 mg) as a solid.

### Production Example 90

To a mixture of benzyl 4-{[1-(tert-butoxycarbonyl)azetidin-3-yl]methyl}piperazine-1-carboxylate (649 mg) and CH₂Cl₂ (7 mL) was added trifluoroacetic acid (1 mL) under ice-bath cooling, and the mixture was stirred at room temperature overnight and was concentrated under reduced pressure, thus obtaining benzyl 4-[(azetidin-3-yl)methyl]piperazine-1-carboxylate n trifluoroacetate (1.12 g) as an oil.

### Production Example 92

To a mixture of benzyl 4-{[1-({2-[2,4-bis(benzyloxy)-5-(propan-2-yl)benzoyl]-2,3-dihydro-1H-isoindol-5-yl}methyl)azetidin-3-yl]methyl}piperazine-1-carboxylate (98 mg) and 10% Pd/C (50% water content, 45 mg) was added MeOH (2 mL) at room temperature, and the mixture was stirred for 2 hours under a hydrogen atmosphere. 10% Pd/C (50% water content, 30 mg) was added, and the mixture was stirred at room temperature for 2 hours under a hydrogen atmosphere. The mixture was filtered through celite (registered trademark) pad and washed with MeOH. The filtrate was concentrated under reduced pressure, thus obtaining [2,4-dihydroxy-5-(propan-2-yl)phenyl][5-({3-[(piperazin-1-yl)methyl]azetidin-1-yl}methyl)-1,3-dihydro-2H-isoindol-2-yl]methanone (51 mg) as a solid.

### Production Example 93

To a THF (50 mL) solution of 2-(dimethylamino)ethylamine (6 mL) was added 1,1'-carbonylbis-1H-imidazole (8.9 g) under ice-bath cooling, and the mixture was stirred at room temperature for 2 hours. The reaction solution was concentrated under reduced pressure, iPrOH (50 mL) and azetidin-3-ol monohydrochloride (5 g) were added to the residue, and the mixture was stirred at 80°C for 3 hours under an argon atmosphere. The reaction solution was concentrated under reduced pressure, and the residue was purified by silica gel column chromatography (CHCl₃/MeOH/ammonia water), thus obtaining N-[2-(dimethylamino)ethyl]-3-hydroxyazetidine-1-carboxamide (9.67 g) as a liquid.

### Production Example 94

To a mixture of 2-(dimethylamino)ethan-1-ol (120 mg), DMF (5 mL) and DIPEA (1.7 mL) was added bis(4-nitrophenyl) carbonate (450 mg) under ice-bath cooling, and the mixture was stirred at room temperature for 2 hours. Benzyl azetidin-3-yl(methyl)carbamate monohydrochloride (260 mg) was added to the reaction solution at room temperature and was stirred at room temperature overnight under an argon atmosphere. Ice and aqueous ammonium chloride solution were added to the reaction solution, and the mixture was extracted twice with ethyl acetate. The organic layer was dried over anhydrous magnesium sulfate, and the solvent was concentrated under reduced pressure. The residue was purified by silica gel column chromatography (basic silica gel, CHCl₃), thus obtaining 2-(dimethylamino)ethyl 3-{[(benzyloxy)carbonyl](methyl)amino}azetidine-1-carboxylate (320 mg) as an oil.

### Production Example 95

A mixture of 2-(dimethylamino)ethyl 3-{[(benzyloxy)carbonyl](methyl)amino}azetidine-1-carboxylate (313 mg), MeOH (6 mL) and 10% Pd/C (52% water content, 110 mg) was stirred at room temperature for 3 hours under a hydrogen atmosphere. Celite (registered trademark) was added, and the mixture was filtered through celite (registered trademark) pad while washing with ethyl acetate. The filtrate was concentrated under reduced pressure, and the residue was purified by silica gel column chromatography (basic silica gel, CHCl₃), thus obtaining 2-(dimethylamino)ethyl 3-(methylamino)azetidine-1-carboxylate (134 mg) as an oil.

### Production Example 96

To a THF (4 mL) solution of benzyl 4-(3-hydroxypropyl)piperazine-1-carboxylate (311 mg) was added sodium hydride (45 mg, about 60% by weight mineral oil dispersion) under ice-bath cooling, and the mixture was warmed to room temperature and stirred for 30 minutes under an argon atmosphere. The reaction mixture was cooled with ice bath, and tert-butyl 2-chloro-7,8-dihydropyrido[4,3-d]pyrimidine-6(5H)-carboxylate (200 mg) was added. The mixture was stirred under ice-bath cooling for 30 minutes and at room temperature for 48 hours. The reaction was quenched with saturated aqueous ammonium chloride solution under ice-bath cooling. Water and ethyl acetate were added, and the organic layer and the aqueous layer were separated. The aqueous layer was extracted with ethyl acetate, and the combined organic layer was dried over anhydrous sodium sulfate, was filtered and was concentrated under reduced pressure. The residue was purified by silica gel column chromatography (CHCl₃/MeOH), thus obtaining tert-butyl 2-(3-{4-[(benzyloxy)carbonyl]piperazin-1-yl}propoxy)-7,8-dihydropyrido[4,3-d]pyrimidine-6(5H)-carboxylate (265 mg) as an oil.

In the same manner as in the production methods of the Production Examples described above, the compounds shown in the tables presented later were produced. In addition, the production method, the structure and the physiochemical data of the compound of each Production Example are shown in the tables presented later.

### Example 1

To a mixture of tert-butyl (1S,45)-5-{(7M)-6-cyclopropyl-8-{[4-(4-{2-[2,4-dihydroxy-5-(propan-2-yl)benzoyl]-2,3-dihydro-1H-isoindole-5-carbonyl } piperazine-1-carbonyl)phenyl]methoxy}-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (72 mg) and CH₂Cl₂ (1 mL) was added trifluoroacetic acid (0.5 mL) under ice-bath cooling, and the mixture was stirred at room temperature for 2 hours. The mixture was concentrated under reduced pressure, and the residue was purified by ODS column chromatography (0.1% formic acid in MeCN solution/0.1% aqueous formic acid solution). Saturated aqueous sodium hydrogen carbonate solution was added to a fraction containing the target compound, and the mixture was then extracted twice with CHCl₃/MeOH (9/1). The organic layer was dried over anhydrous sodium sulfate and was concentrated under reduced pressure. The residue was washed with Et₂O and was dried under reduced pressure, thus obtaining [5-(4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}piperazine-1-carbonyl)-1,3-dihydro-2H-isoindol-2-yl][2,4-dihydroxy-5-(propan-2-yl)phenyl]methanone (15 mg) as a solid.

### Example 5

Under an argon atmosphere, to a CH₂Cl₂ (2 mL) solution of tert-butyl (1S,4S)-5-{(7M)-6-cyclopropyl-8-[(4-{4-[(4-{3-[2,4-dihydroxy-5-(propan-2-yl)phenyl]-5-hydroxy-4H-1,2,4-triazol-4-yl}phenyl)methyl]piperazine-1-carbonyl}phenyl)methoxy]-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (53 mg) and triisopropylsilane (20 µL) was added trifluoroacetic acid (0.5 mL) under ice-bath cooling. The mixture was stirred at room temperature for 3 hours and was concentrated under reduced pressure. The residue was purified by ODS column chromatography (0.1% formic acid in MeCN solution/0.1% aqueous formic acid solution). Saturated aqueous sodium hydrogen carbonate solution was added to a fraction containing the target compound under ice-bath cooling, and the mixture was diluted with water and CHCl₃/iPrOH (4/1). Two layers were separated, and the aqueous layer was extracted with CHCl₃/iPrOH (4/1). The combined organic layer was dried over anhydrous sodium sulfate, was filtered and was concentrated under reduced pressure. The resulting solid was washed with Et₂O, thus obtaining {4-[({(7M)-6-cyclopropyl-4-[(1 S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}{4-[(4-{3-[2,4-dihydroxy-5-(propan-2-yl)phenyl]-5-hydroxy-4H-1,2,4-triazol-4-yl}phenyl)methyl]piperazin-1-yl}methanone (17.6 mg) as a solid.

In the same manner as in the production methods of the Examples described above, the Example compounds shown in the tables presented later were produced. In addition, the production method and the physiochemical data of the compound of each Example are shown in tables presented later.

In the tables presented below, the following abbreviations are sometimes used.

PEx: Production Example No., Ex: Example No., PSyn: Production Example No. produced in the same method, Syn: Example No. produced in the same method (for example, Syn: 9 represents that it was produced by the same method as for Example 9), Str: chemical structural formula (a compound with "*" in the chemical structural formula represents that the compound is single structure with regard to the axial chirality or central chirality), n HCl: n hydrochloride (the compound with Production Example No. shows that the compound is monohydrochloride to tetrahydrochloride), n TfOH: n trifluoromethanesulfonate (the compound with Production Example No. shows that the compound is monotrifluoromethanesulfonate to tritrifluoromethanesulfonate), n TFA: n trifluoroacetate (the compound with Production Example No. shows that the compound is monotrifluoroacetate to tritrifluoroacetate), DAT: physiochemical data, ESI+: m/z value in mass spectrometry (ionization method ESI, [M+H]⁺ unless otherwise specified), ESI-: m/z value in mass spectrometry (ionization method ESI, [M-H]⁻ unless otherwise specified), NMR: δ value (ppm) of peak in ¹H-NMR (500 MHz) in DMSO-d₆ at 27°C, NMR (400 MHz): δ value (ppm) of peak in ¹H-NMR (400 MHz) in DMSO-d₆ at 27°C, NMR (90°C): δ value (ppm) of peak in ¹H-NMR (500 MHz) in DMSO-d₆ at 90°C, s: singlet (spectrum), d: doublet (spectrum), dd: double doublet (spectrum), t: triplet (spectrum), tt: triple triplet (spectrum), dq: double quartet (spectrum), q: quartet (spectrum), m: multiplet (spectrum), br: broad (spectrum) (example: br s).

**[Table 3-1]**

| PEx | Str |
|---|---|
| 1 | 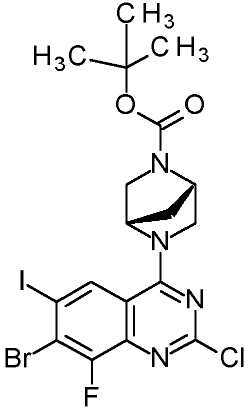 |
| 2 | 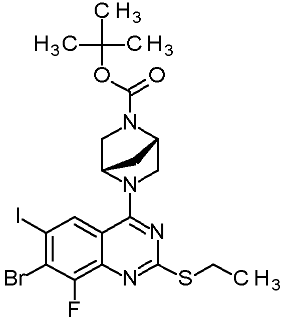 |
| 3 | 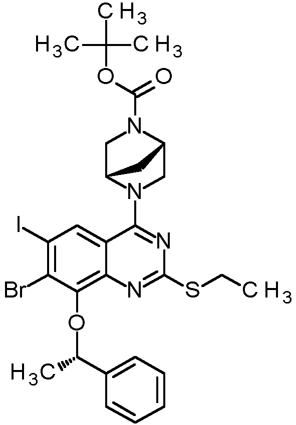 |

**[Table 3-2]**

| PEx | Str |
|---|---|
| 4 | 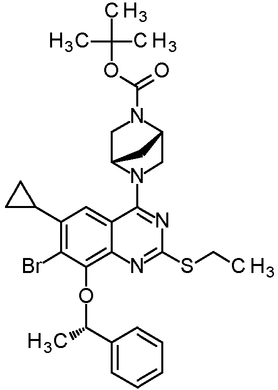 |
| 5 | 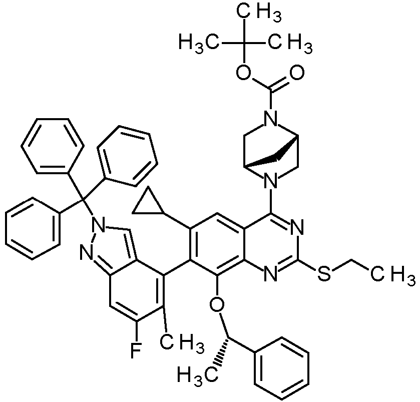 |
| 6 | 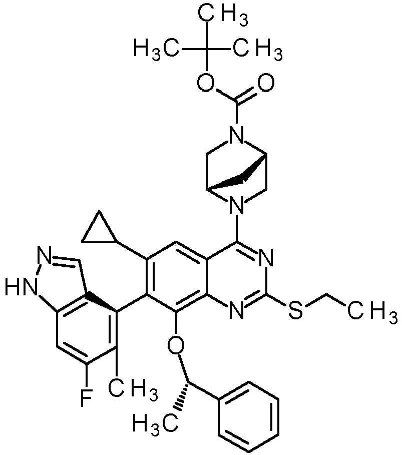 |

**[Table 3-3]**

| PEx | Str |
|---|---|
| 7 | 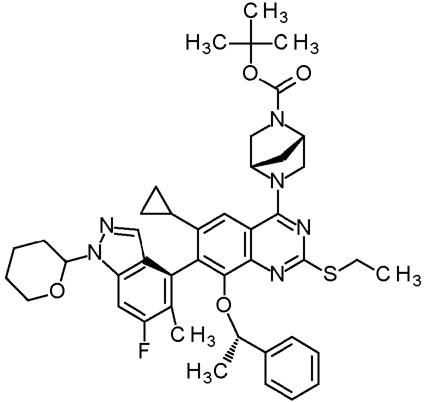 |
| 8 | 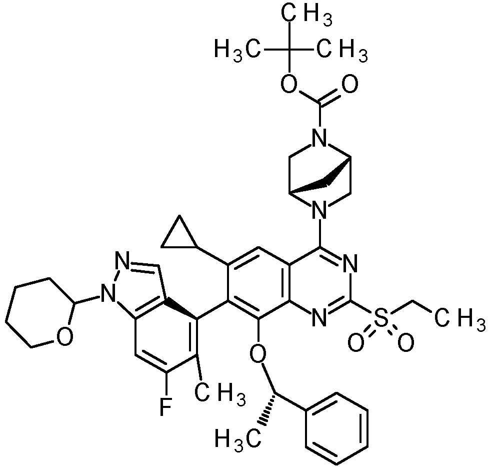 |
| 9 | 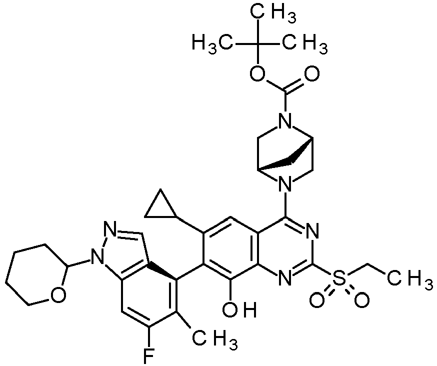 |

**[Table 3-4]**

| PEx | Str |
|---|---|
| 10 | 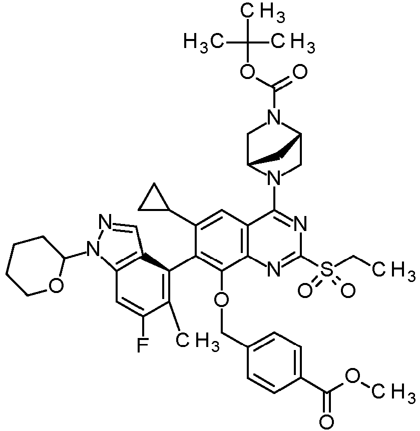 |
| 11 | 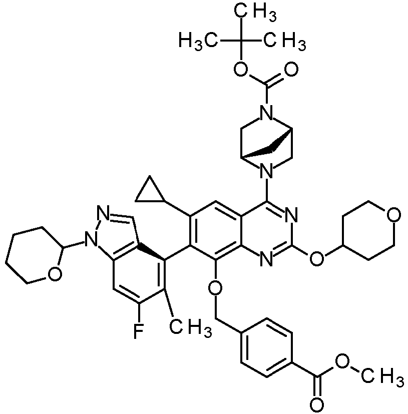 |
| 12 | 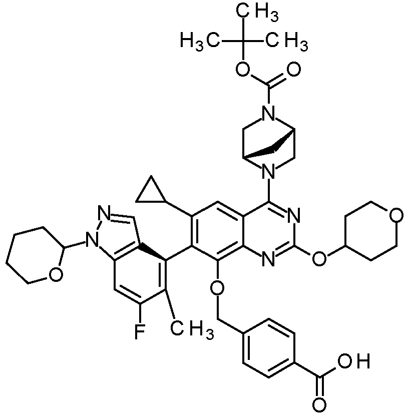 |

**[Table 3-5]**

| PEx | Str | PEx | Str |
|---|---|---|---|
| 13 | 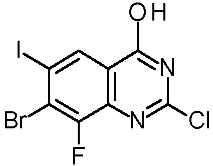 | 16 | 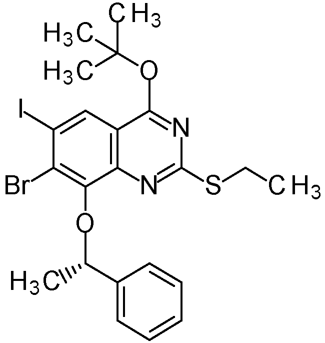 |
| 14 | 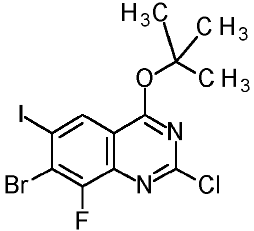 | 17 | 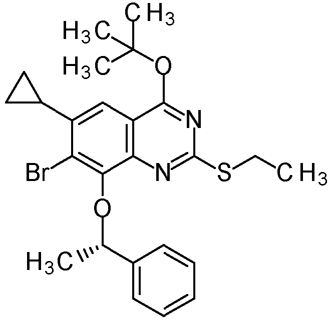 |
| 15 | 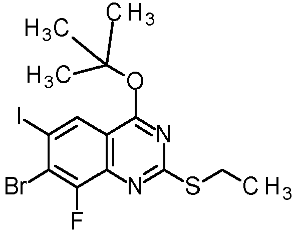 | | |

**[Table 3-6]**

| PEx | Str |
|---|---|
| 18 | 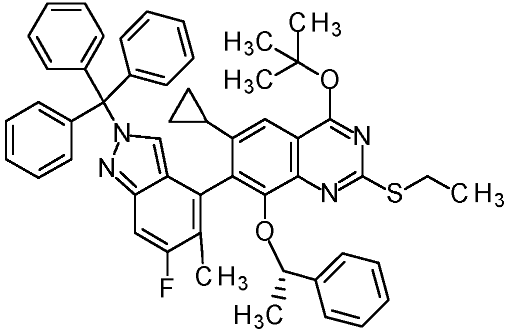 |
| 19 | 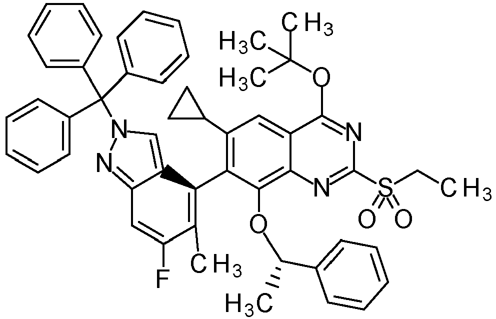 |
| 20 | 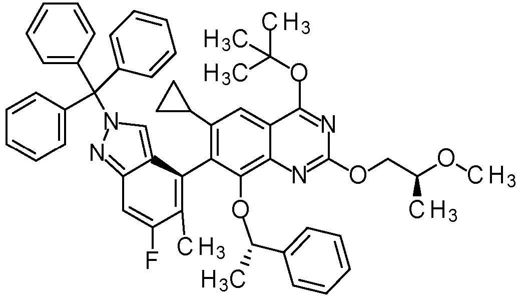 |

**[Table 3-7]**

| PEx | Str |
|---|---|
| 21 | 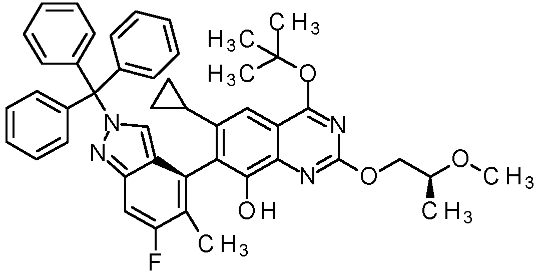 |
| 22 | 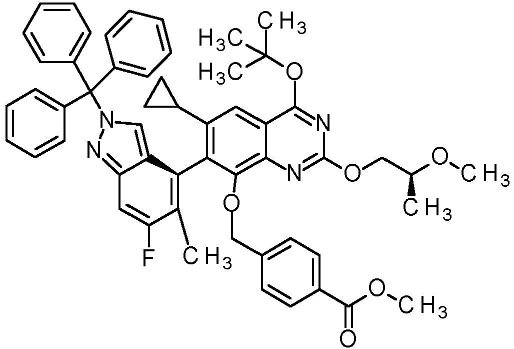 |
| 23 | 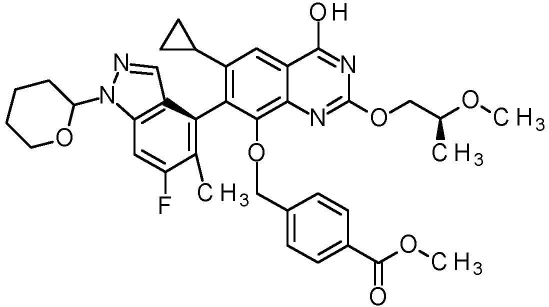 |

**[Table 3-8]**

| PEx | Str |
|---|---|
| 24 | 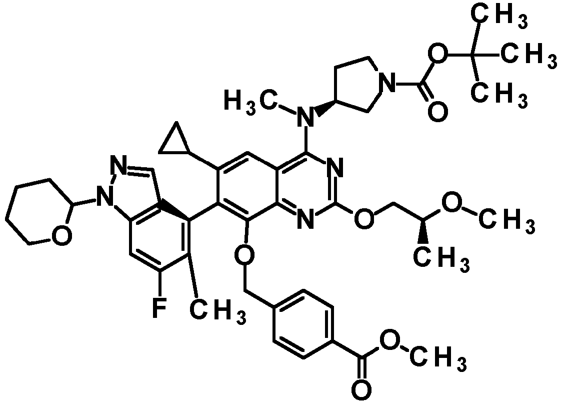 |
| 25 | 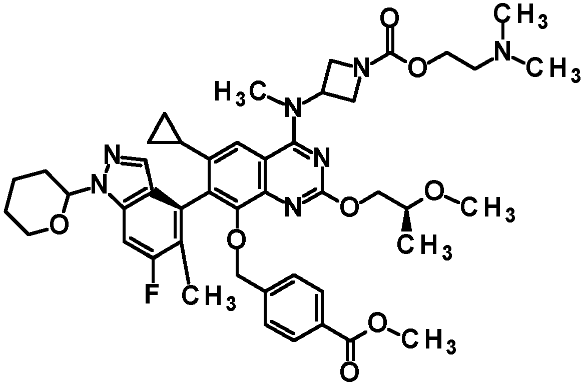 |
| 26 | 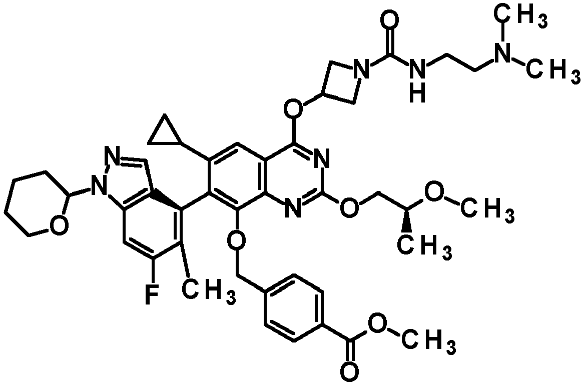 |

**[Table 3-9]**

| PEx | Str |
|---|---|
| 27 | 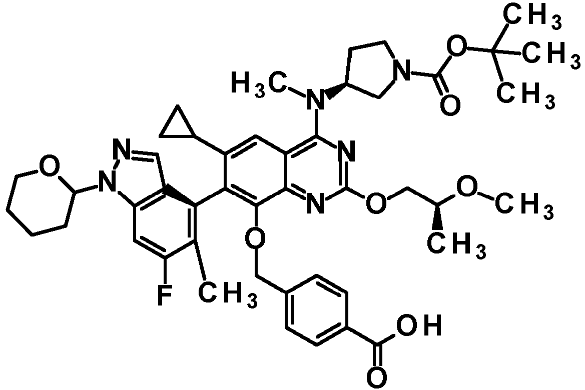 |
| 28 | 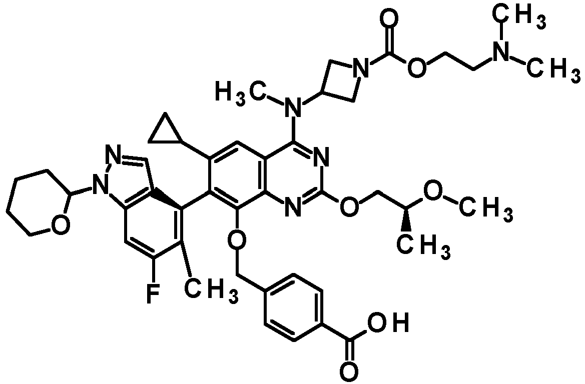 |
| 29 | 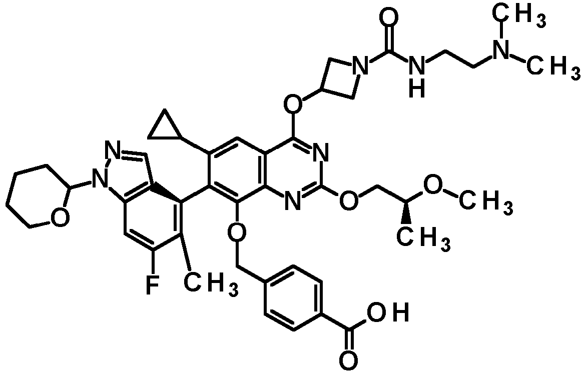 |

**[Table 3-10]**

| PEx | Str | PEx | Str |
|---|---|---|---|
| 30 | 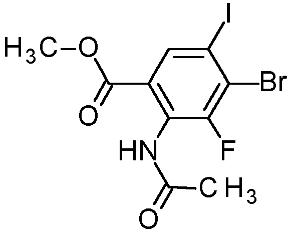 | 33 | 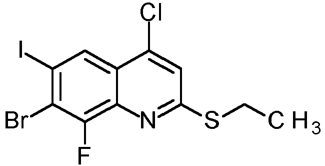 |
| 31 | 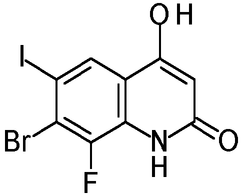 | 34 | 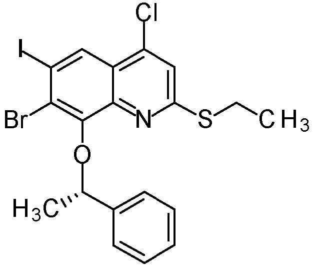 |
| 32 | 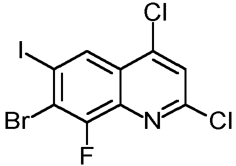 | 35 | 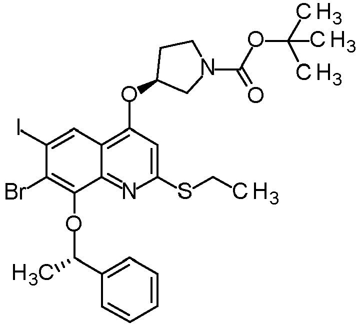 |

**[Table 3-11]**

| PEx | Str |
|---|---|
| 36 | 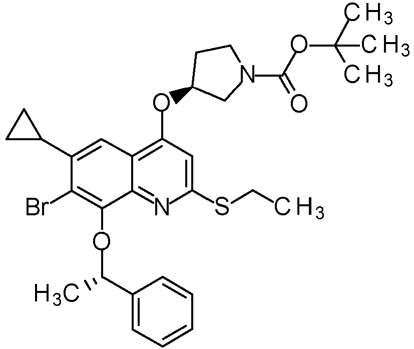 |
| 37 | 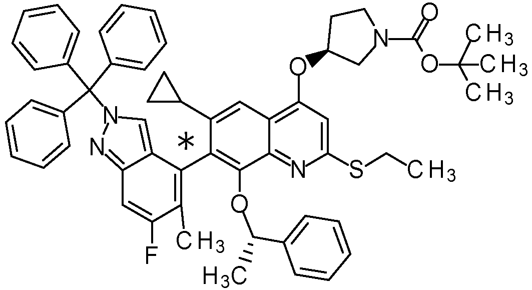 |
| 38 | 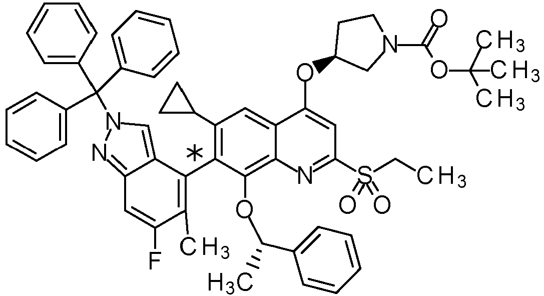 |

**[Table 3-12]**

| PEx | Str |
|---|---|
| 39 | 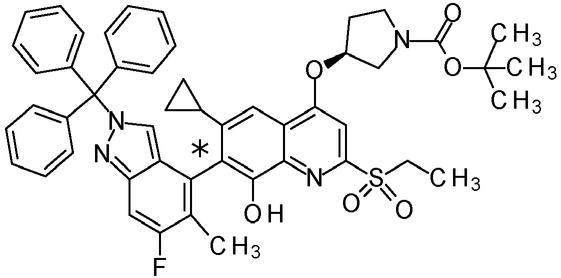 |
| 40 | 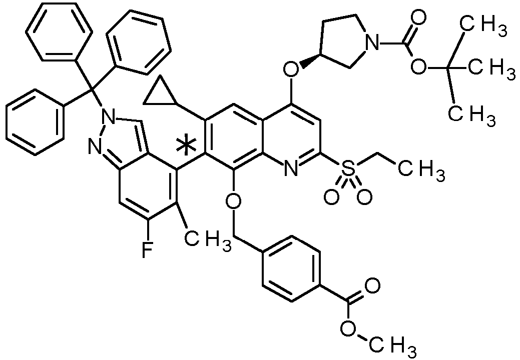 |
| 41 | 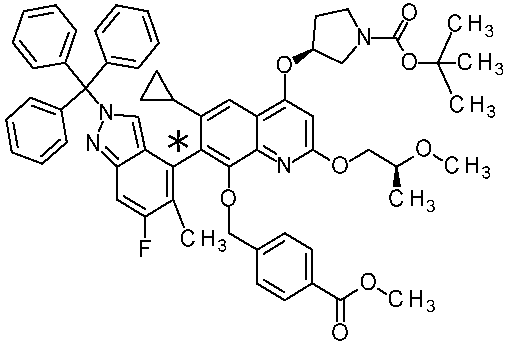 |

**[Table 3-13]**

| PEx | Str |
|---|---|
| 42 | 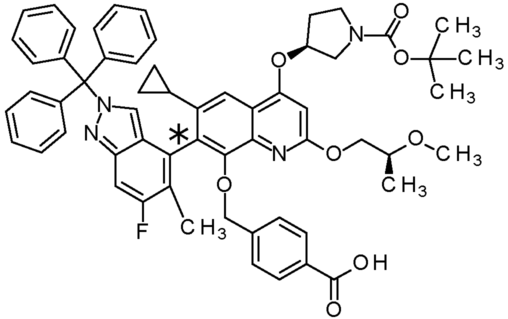 |
| 43 | 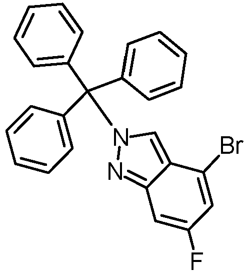 |
| 44 | 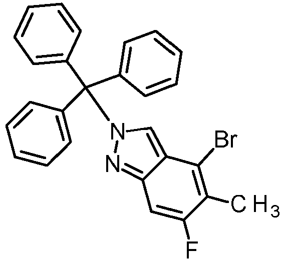 |
| 45 | 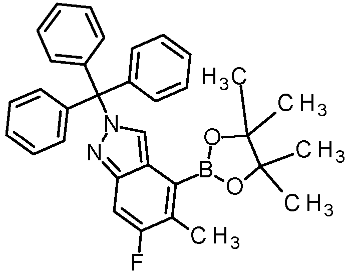 |

**[Table 3-14]**

| PEx | Str |
|---|---|
| 46 | 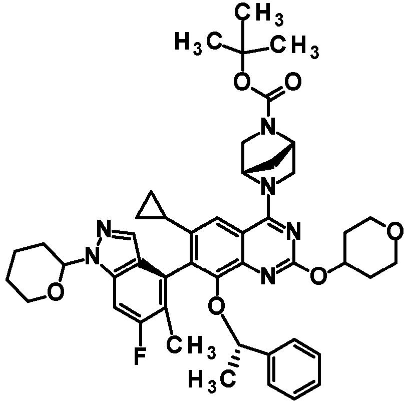 |
| 47 | 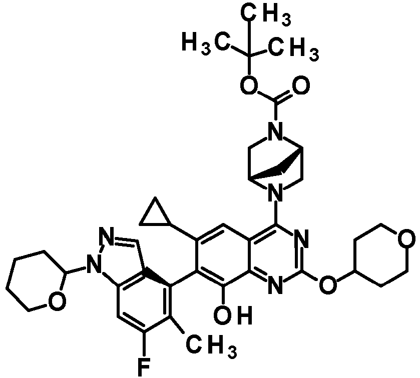 |
| 48 | 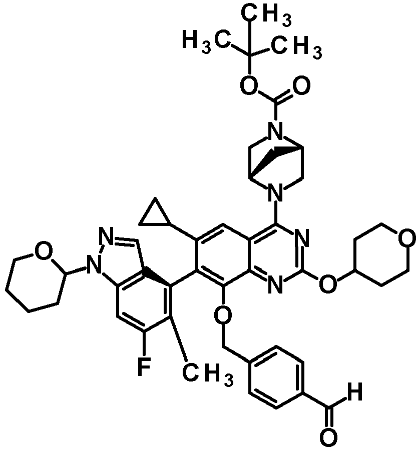 |

**[Table 3-15]**

| PEx | Str |
|---|---|
| 49 | 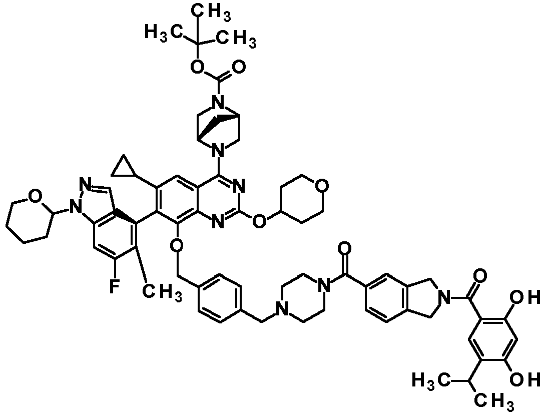 |
| 50 | 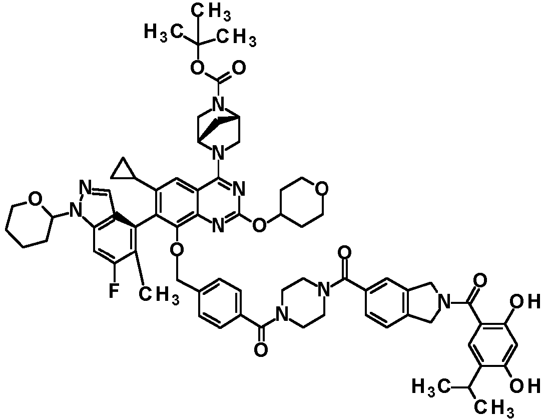 |
| 51 | 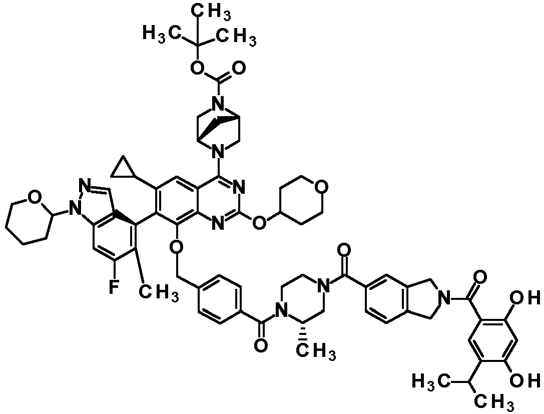 |

**[Table 3-16]**

| PEx | Str |
|---|---|
| 52 | 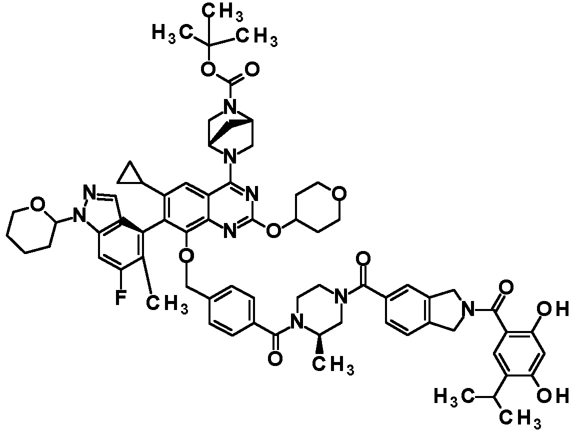 |
| 53 | 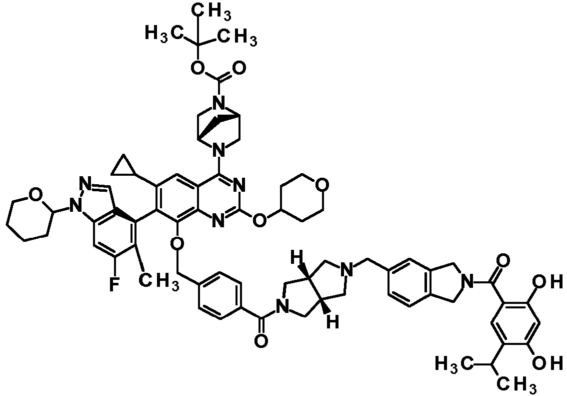 |
| 54 | 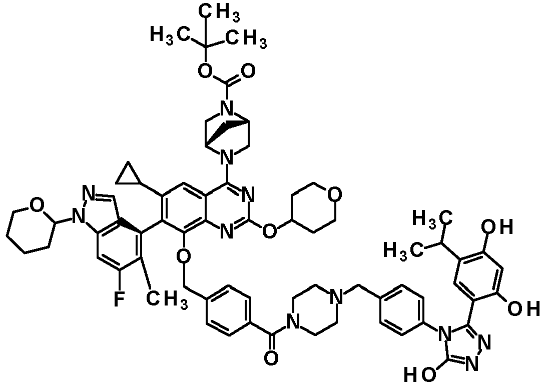 |

**[Table 3-17]**

| PEx | Str |
|---|---|
| 55 | 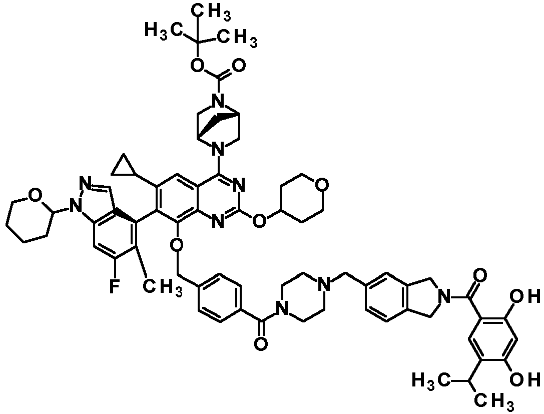 |
| 56 | 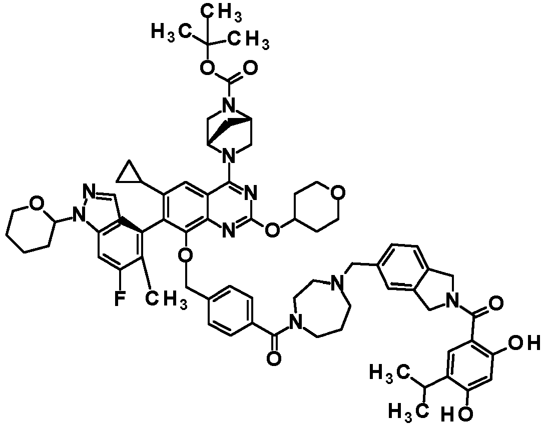 |
| 57 | 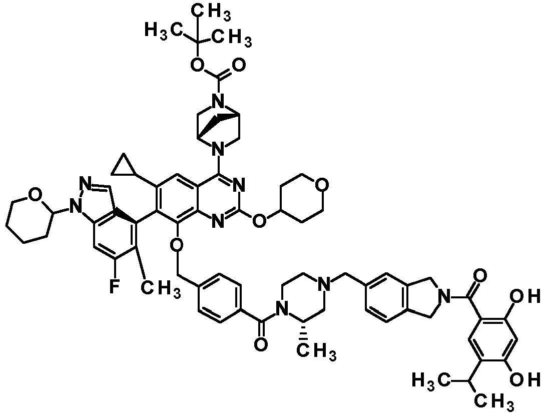 |

**[Table 3-18]**

| PEx | Str |
|---|---|
| 58 | 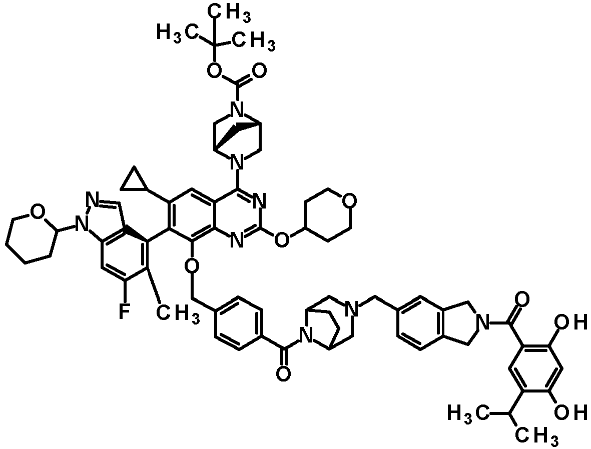 |
| 59 | 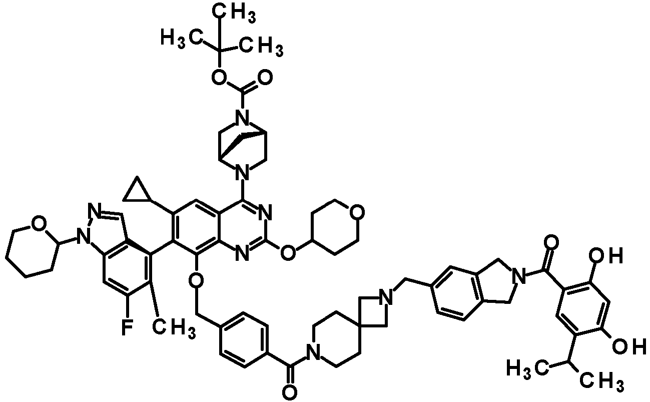 |
| 60 | 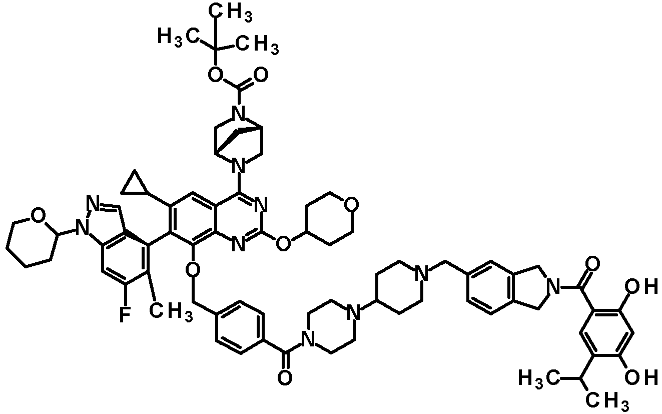 |

**[Table 3-19]**

| PEx | Str |
|---|---|
| 61 | 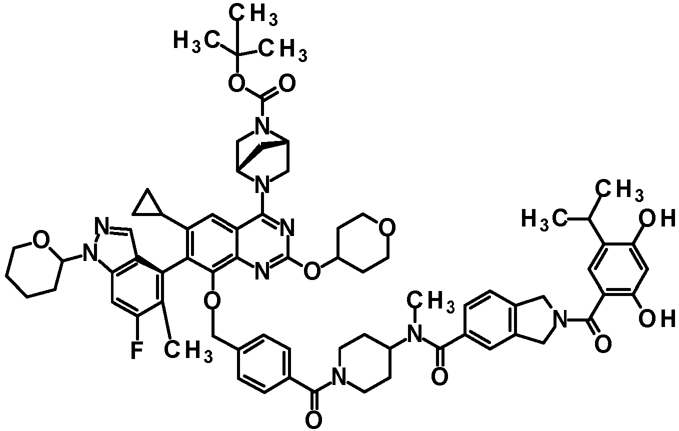 |
| 62 | 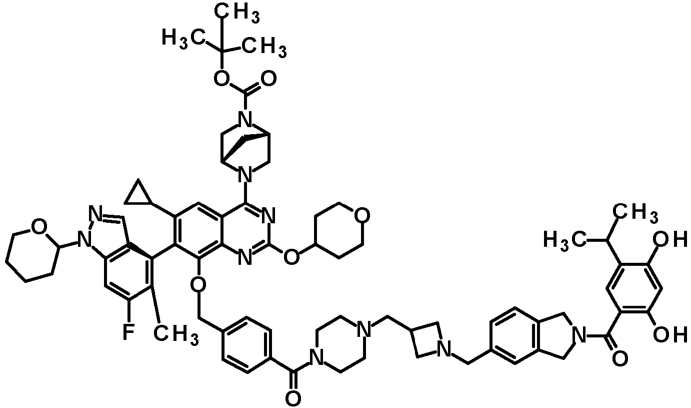 |
| 63 | 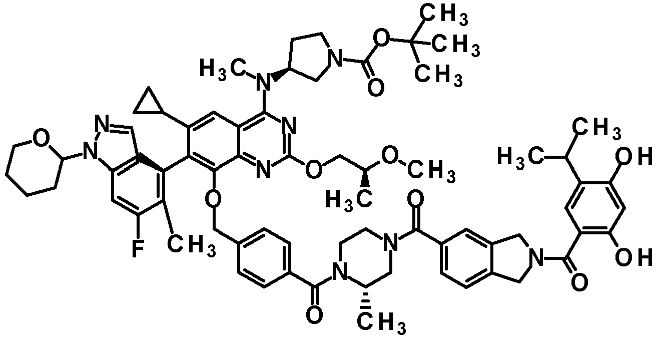 |

**[Table 3-20]**

| PEx | Str |
|---|---|
| 64 | 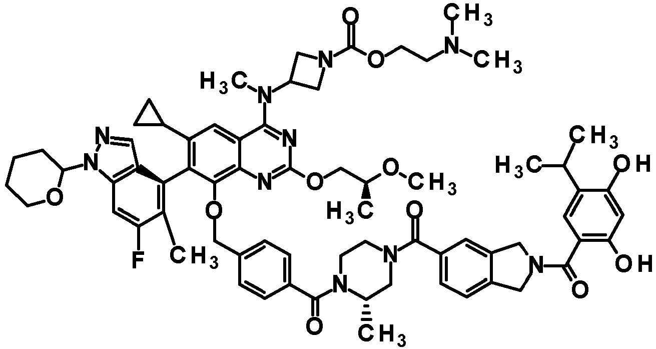 |
| 65 | 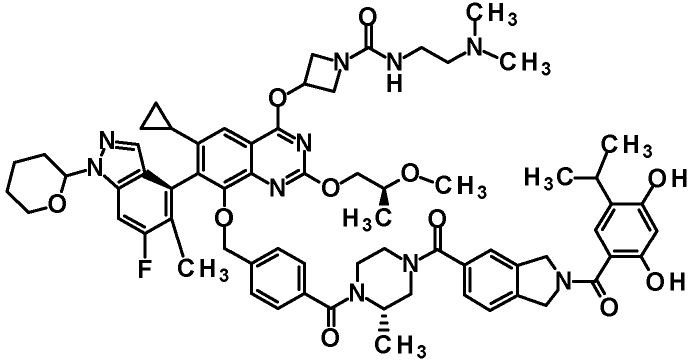 |
| 66 | 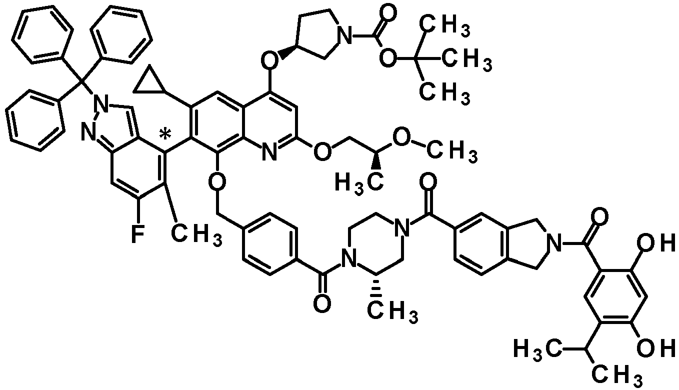 |

**[Table 3-21]**

| PEx | Str |
|---|---|
| 67 | 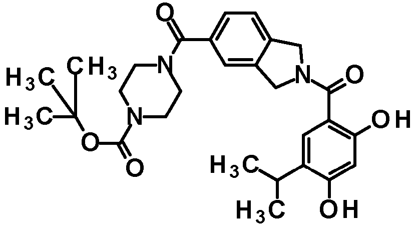 |
| 68 | 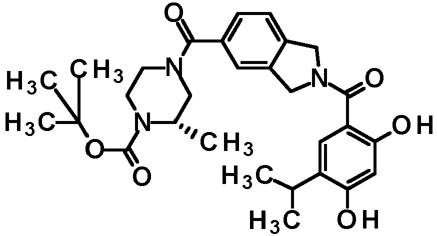 |
| 69 | 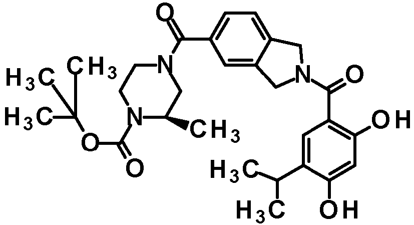 |
| 70 | 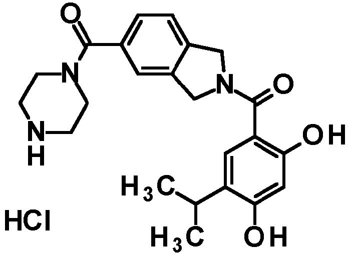 |
| 71 | 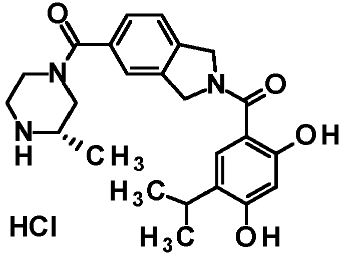 |

**[Table 3-22]**

| PEx | Str |
|---|---|
| 72 | 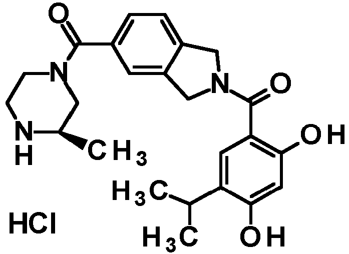 |
| 73 | 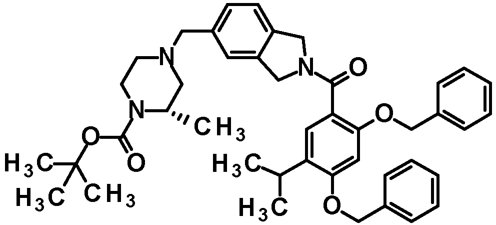 |
| 74 | 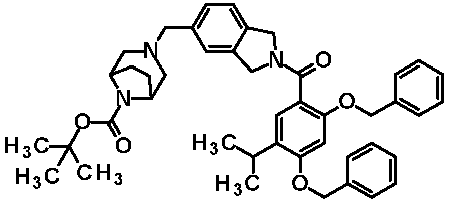 |
| 75 | 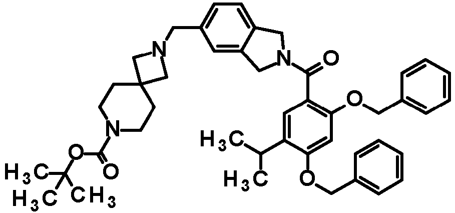 |
| 76 | 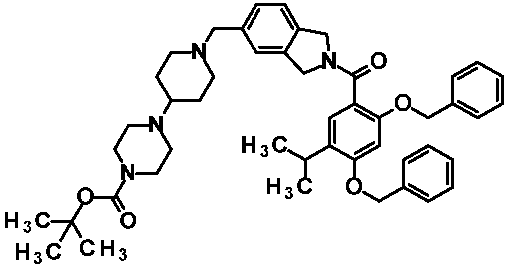 |

**[Table 3-23]**

| PEx | Str |
|---|---|
| 77 | 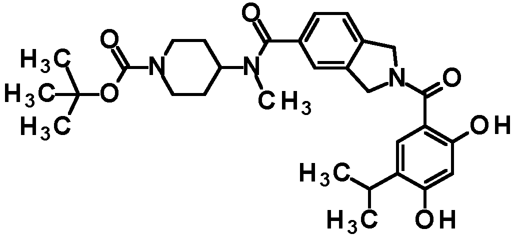 |
| 78 | 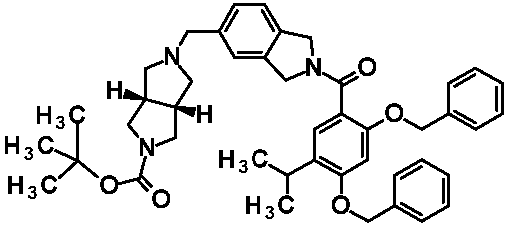 |
| 79 | 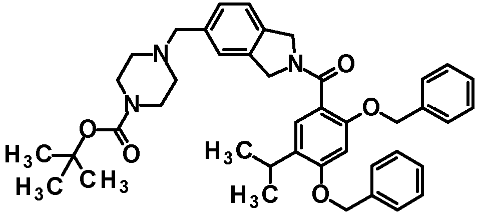 |
| 80 | 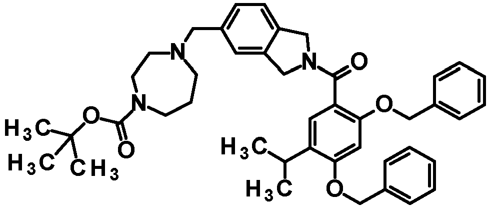 |
| 81 | 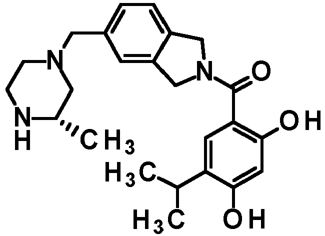 |

**[Table 3-24]**

| PEx | Str | PEx | Str |
|---|---|---|---|
| 82 | 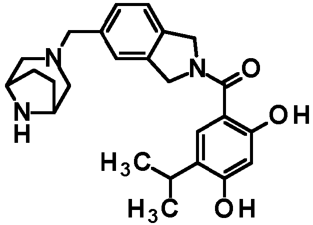 | 86 | 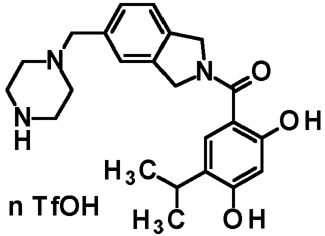 |
| 83 | 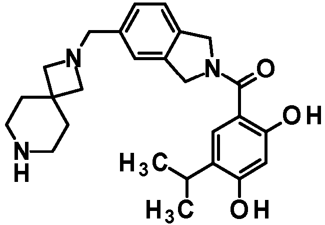 | 87 | 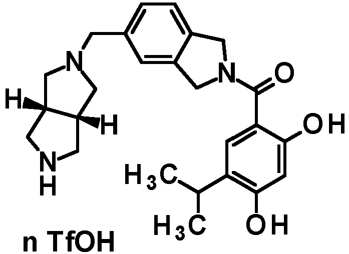 |
| 84 | 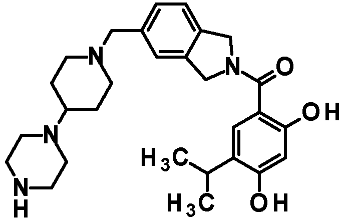 | 88 | 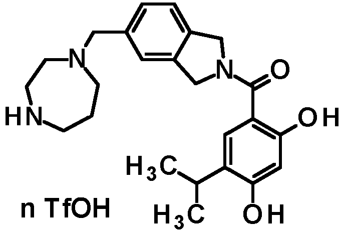 |
| 85 | 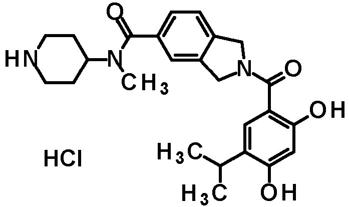 | | |

**[Table 3-25]**

| PEx | Str |
|---|---|
| 89 | 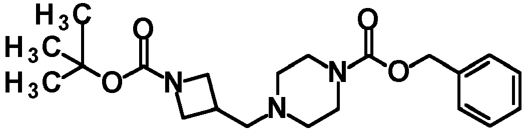 |
| 90 | 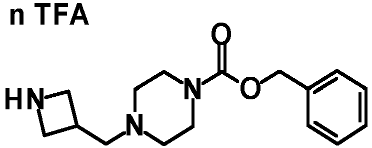 |
| 91 | 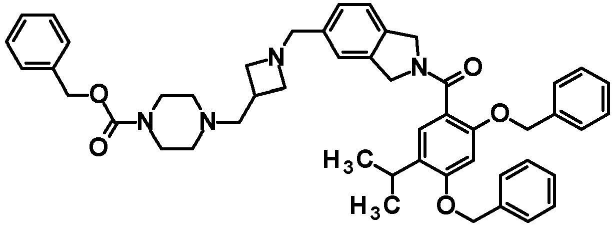 |
| 92 | 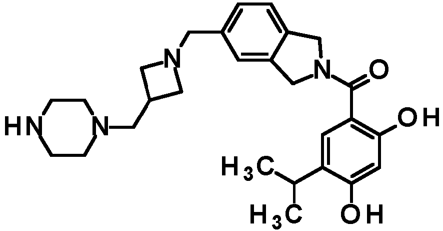 |

**[Table 3-26]**

| PEx | Str |
|---|---|
| 93 | 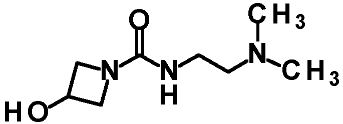 |
| 94 | 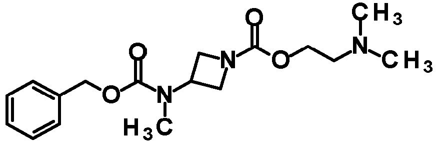 |
| 95 | 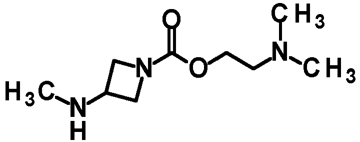 |

**[Table 3-27]**

| PEx | Str |
|---|---|
| 96 | 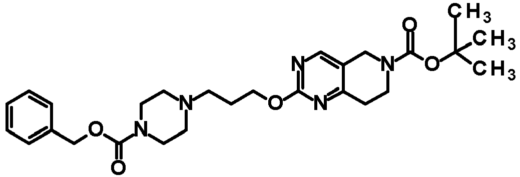 |
| 97 | 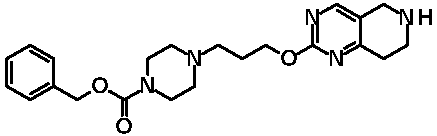 |
| 98 | 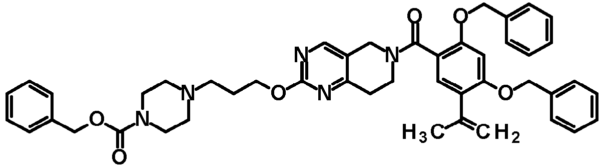 |
| 99 | 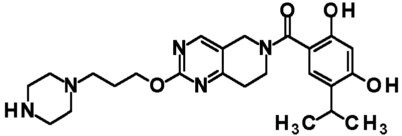 |
| 100 | 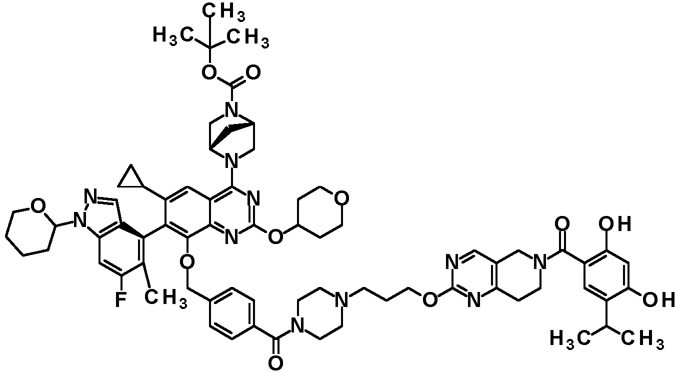 |

**[Table 4-1]**

| PEx | PSyn | DAT |
|---|---|---|
| 1 | 1 | ESI+: 583.1, 585.1 |
| 2 | 2 | ESI+: 611.1 |
| 3 | 3 | ESI+: 713.1 |
| 4 | 4 | ESI+: 627.5 |
| 5 | 5 | ESI+: 937.6 |
| 6 | 6 | ESI+:695.4 |
| | | NMR: 0.52-0.66 (3H, m), 0.66-0.75 (1H, m), 0.86 (3H, br d), 1,27 (3H, t), 1.30-1.46 (10H, m), 1.83 (3H, br s), 1.90-2.01 (1H, m), 2.04 (1H, br d), 2.98-3.10 (2H, m), 3.45-3.55 (2H, m), 3.67-3.82 (1H, m), 4.33 (1H, br d), 4.52-4.64 (1H, m), 5.15 (1H, br d), 6.02-6.10 (1H, m), 6.82 (2H, br d), 7.12-7.20 (3H, m), 7.31 (1H, br s), 7.37 (1H, d), 7.51-7.57 (1H, m), 13.04 (1H, br s) |
| 7 | 7 | ESI+: 779.5 |
| 8 | 8 | ESI+: 811.5 |
| 9 | 9 | ESI+: 707.4 |
| 10 | 10 | ESI+: 855.5 |
| 11 | 11 | ESI+: 863.6 |
| 12 | 12 | ESI+: 849.7 |
| 13 | 13 | ESI+: 402.9, 404.9, 406.9 |
| 14 | 14 | ESI+: 480.9, 482.8 [M+Na]+ |
| 15 | 15 | ESI+: 508.9 [M+Na]+ |
| 16 | 16 | ESI+: 587.2 |
| 17 | 17 | ESI+: 503.4 |
| 18 | 18 | ESI+: 813.4 |
| 19 | 19 | ESI+: 867.7 [M+Na]+ |
| | | NMR: 0.43-0.50 (1H, m), 0.66-0.75 (1H, m), 0.77-0.89 (2H, m), 0.94 (3H, d), 1.25 (3H, t), 1.46-1.54 (1H, m), 1.75 (9H, s), 1.80 (3H, d), 3.47-3.54 (2H, m), 5.94 (1H, q), 6.90-6.94 (2H, m), 7.01-7.09 (6H, m), 7.16-7.24 (3H, m), 7.28-7.35 (9H, m), 7.35 (1H, s), 7.44 (1H, d), 7.52 (1H, d) |
| 20 | 11 | ESI+: 841.6 |

**[Table 4-2]**

| PEx | PSyn | DAT |
|---|---|---|
| 21 | 39 | ESI+: 737.6 |
| 22 | 10 | ESI+: 907.6 [M+Na]+ |
| 23 | 23 | ESI+: 671.5 |
| 24 | 24 | ESI+: 853.7 |
| 25 | 24 | ESI+: 854.7 |
| 26 | 24 | ESI+: 840.7 |
| 27 | 12 | ESI+: 839.6 |
| 28 | 12 | ESI+: 840.7 |
| 29 | 29 | ESI+: 826.7 |
| 30 | 30 | ESI+: 417.9 |
| 31 | 31 | ESI+: 386.0 |
| 32 | 32 | ESI+: 419.8, 421.9, 423.9 |
| 33 | 33 | ESI+: 445.9, 447.9 |
| 34 | 34 | ESI+: 548.0, 550.0 |
| 35 | 35 | ESI+: 699.2, 701.2 |
| 36 | 36 | ESI+: 613.2 |
| 37 | 37 | ESI+: 925.3 |
| | | NMR: 0.29-0.37 (1H, m), 0.54-0.64 (1H, m), 0.64-0.77 (2H, m), 0.78-0.87 (3H, m), 1.22-1.46 (13H, m), 1.83 (3H, br s), 2.16-2.28 (2H, m), 3.07-3.26 (2H, m), 3.41-3,50 (2H, m), 3.55-3.62 (2H, m), 5.28 (1H, br s), 6.21-6.29 (1H, m), 6.82-6.89 (2H, m), 6.91 (1H, s), 7.00-7.08 (6H, m), 7.16-7.24 (4H, m), 7.25-7.33 (9H, m), 7.33-7.37 (1H, m), 7.48 (1H, d) |
| 38 | 38 | ESI+: 979.5 [M+Na]+ |
| 39 | 39 | ESI+: 875.7 [M+Na]+ |
| 40 | 40 | ESI+: 1001.4 |
| 41 | 41 | ESI+: 997.7 |
| 42 | 42 | ESI+: 983.6 |
| 43 | 43 | NMR: 7.08-7.15 (6H, m), 7.36-7.44 (10H, m), 7.49-7.54 (1H, m), 7.89 (1H, d) |
| 44 | 44 | NMR: 2.34 (3H, d), 7.07-7.13 (6H, m), 7.36-7.43 (9H, m), 7.51 (1H, d), 7.79 (1H, d) |
| 45 | 45 | NMR: 1.21 (12H, s), 2.44 (3H, d), 7.04-7.11 (6H, m), 7.34-7.44 (9H, m), 7.49 (1H, d), 8.09 (1H, d) |

**[Table 4-3]**

| PEx | PSyn | DAT |
|---|---|---|
| 46 | 11 | ESI+: 819.7 |
| 47 | 9 | ESI+: 715.7 |
| 48 | 48 | ESI+: 833.8 |
| 49 | 49 | ESI+: 1226.7 |
| 50 | 50 | ESI+: 1240.8 |
| 51 | 50 | ESI+: 1254.8 |
| 52 | 50 | ESI+: 1254.7 |
| 53 | 50 | ESI+: 1252.6 |
| 54 | 50 | ESI+: 1240.8 |
| 55 | 50 | ESI+: 1226.9 |
| 56 | 50 | ESI-: 1238.9 |
| 57 | 50 | ESI-: 1238.5 |
| 58 | 50 | ESI+: 1252.9 |
| 59 | 50 | ESI+: 1266.8 |
| 60 | 50 | ESI+: 1309.9 |
| 61 | 50 | ESI+: 1269.0 |
| 62 | 50 | ESI+: 1296.0 |
| 63 | 50 | ESI+: 1244.9 |
| 64 | 50 | ESI-: 1243.8 |
| 65 | 50 | ESI+: 1231.9 |
| 66 | 50 | ESI+: 1388.9 |
| 67 | 67 | ESI+: 510.6 |
| 68 | 67 | ESI+: 524.5 |
| 69 | 67 | ESI+: 524.6 |
| 70 | 70 | ESI+: 410.3 |
| 71 | 70 | ESI+: 424.5 |
| 72 | 70 | ESI+: 424.5 |
| 73 | 73 | ESI+: 690.7 |
| 74 | 73 | ESI+: 702.6 |
| 75 | 73 | ESI+: 716.7 |
| 76 | 73 | ESI+: 760.0 |
| 77 | 67 | ESI+: 538.7 |
| 78 | 73 | ESI+: 702.4 |
| 79 | 73 | ESI+: 676.3 |
| 80 | 73 | ESI+: 690.3 |

**[Table 4-4]**

| PEx | PSyn | DAT |
|---|---|---|
| 81 | 81 | ESI+: 410.4 |
| 82 | 81 | ESI+: 422.4 |
| 83 | 81 | ESI+: 436.4 |
| 84 | 81 | ESI+: 479.5 |
| 85 | 70 | ESI+: 438.4 |
| 86 | 86 | ESI+: 396.2 |
| 87 | 86 | ESI+: 422.2 |
| 88 | 86 | ESI+: 410.2 |
| 89 | 73 | ESI+: 390.5 |
| 90 | 90 | ESI+: 290.4 |
| 91 | 73 | ESI+: 779.8 |
| 92 | 92 | ESI+: 465.4 |
| 93 | 93 | ESI+: 188.1 |
| 94 | 94 | ESI+: 336.2 |
| 95 | 95 | ESI+: 202.1 |
| 96 | 96 | ESI+: 512.3 |
| 97 | 90 | ESI+: 412.3 |
| 98 | 50 | ESI+: 768.3 |
| 99 | 92 | ESI+: 456.4 |
| 100 | 50 | ESI+: 1286.7 |

**[Table 5-1]**

| Ex | Str |
|---|---|
| 1 | 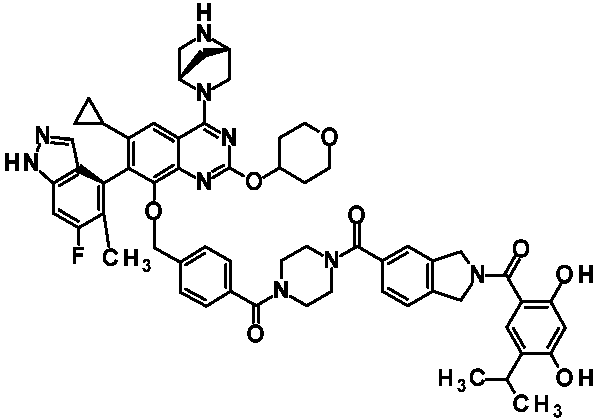 |
| 2 | 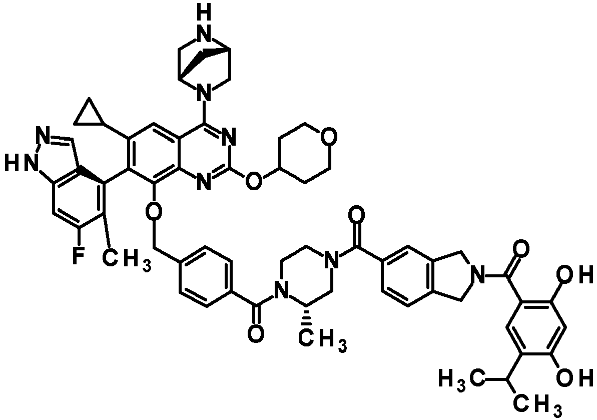 |
| 3 | 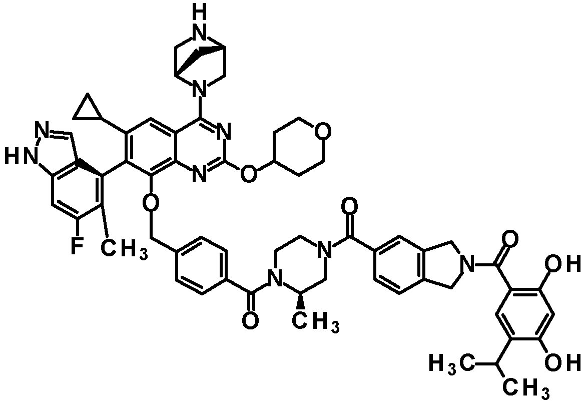 |

**[Table 5-2]**

| Ex | Str |
|---|---|
| 4 | 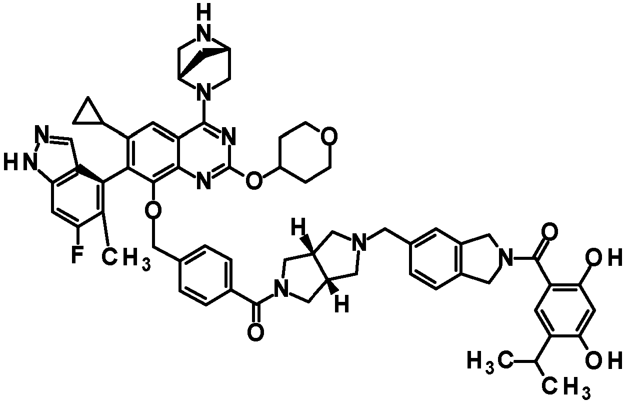 |
| 5 | 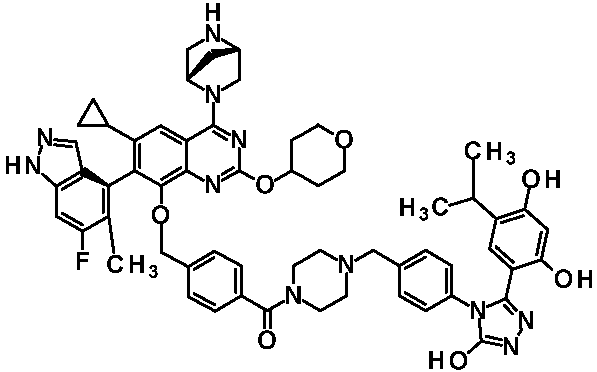 |
| 6 | 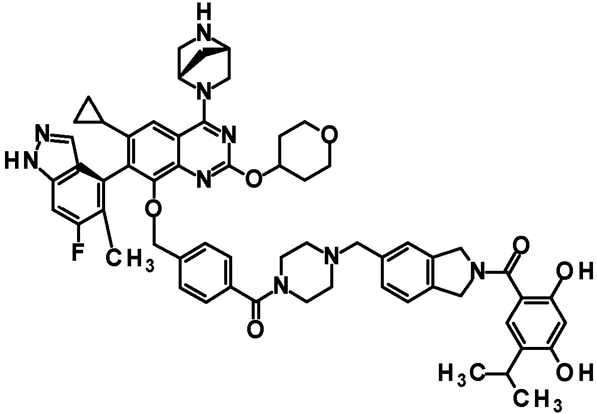 |

**[Table 5-3]**

| Ex | Str |
|---|---|
| 7 | 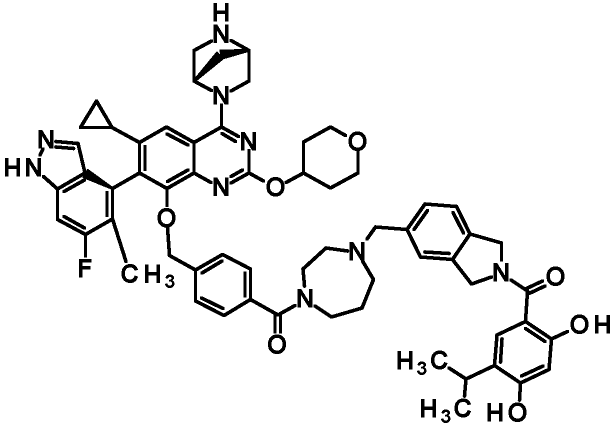 |
| 8 | 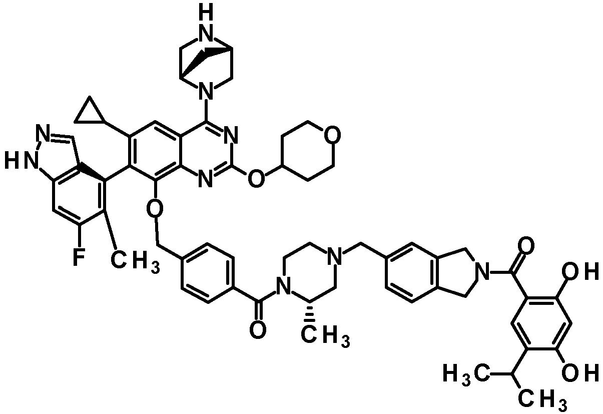 |
| 9 | 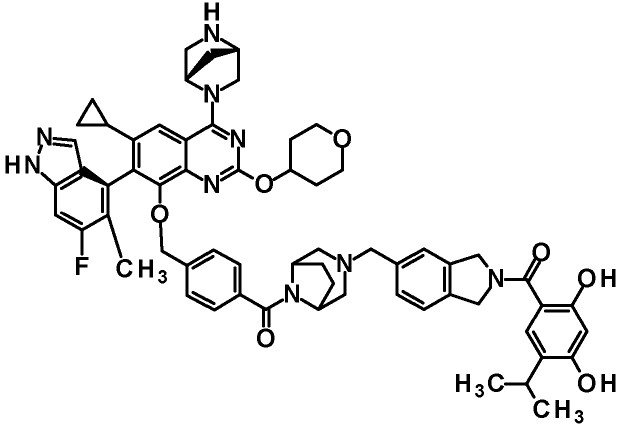 |

**[Table 5-4]**

| Ex | Str |
|---|---|
| 10 | 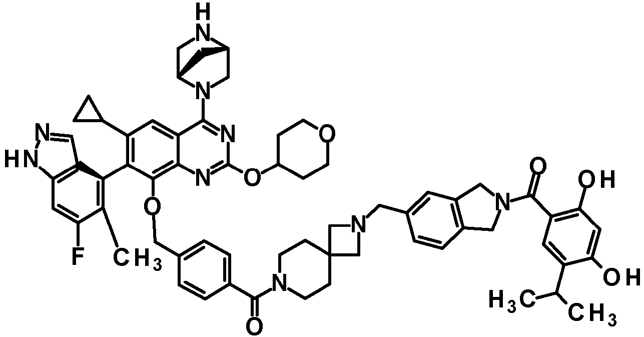 |
| 11 | 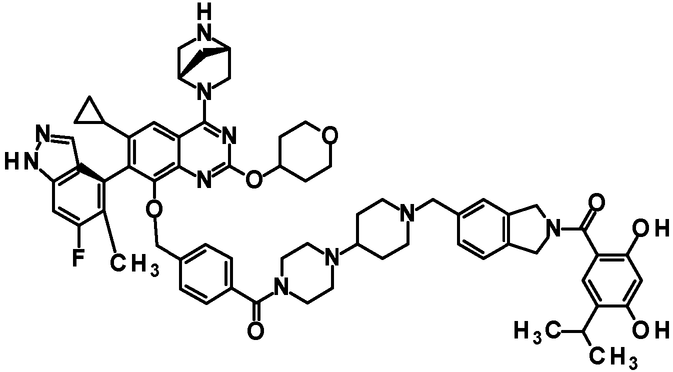 |
| 12 | 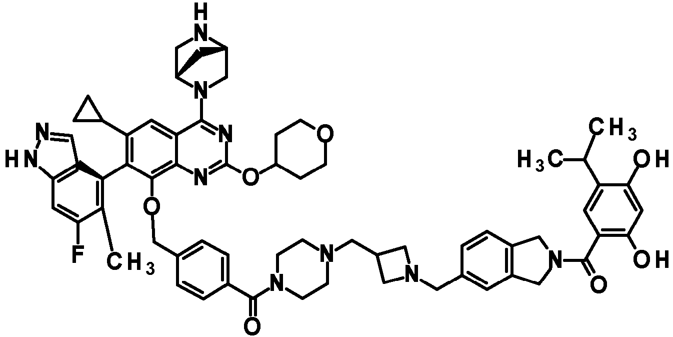 |

**[Table 5-5]**

| Ex | Str |
|---|---|
| 13 | 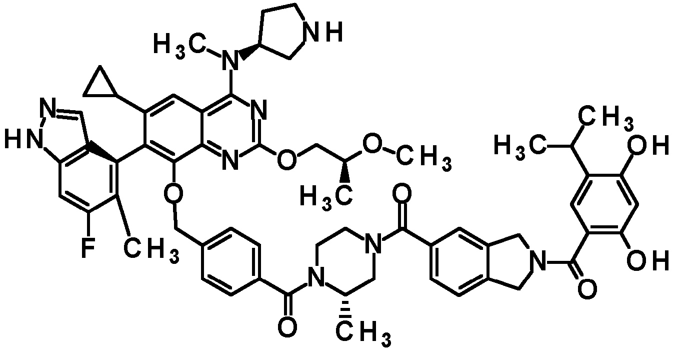 |
| 14 | 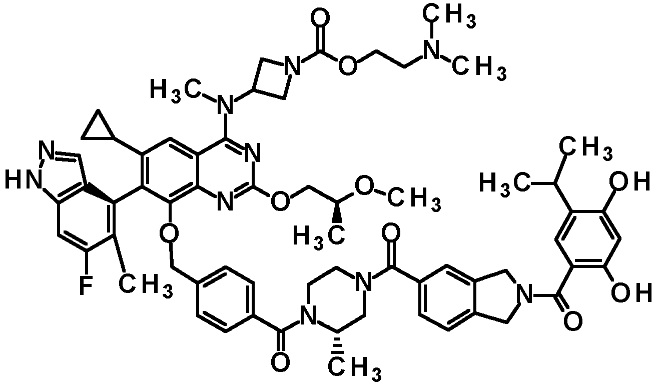 |
| 15 | 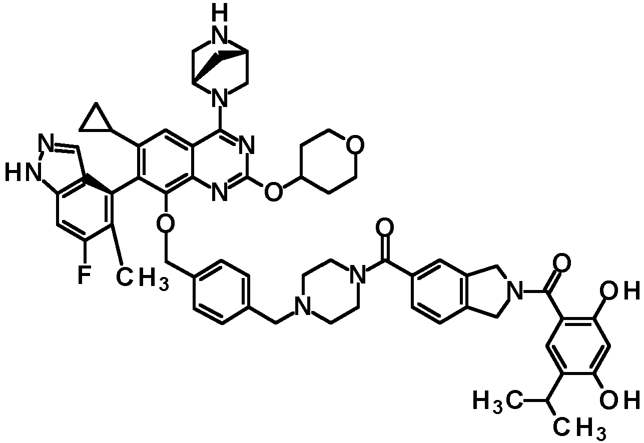 |

**[Table 5-6]**

| Ex | Str |
|---|---|
| 16 | 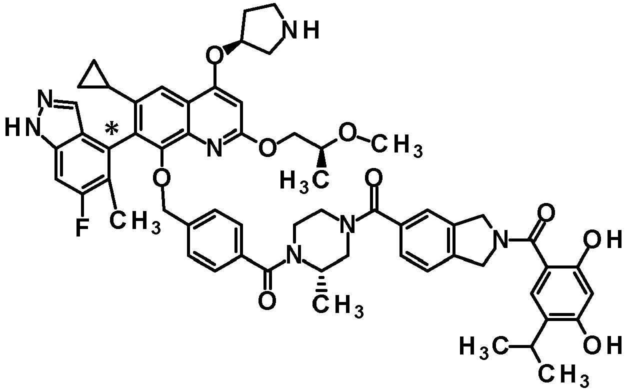 |
| 17 | 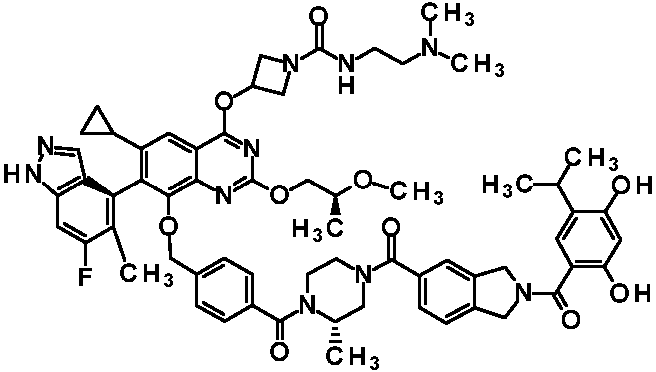 |
| 18 | 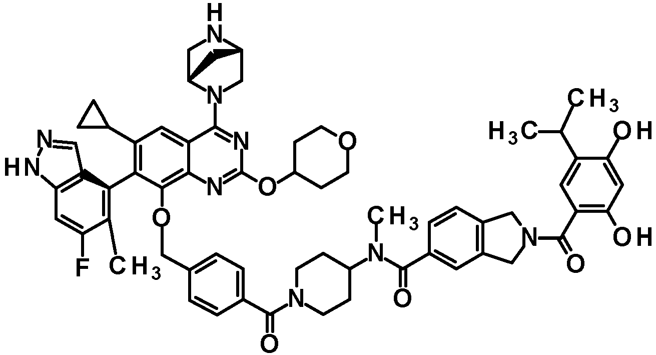 |

**[Table 5-7]**

| Ex | Str |
|---|---|
| 19 | 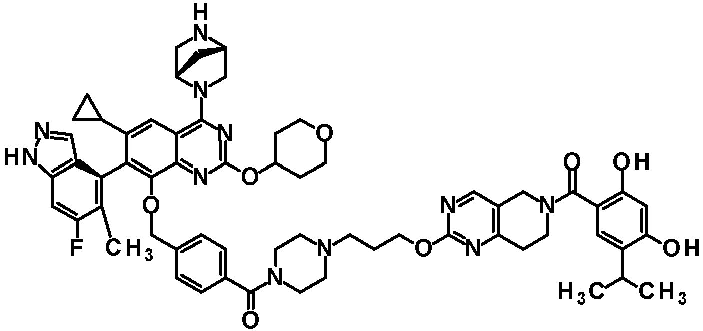 |

**[Table 6]**

| Ex | Syn | DAT |
|---|---|---|
| 1 | 1 | ESI+: 1056.7 |
| 2 | 1 | ESI+: 1070.7 |
| 3 | 1 | ESI+: 1070.6 |
| 4 | 1 | ESI+: 1068.8 |
| 5 | 5 | ESI+: 1056.9 |
| 6 | 1 | ESI+: 1042.9 |
| 7 | 1 | ESI+: 1056.7 |
| 8 | 1 | ESI+: 1056.8 |
| 9 | 1 | ESI+: 1069.0 |
| 10 | 1 | ESI+: 1082.9 |
| 11 | 1 | ESI+: 1125.7 |
| 12 | 1 | ESI+: 1111.7 |
| 13 | 1 | ESI+: 1060.9 |
| 14 | 1 | ESI+: 1161.8 |
| 15 | 1 | ESI+: 1042.8 |
| 16 | 1 | ESI+: 1046.8 |
| 17 | 1 | ESI+: 1147.7 |
| 18 | 1 | ESI+: 1084.9 |
| 19 | 5 | ESI+: 1102.6 |

As examples of specific compounds of the formula (I) included in the present invention, compounds having any of the following structures are shown. These compounds were produced also by the typical production methods shown above, the production methods of the Production Examples and the Examples, a combination of the production methods or a method that was obvious to a person skilled in the art.

Furthermore, G12D mutant KRAS protein degradation activity and cell growth inhibitory effect on a human G12D mutant KRAS-positive pancreatic cancer line were observed for these compounds by the test methods of the Test Examples described above. Therefore, the compounds can be used as an active ingredient of a pharmaceutical composition, for example, a pharmaceutical composition for treating pancreatic cancer.

**[Table 7]**

| Str |
|---|
| 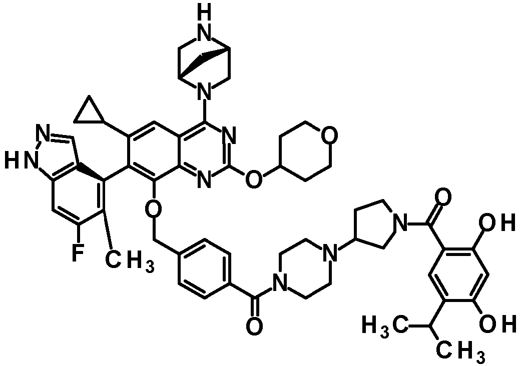 |
| 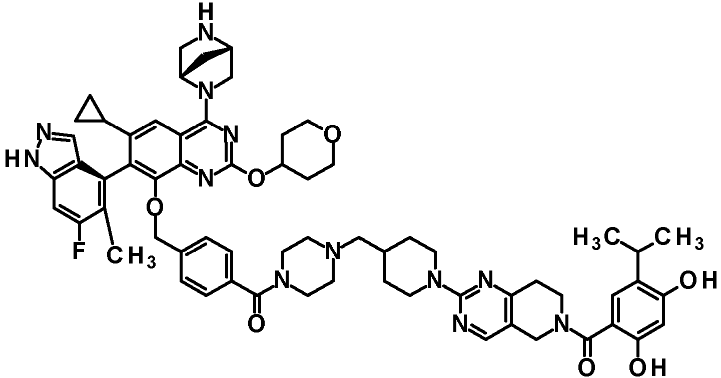 |

### Industrial Applicability

The compound or a salt thereof of the present invention is excellent in the degradation-inducing action on a KRAS protein, and/or is useful as a KRAS inhibitor and can be used as an active ingredient of a pharmaceutical composition for treating cancer, in particular, a pharmaceutical composition for treating pancreatic cancer.

## Claims

1. A compound of the formula (I) or a salt thereof, 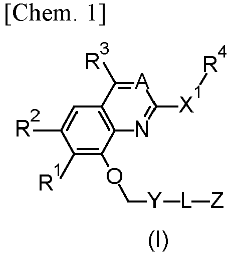 (wherein in the formula,
A is CR^{A} or N,
R^{A} is H or C₁₋₃ alkyl,
R¹ is naphthyl optionally substituted with OH, or R¹ is a group selected from the group consisting of the formula (IIa) and the formula (IIb) below, 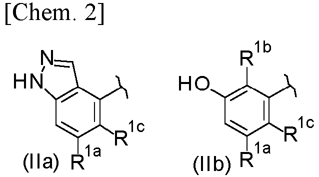
R^{1a} and R^{1b}, which are the same as or different from each other, are H, methyl, F or Cl,
R^{1c} is F, Cl, methyl or ethyl,
R² is H, halogen, optionally substituted C₁₋₃ alkyl, cyclopropyl or vinyl,
R³ is a group selected from the group consisting of the formula (III), the formula (IV), the formula (V), the formula (VI), the formula (VII), the formula (VIII), the formula (IX), the formula (X), the formula (XI), the formula (XII), the formula (XIII), the formula (XIV) and the formula (XV) below, or R³ is a saturated or unsaturated 7-membered or 8-membered bridged heterocyclic group containing one or two nitrogen atoms optionally substituted with OH, wherein when the bridged heterocyclic group is substituted with OH, OH is attached only to a carbon atom that is an atom forming a bridged heterocyclic ring, 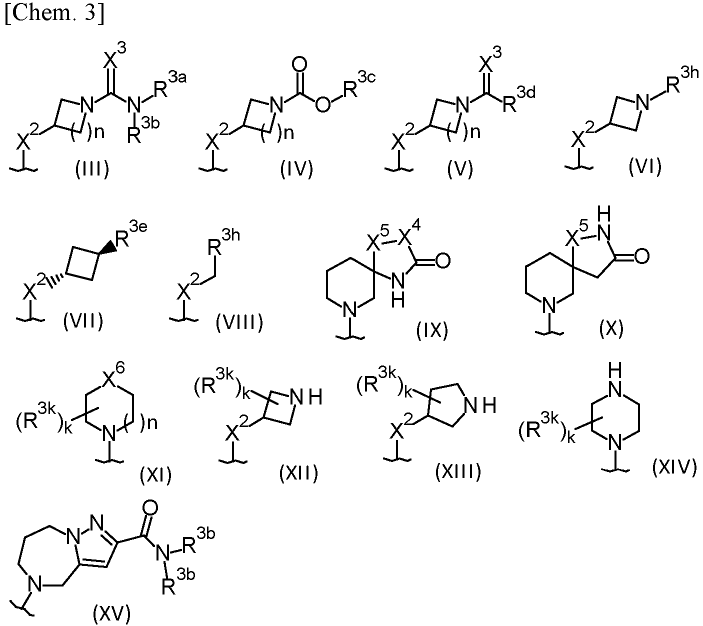
R^{3a} is -(CH₂)ₚCHR^{3f}-NR^{N1}R^{N2}; -(CH₂)ₚCHR^{3f}-OR^{3g}; a 5-membered or 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2},or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or C₁₋₃ alkyl,
R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2},or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3e} is -O-C₂₋₃ alkylene-NR^{N1}R^{N2} or -NR^{N1}R^{N2},
R^{3f} is H, F or C₁₋₃ alkyl,
R³⁹ is H or C₁₋₃ alkyl,
R^{3h} is optionally substituted 5-membered heteroaryl containing one to four hetero atoms selected from the group consisting of oxygen, sulfur and nitrogen, optionally substituted 6-membered heteroaryl containing one to three nitrogen atoms, or cyclobutyl optionally substituted with -NR^{N1}R^{N2},
R^{N1} and R^{N2}, which are the same as or different from each other, are H or C₁₋₃ alkyl, or
R^{N1} and R^{N2}, together with the nitrogen atom to which they are attached, optionally form an optionally substituted 4-membered to 6-membered saturated heterocyclic group containing one or two hetero atoms selected from the group consisting of oxygen, sulfur and nitrogen, or
R^{3f} and R^{N1}, together with the carbon atom and the nitrogen atom to which they are attached, optionally form an optionally substituted 4-membered to 6-membered saturated heterocyclic group containing one or two hetero atoms selected from the group consisting of oxygen, sulfur and nitrogen,
each R^{3k}, which is the same as or different from each other, is a group selected from the group consisting of OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, where R^{3k} is attached only to a carbon atom that is an atom forming a ring represented by the formula (XI), the formula (XII), the formula (XIII) or the formula (XIV),
X² is -O-, -NH- or -N(C₁₋₃ alkyl)-,
X³ is O or S,
X⁴ is -CH₂- or -NH-,
X⁵ is a bond, -CH₂- or C=O,
X⁶ is -CH₂- or -O-,
n is 1 or 2,
p is 1 or 2,
k is an integer of 0 to 2,
R⁴ is optionally substituted C₁₋₆ alkyl, an optionally substituted 4-membered to 6-membered saturated heterocyclic group containing one or two hetero atoms selected from oxygen, sulfur and nitrogen, optionally substituted 5-membered heteroaryl containing one to four hetero atoms selected from oxygen, sulfur and nitrogen or optionally substituted 6-membered heteroaryl containing one to three nitrogen atoms,
X¹ is a bond, -CH₂-, -O-, -S- or -NR^{4X}-,
R^{4X} is H or C₁₋₃ alkyl,
Y is phenylene optionally substituted with a group selected from the group consisting of F and Cl or pyridinediyl,
L is -(L¹-L²-L³-L⁴-L⁵-L⁶-L⁷)-,
L¹, L², L³, L⁴ , L⁵, L⁶ and L⁷, which are the same as or different from each other, are groups selected from the group consisting of a bond, -O-, -S-, -NR^{L1}-, acetylene-1,2-diyl, optionally substituted azetidinediyl, optionally substituted pyrrolidinediyl, optionally substituted piperidinediyl, optionally substituted piperazinediyl, optionally substituted diazepanediyl, optionally substituted C₁₋₃ alkylene, a saturated or unsaturated 7-membered or 8-membered bridged heterocyclic divalent group containing one or two nitrogen atoms, a saturated 7-membered to 11-membered spiroheterocyclic divalent group containing two nitrogen atoms, a saturated 8-membered to 10-membered bicycloheterocyclic divalent group containing two nitrogen atoms, optionally substituted phenylene, optionally substituted pyridinediyl, C=O, S=O and S(=O)₂,
R^{L1} is H or C₁₋₃ alkyl,
Z is a group selected from the group consisting of the formula (XVI), the formula (XVII), the formula (XVIII), the formula (XIX), the formula (XX), the formula (XXI), the formula (XXII), the formula (XXIII), the formula (XXIV), the formula (XXV), the formula (XXVI), the formula (XXVII), the formula (XXVIII), the formula (XXIX), the formula (XXX), the formula (XXXI), the formula (XXXII), the formula (XXXIII), the formula (XXXIV) and the formula (XXXV) below, 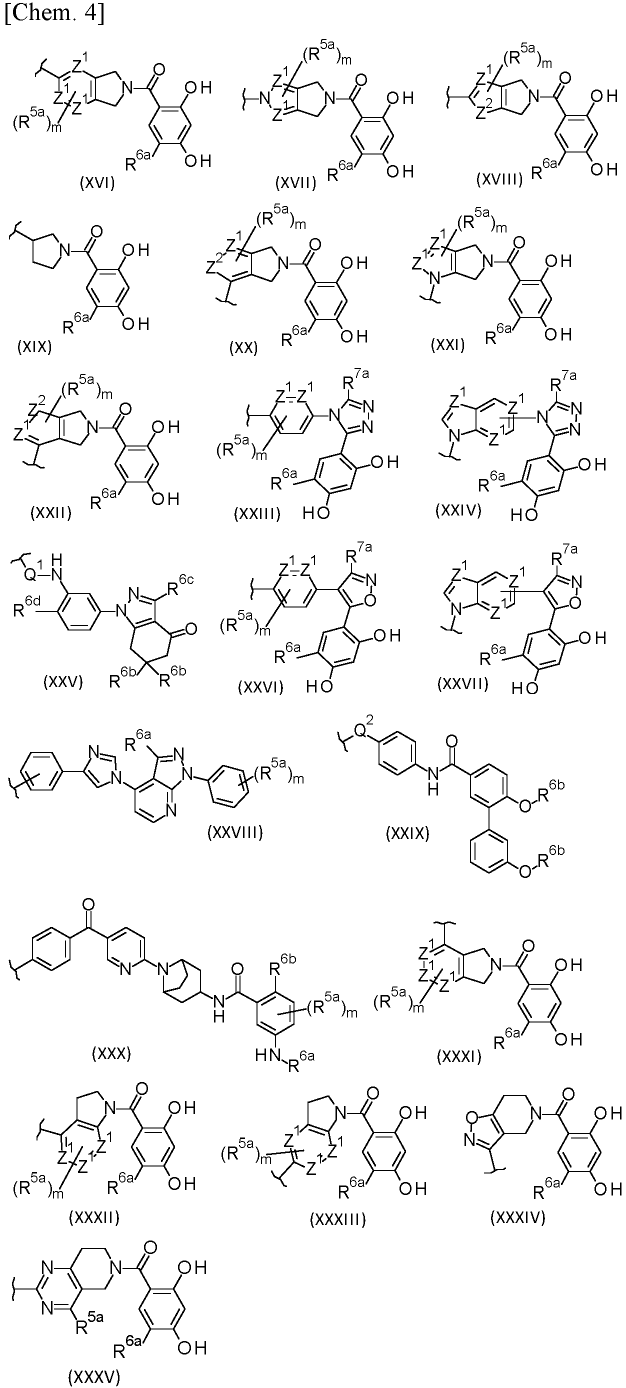
each Z¹, which is the same as or different from each other, is CH or N,
Z² is NH, NCH₃, O or S,
each R^{5a}, which is the same as or different from each other, is H, halogen, C₁₋₃ alkyl or -(C=O)NH₂,
R^{6a} is C₁₋₆ alkyl optionally substituted with F,
each R^{6b}, which is the same as or different from each other, is C₁₋₃ alkyl,
R^{6c} is C₁₋₃ alkyl optionally substituted with F,
R^{6d} is -(C=O)NR^{N3}R^{N4},
R^{N3} and R^{N4}, which are the same as or different from each other, are H or C₁₋₃ alkyl,
R^{7a} is OH or -(C=O)NR^{N5}R^{N6},
R^{N5} and R^{N6}, which are the same as or different from each other, are H, cyclopropyl or C₁₋₃ alkyl optionally substituted with F, and
m is an integer of 0 to 2).

2. The compound or a salt thereof according to claim 1,
wherein A is CR^{A} or N,
R^{A} is H,
R¹ is the formula (IIa) below, 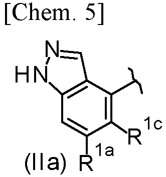
R^{1a} is F,
R^{1c} is methyl,
R² is cyclopropyl,
R³ is a group selected from the group consisting of the formula (III-a), the formula (IV-a), the formula (V-a), the formula (XIII-a) and the formula (XXXVI) below, 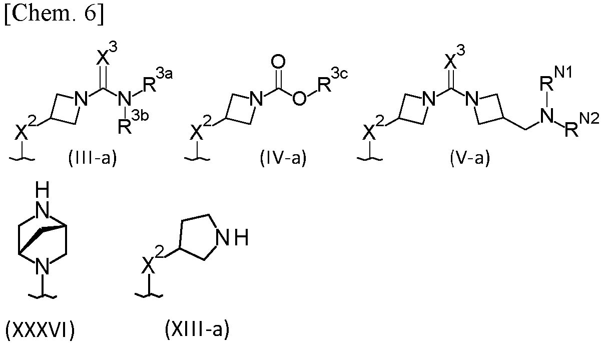
R^{3a} is -(CH₂)ₚCHR^{3f}-NR^{N1}R^{N2};
R^{3b} is H,
R^{3c} is -(CH₂)ₚCHR^{3f}-NR^{N1}R^{N2} or C₃₋₆ cycloalkyl optionally substituted with - NR^{N1}R^{N2}
R^{3f} is H,
R^{N1} and R^{N2} are both C₁₋₃ alkyl,
X² is -O-, -NH- or -N(C₁₋₃ alkyl)-,
X³ is O,
p is 1,
R⁴ is C₁₋₆ alkyl optionally substituted with OCH₃ or tetrahydropyranyl,
X¹ is -O-,
Y is phenylene optionally substituted with F,
L is a group selected from the group consisting of the formula (XXXX-a), the formula (XXXX-b), the formula (XXXXI), the formula (XXXXII), the formula (XXXXIII), the formula (XXXXIV), the formula (XXXXV), the formula (XXXXVI), the formula (XXXXVII) and the formula (XXXXVIII), 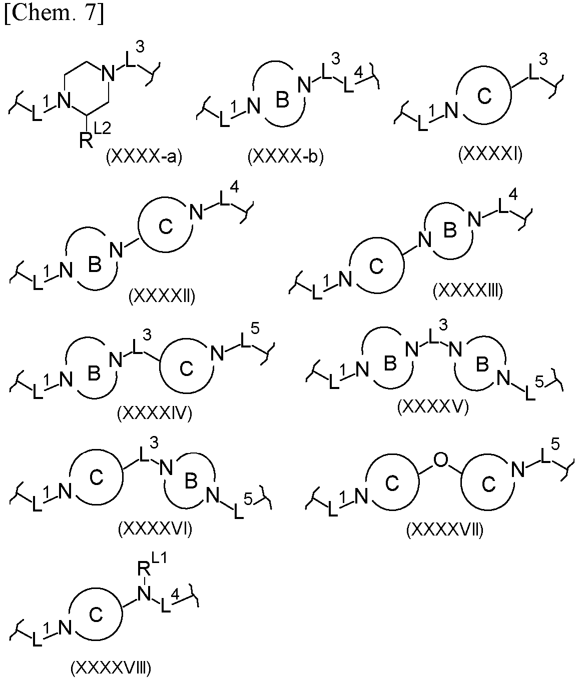
L¹ is -(CH₂)-, C=O or S(=O)₂,
L³, L⁴ and L⁵, which are the same as or different from each other, are a bond, -O-, C₁₋₃ alkylene or C=O,
R^{L1} is H or C₁₋₃ alkyl,
R^{L2} is C₁₋₃ alkyl or oxo,
ring B is piperazine, diazepane, diazabicyclo[2.2.1]heptane, diazabicyclo[3.2.1]octane, diazaspiro[3.3]heptane, diazaspiro[3.4]octane, diazaspiro[3.5]nonane, diazaspiro[4.5]decane, diazaspiro[5.5]undecane or diazabicyclo[3.3.0]octane,
ring C is azetidine, pyrrolidine or piperidine,
Z is a group selected from the group consisting of the formula (XVI-a), the formula (XXIII-a), the formula (XIX) and the formula (XXXV) below, 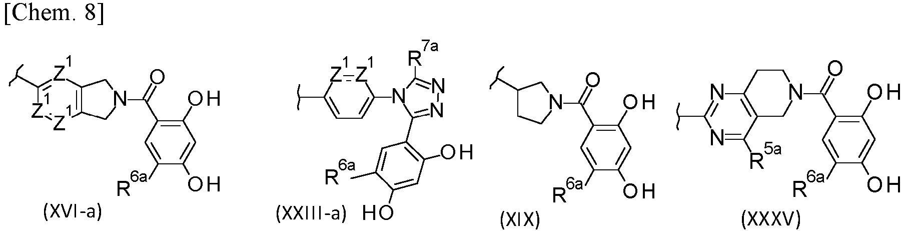
each Z¹ is CH,
R^{5a} is H,
R^{6a} is C₁₋₃ alkyl,
R^{7a} is OH or -(C=O)NR^{N5}R^{N6},
R^{N5} is H, and
R^{N6} is C₁₋₃ alkyl.

3. The compound or a salt thereof according to claim 2, wherein R³ is a group selected from the group consisting of the formula (III-a), the formula (IV-a), the formula (XXXVI) and the formula (XIII-a) below, 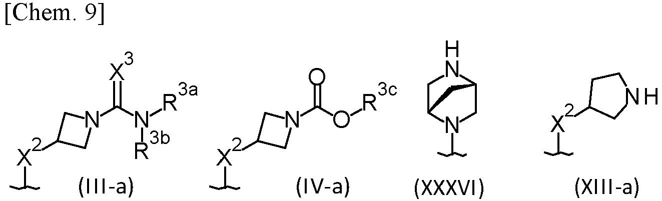
R^{3a} is -(CH₂)ₚCHR^{3f}-NR^{N1}R^{N2};
R^{3b} is H,
R^{3c} is -(CH₂)ₚCHR^{3f}-NR^{N1}R^{N2};
R^{3f} is H,
R^{N1} and R^{N2} are both C₁₋₃ alkyl,
X² is -O- or -N(C₁₋₃ alkyl)-,
X³ is O,
p is 1,
R⁴ is C₁₋₃ alkyl optionally substituted with OCH₃ or tetrahydropyranyl,
Y is phenylene,
L is a group selected from the group consisting of the formula (XXXX-a), the formula (XXXX-b), the formula (XXXXII), the formula (XXXXIV) and the formula (XXXXVIII) below, 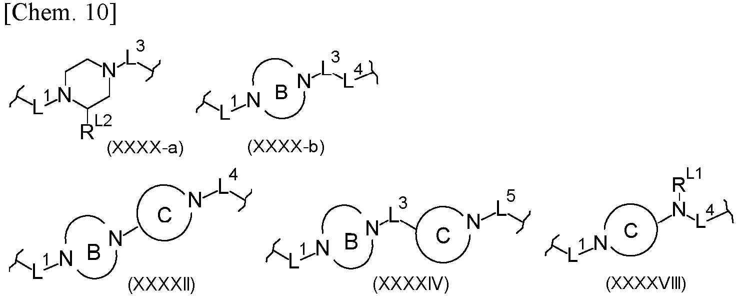
L¹ is -(CH₂)- or C=O,
L³, L⁴ and L⁵, which are the same as or different from each other, are a bond, -O-, C₁₋₃ alkylene or C=O,
R^{L1} and R^{L2} are C₁₋₃ alkyl,
ring B is piperazine, diazepane, diazabicyclo[3.2.1]octane, diazaspiro[3.5]nonane or diazabicyclo[3.3.0]octane,
ring C is azetidine or piperidine,
Z is a group selected from the group consisting of the formula (XVI-a), the formula (XXIII-a), the formula (XIX) and the formula (XXXV) below, 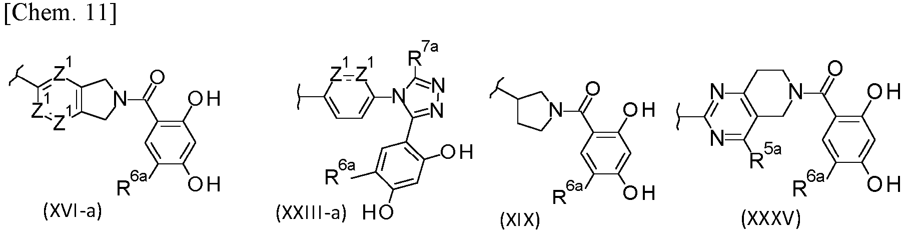
each Z¹ is CH,
R^{5a} is H,
R^{6a} is isopropyl, and
R^{7a} is OH.

4. The compound or a salt thereof according to claim 1, wherein R³ is a group selected from the group consisting of the formula (III), the formula (IV), the formula (V), the formula (VI), the formula (VII), the formula (VIII), the formula (IX), the formula (X), the formula (XI), the formula (XII), the formula (XIII), the formula (XIV) and the formula (XV) below, or R³ is a saturated or unsaturated 7-membered or 8-membered bridged heterocyclic group containing one or two nitrogen atoms optionally substituted with OH, wherein when the bridged heterocyclic group is substituted with OH, OH is attached only to a carbon atom that is an atom forming a bridged heterocyclic ring, 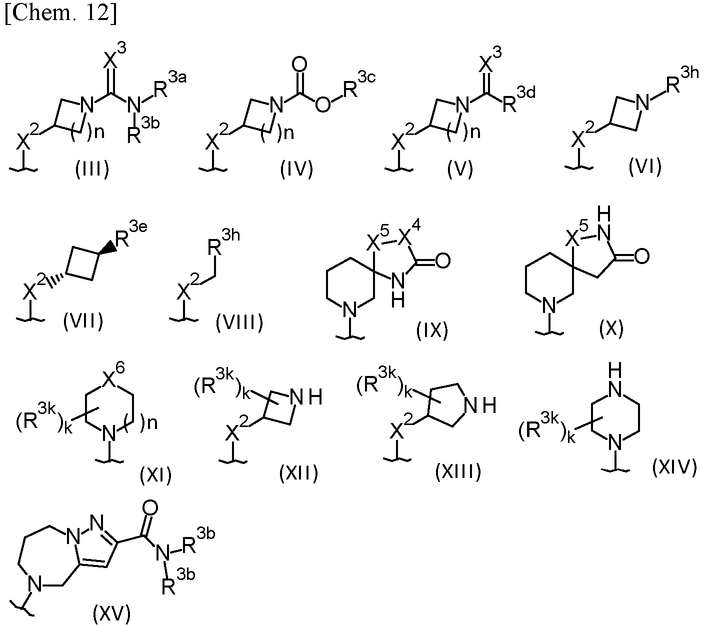
R^{3a} is -(CH₂)ₚCHR^{3f}-NR^{N1}R^{N2}; -(CH₂)ₚCHR^{3f}-OR^{3g}; a 5-membered or 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2},or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or C₁₋₃ alkyl,
R^{3c}and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group containing one or two hetero atoms selected from the group consisting of oxygen, sulfur and nitrogen optionally substituted with a group selected from the group consisting of C₁₋₃ alkyl, C₁₋₃ alkylene-OR^{3g}, C₁₋₃ alkylene-NR^{N1}R^{N2} and - NR^{N1}R^{N2},or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3e} is -O-C₂₋₃ alkylene-NR^{N1}R^{N2} or -NR^{N1}R^{N2},
R^{3f} is H, F or C₁₋₃ alkyl,
R^{3g} is H or C₁₋₃ alkyl,
R^{3h} is optionally substituted 5-membered heteroaryl containing one to four hetero atoms selected from the group consisting of oxygen, sulfur and nitrogen, optionally substituted 6-membered heteroaryl containing one to three nitrogen atoms, or cyclobutyl optionally substituted with -NR^{N1}R^{N2},
R^{N1} and R^{N2}, which are the same as or different from each other, are H or C₁₋₃ alkyl, or
R^{N1} and R^{N2}, together with the nitrogen atom to which they are attached, optionally form an optionally substituted 4-membered to 6-membered saturated heterocyclic group containing one or two hetero atoms selected from the group consisting of oxygen, sulfur and nitrogen, or
R^{3f} and R^{N1}, together with the carbon atom and the nitrogen atom to which they are attached, optionally form an optionally substituted 4-membered to 6-membered saturated heterocyclic group containing one or two hetero atoms selected from the group consisting of oxygen, sulfur and nitrogen,
each R^{3k}, which is the same as or different from each other, is a group selected from the group consisting of OH, optionally substituted C₁₋₃ alkyl, -O-(optionally substituted C₁₋₃ alkyl), -NH-(optionally substituted C₁₋₃ alkyl), -N-(optionally substituted C₁₋₃ alkyl)₂, halogen, -CN and oxo, where R^{3k} is attached only to a carbon atom that is an atom forming a ring represented by the formula (XI), the formula (XII), the formula (XIII) or the formula (XIV),
X² is -O-, -NH- or -N(C₁₋₃ alkyl)-,
X³ is O or S,
X⁴ is -CH₂- or -NH-,
X⁵ is a bond, -CH₂- or C=O,
X⁶ is -CH₂- or -O-,
provided that when R^{3c} is -(CH₂)ₚCHR^{3f}-NR^{N1}R^{N2}; X² in the formula (IV) is -O-, - NH- or -N(C₂₋₃ alkyl)-,
n is 1 or 2,
p is 1 or 2,
k is an integer of 0 to 2,
Z is a group selected from the group consisting of the formula (XVI), the formula (XVII), the formula (XVIII), the formula (XIX), the formula (XX), the formula (XXI), the formula (XXII), the formula (XXIII), the formula (XXIV), the formula (XXV), the formula (XXVI), the formula (XXVII), the formula (XXVIII), the formula (XXIX), the formula (XXX), the formula (XXXI), the formula (XXXII), the formula (XXXIII), the formula (XXXIV) and the formula (XXXV) below, 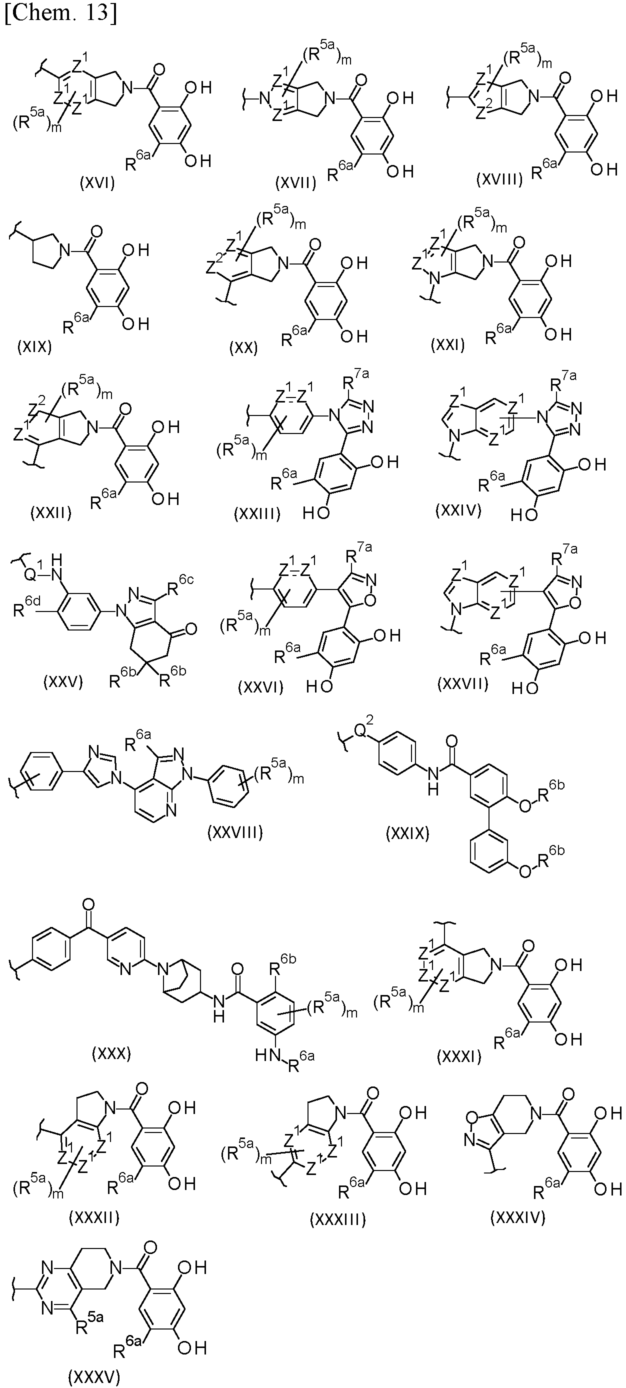
each Z¹, which is the same as or different from each other, is CH or N,
Z² is NH, NCH₃, O or S,
each R^{5a}, which is the same as or different from each other, is halogen, C₁₋₃ alkyl or - (C=O)NH₂,
R^{6a} is C₁₋₆ alkyl optionally substituted with F,
each R^{6b}, which is the same as or different from each other, is C₁₋₃ alkyl,
R^{6c} is C₁₋₃ alkyl optionally substituted with F,
R^{6d} is -(C=O)NR^{N3}R^{N4},
R^{N3} and R^{N4}, which are the same as or different from each other, are H or C₁₋₃ alkyl,
R^{7a} is OH or -(C=O)NR^{N5}R^{N6},
R^{N5} and R^{N6}, which are the same as or different from each other, are H, cyclopropyl or C₁₋₃ alkyl optionally substituted with F, and
m is an integer of 0 to 2.

5. The compound or a salt thereof according to claim 4,
wherein A is CR^{A} or N,
R^{A} is H,
R¹ is the formula (IIa) below, 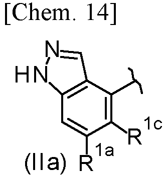
R^{1a} is F,
R^{1c} is methyl,
R² is cyclopropyl,
R³ is a group selected from the group consisting of the formula (III-a), the formula (IV-a), the formula (V-a), the formula (XIII-a) and the formula (XXXVI) below, 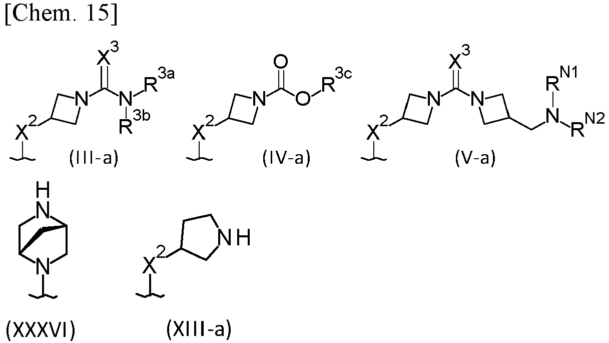
R^{3a} is -(CH₂)ₚCHR^{3f}-NR^{N1}R^{N2},
R^{3b} is H,
R^{3c} is -(CH₂)ₚCHR^{3f}-NR^{N1}R^{N2} or C₃₋₆ cycloalkyl optionally substituted with - NR^{N1}R^{N2},
R^{3f} is H,
R^{N1} and R^{N2} are both C₁₋₃ alkyl,
X² is -O-, -NH- or -N(C₁₋₃ alkyl)-,
X³ is O,
provided that when R^{3c} is -(CH₂)ₚCHR^{3f}-NR^{N1}R^{N2}; X² in the formula (IV-a) is -O-, - NH- or -N(C₂₋₃ alkyl)-,
p is 1,
R⁴ is C₁₋₆ alkyl optionally substituted with OCH₃ or tetrahydropyranyl,
X¹ is -O-,
Y is phenylene optionally substituted with F,
L is a group selected from the group consisting of the formula(XXXX), the formula (XXXX-a), the formula (XXXXI), the formula (XXXXII), the formula (XXXXIII), the formula (XXXXIV), the formula (XXXXV), the formula (XXXXVI), the formula (XXXXVII) and the formula (XXXXVIII), 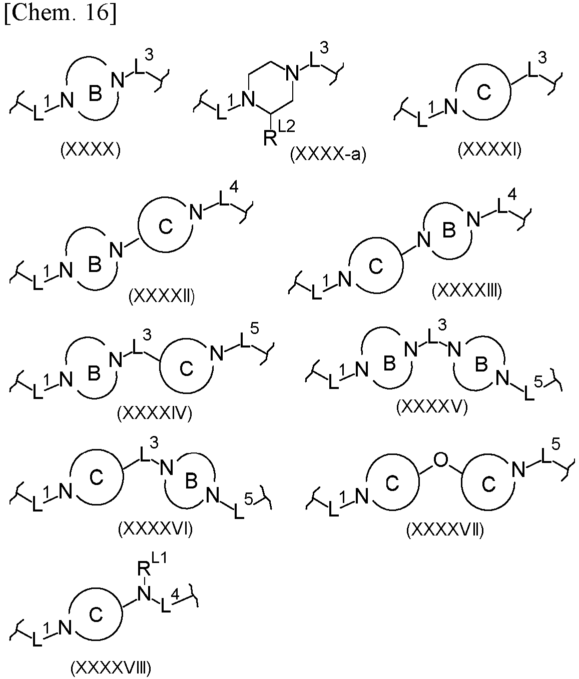
L¹ is -(CH₂)-, C=O or S(=O)₂,
L³, L⁴ and L⁵, which are the same as or different from each other, are -(CH₂)- or C=O,
R^{L1} is H or C₁₋₃ alkyl,
R^{L2} is C₁₋₃ alkyl or oxo,
ring B is piperazine, diazepane, diazabicyclo[2.2.1]heptane, diazabicyclo[3.2.1]octane, diazaspiro[3.3]heptane, diazaspiro[3.4]octane, diazaspiro[3.5]nonane, diazaspiro[4.5]decane, diazaspiro[5.5]undecane or diazabicyclo[3.3.0]octane,
ring C is azetidine, pyrrolidine or piperidine,
Z is a group selected from the group consisting of the formula (XVI-a) and the formula (XXIII-a) below, 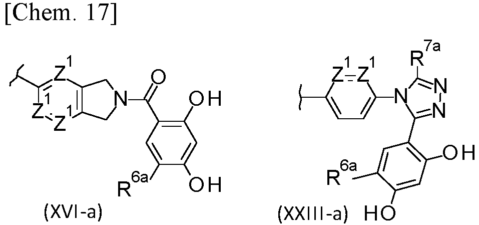
each Z¹ is CH,
R^{6a} is C₁₋₃ alkyl,
R^{7a} is OH or -(C=O)NR^{N5}R^{N6},
R^{N5} is H, and
R^{N6} is C₁₋₃ alkyl.

6. A pharmaceutical composition comprising the compound or a salt thereof according to claim 1 and one or more pharmaceutically acceptable excipients.

7. The pharmaceutical composition according to claim 6, which is a pharmaceutical composition for treating pancreatic cancer.

8. Use of the compound or a salt thereof according to claim 1 for the manufacture of a pharmaceutical composition for treating pancreatic cancer.

9. The compound or a salt thereof according to claim 1 for use in treatment of pancreatic cancer.

10. Use of the compound or a salt thereof according to claim 1 for treatment of pancreatic cancer.

11. A method for treating pancreatic cancer, the method comprising administering an effective amount of the compound or a salt thereof according to claim 1 to a subject.
